(19) Europäisches Patentamt
European Patent Office
Office européen des brevets

(11) EP 2 971 153 B1

(12) EUROPEAN PATENT SPECIFICATION

(45) Date of publication and mention of the grant of the patent:
**10.10.2018 Bulletin 2018/41**

(21) Application number: **14768725.5**

(22) Date of filing: **12.03.2014**

(51) Int Cl.:
*C12Q 1/68* (2018.01) *G01N 33/574* (2006.01)
*C12N 9/00* (2006.01)

(86) International application number:
**PCT/US2014/023976**

(87) International publication number:
**WO 2014/150688 (25.09.2014 Gazette 2014/39)**

# (54) GLYCINE, MITOCHONDRIAL ONE-CARBON METABOLISM, AND CANCER

GLYCIN, MITOCHONDRIALER EIN-KOHLENSTOFFMETABOLISMUS UND KREBS

GLYCINE, MÉTABOLISME MITOCHONDRIAL MONOCARBONÉ ET CANCER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.03.2013 US 201361791082 P**

(43) Date of publication of application:
**20.01.2016 Bulletin 2016/03**

(73) Proprietor: **The General Hospital Corporation**
**Boston, MA 02114 (US)**

(72) Inventors:
- **JAIN, Mohit**
  **La Jolla, CA 92037 (US)**
- **NILSSON, Roland**
  **S-11342 Stockholm (SE)**
- **MOOTHA, Vamsi K.**
  **Boston**
  **Massachusetts 02114 (US)**

(74) Representative: **Donald, Jenny Susan**
**Forresters IP LLP**
**Skygarden**
**Erika-Mann-Strasse 11**
**80636 München (DE)**

(56) References cited:
**WO-A1-2011/023753 US-A1- 2009 023 149**
**US-A1- 2010 179 106 US-A1- 2010 203 508**
**US-B1- 7 419 968**

- **M. JAIN ET AL: "Metabolite Profiling Identifies a Key Role for Glycine in Rapid Cancer Cell Proliferation", SCIENCE, vol. 336, no. 6084, 25 May 2012 (2012-05-25), pages 1040-1044, XP055182058, ISSN: 0036-8075, DOI: 10.1126/science.1218595**
- **S. D. SELCUKLU ET AL: "MicroRNA-9 Inhibition of Cell Proliferation and Identification of Novel miR-9 Targets by Transcriptome Profiling in Breast Cancer Cells", JOURNAL OF BIOLOGICAL CHEMISTRY, vol. 287, no. 35, 24 August 2012 (2012-08-24), pages 29516-29528, XP055283033, US ISSN: 0021-9258, DOI: 10.1074/jbc.M111.335943**
- **UNHEE LIM ET AL: "Gene-nutrient interactions among determinants of folate and one-carbon metabolism on the risk of non-Hodgkin lymphoma: NCI-SEER Case-Control Study", BLOOD, vol. 109, no. 7, 1 April 2007 (2007-04-01) , pages 3050-3059, XP055305114, DOI: 10.1182/blood-2006-**
- **STÉPHANE PUYO ET AL: "Abstract 2692: Overexpression of serine hydroxy methyl transferase 2 (SHMT2) in high grade prostate cancers as a marker of tumor response to oxaliplatin", CANCER RESEARCH, vol. 70, no. 8 Supplement, 15 April 2010 (2010-04-15), pages 2692-2692, XP055304892, US ISSN: 0008-5472, DOI: 10.1158/1538-7445.AM10-2692**
- **JAIN, M. ET AL.: 'Metabolite Profiling Identifies A Key Role For Glycine In Rapid Cancer Cell Proliferation.' SCIENCE vol. 336, no. 6084, 25 May 2012, pages 1040 - 1044, XP055182058**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 971 153 B1

- VAZQUEZ, A ET AL.: 'Overexpression Of The Mitochondrial Folate And Glycine-Serine Pathway: A New Determinant Of Methotrexate Selectivity In Tumors.' CANCER RESEARCH vol. 73, 07 November 2012, pages 478 - 482, XP055283031
- SELCUKLU, SD ET AL.: 'MicroRNA-9 Inhibition Of Cell Proliferation And Identification Of Novel miR-9 Targets By Transcriptome Profiling In Breast Cancer Cells.' JOURNAL OF BIOLOGICAL CHEMISTRY vol. 287, no. 35, 02 July 2012, pages 29516 - 29528, XP055283033

SEQUENCE LISTING

<110> The General Hospital Corporation

<120> Glycine, Mitochondrial One-Carbon Metabolism, and Cancer

<130> 00786-0779WO1

<150> US 61/791,082
<151> 2013-03-15

<160> 11

<170> FastSEQ for Windows Version 4.0

<210> 1
<211> 942
<212> DNA
<213> Artificial Sequence

<220>
<223> yeast codon optimized gene encoding human MTHFD2

<400> 1

```
catatggagg ccgtggttat cagtggccgc aagctggccc agcagatcaa gcaggaggtg 60
cgccaagaag tggaagaatg ggttgccagc ggcaacaagc gcccgcatct gagcgtgatc 120
ctggtgggcg agaacccggc aagccacagc tacgtgctga acaaaacacg cgcagcagca 180
gtggtgggca tcaacagcga gacaatcatg aaaccggcca gcatcagcga ggaagagtta 240
ctgaacttaa ttaacaagct gaataacgac gacaacgtgg acggcctgct ggtgcagtta 300
ccgctgccgg aacatatcga cgaacgccgc atctgcaacg ccgtgagtcc tgataaggac 360
gtggacggct ttcacgtgat caatgttggc cgcatgtgct tagaccagta cagcatgctg 420
ccggcaaccc cttgggggcgt ttgggagatc atcaagcgca ccggtatccc gaccctgggt 480
aagaacgttg tggtggccgg ccgtagcaag aacgtgggca tgcctatcgc aatgttactg 540
cacaccgacg gcgcacatga acgtcctggt ggcgatgcaa ccgttaccat cagtcaccgt 600
tacaccccga aggagcaact gaaaaagcac accatcctgg ccgacattgt gatcagcgca 660
gccggcattc cgaacctgat caccgcagac atgatcaaag agggcgccgc cgtgatcgac 720
gtgggcatta accgcgtgca cgatccggtg acagccaaac cgaagctggt gggtgacgtg 780
gacttcgagg gcgtgcgtca aaaagccggc tacatcaccc cggttcctgg tggcgttggc 840
cctatgaccg tggccatgct gatgaagaac accatcattg ccgccaagaa ggtgctgcgt 900
ctggaggagc gcgagcacca tcatcaccac cactaaggta cc                   942
```

<210> 2
<211> 310
<212> PRT
<213> Artificial Sequence

<220>
<223> MTHFD2 6-HIS fusion protein

<400> 2

```
Met Glu Ala Val Val Ile Ser Gly Arg Lys Leu Ala Gln Gln Ile Lys
 1               5                  10                  15
Gln Glu Val Arg Gln Glu Val Glu Glu Trp Val Ala Ser Gly Asn Lys
            20                  25                  30
Arg Pro His Leu Ser Val Ile Leu Val Gly Glu Asn Pro Ala Ser His
        35                  40                  45
Ser Tyr Val Leu Asn Lys Thr Arg Ala Ala Ala Val Val Gly Ile Asn
    50                  55                  60
Ser Glu Thr Ile Met Lys Pro Ala Ser Ile Ser Glu Glu Glu Leu Leu
65                  70                  75                  80
Asn Leu Ile Asn Lys Leu Asn Asn Asp Asp Asn Val Asp Gly Leu Leu


                85                  90                  95
Val Gln Leu Pro Leu Pro Glu His Ile Asp Glu Arg Arg Ile Cys Asn
            100                 105                 110
Ala Val Ser Pro Asp Lys Asp Val Asp Gly Phe His Val Ile Asn Val
        115                 120                 125
Gly Arg Met Cys Leu Asp Gln Tyr Ser Met Leu Pro Ala Thr Pro Trp
    130                 135                 140
Gly Val Trp Glu Ile Ile Lys Arg Thr Gly Ile Pro Thr Leu Gly Lys
145                 150                 155                 160
Asn Val Val Val Ala Gly Arg Ser Lys Asn Val Gly Met Pro Ile Ala
                165                 170                 175
Met Leu Leu His Thr Asp Gly Ala His Glu Arg Pro Gly Gly Asp Ala
            180                 185                 190
Thr Val Thr Ile Ser His Arg Tyr Thr Pro Lys Glu Gln Leu Lys Lys
        195                 200                 205
His Thr Ile Leu Ala Asp Ile Val Ile Ser Ala Ala Gly Ile Pro Asn
    210                 215                 220
Leu Ile Thr Ala Asp Met Ile Lys Glu Gly Ala Ala Val Ile Asp Val
225                 230                 235                 240
Gly Ile Asn Arg Val His Asp Pro Val Thr Ala Lys Pro Lys Leu Val
                245                 250                 255
Gly Asp Val Asp Phe Glu Gly Val Arg Gln Lys Ala Gly Tyr Ile Thr
            260                 265                 270
Pro Val Pro Gly Gly Val Gly Pro Met Thr Val Ala Met Leu Met Lys
        275                 280                 285
Asn Thr Ile Ile Ala Ala Lys Lys Val Leu Arg Leu Glu Glu Arg Glu
    290                 295                 300
His His His His His His
305                 310
```

<210> 3
<211> 236
<212> DNA
<213> Artificial Sequence

<220>
<223> yeast codon-optimized construct

<400> 3

```
tggcgaatgg gacgcgccct gtagcggcgc attaagcgcg gcgggtgtgg tggttacgcg 60
cagcgtgacc gctacacttg ccagcgccct agcgcccgct cctttcgctt tcttcccttc 120
ctttctcgcc acgttcgccg gctttccccg tcaagctcta aatcgggggc tccctttagg 180
gttccgattt agtgctttac ggcacctcga ccccaaaaaa cttgattagg gtgatg     236
```

<210> 4
<211> 350

<212> PRT
<213> Homo sapien

<400> 4

```
Met Ala Ala Thr Ser Leu Met Ser Ala Leu Ala Ala Arg Leu Leu Gln
 1               5                  10                  15
Pro Ala His Ser Cys Ser Leu Arg Leu Arg Pro Phe His Leu Ala Ala
            20                  25                  30
Val Arg Asn Glu Ala Val Val Ile Ser Gly Arg Lys Leu Ala Gln Gln
        35                  40                  45
Ile Lys Gln Glu Val Arg Gln Glu Val Glu Glu Trp Val Ala Ser Gly
    50                  55                  60
Asn Lys Arg Pro His Leu Ser Val Ile Leu Val Gly Glu Asn Pro Ala
65                  70                  75                  80
Ser His Ser Tyr Val Leu Asn Lys Thr Arg Ala Ala Ala Val Val Gly
                85                  90                  95

Ile Asn Ser Glu Thr Ile Met Lys Pro Ala Ser Ile Ser Glu Glu Glu
            100                 105                 110
Leu Leu Asn Leu Ile Asn Lys Leu Asn Asn Asp Asp Asn Val Asp Gly
        115                 120                 125
Leu Leu Val Gln Leu Pro Leu Pro Glu His Ile Asp Glu Arg Arg Ile
    130                 135                 140
Cys Asn Ala Val Ser Pro Asp Lys Asp Val Asp Gly Phe His Val Ile
145                 150                 155                 160
Asn Val Gly Arg Met Cys Leu Asp Gln Tyr Ser Met Leu Pro Ala Thr
                165                 170                 175
Pro Trp Gly Val Trp Glu Ile Ile Lys Arg Thr Gly Ile Pro Thr Leu
            180                 185                 190
Gly Lys Asn Val Val Val Ala Gly Arg Ser Lys Asn Val Gly Met Pro
        195                 200                 205
Ile Ala Met Leu Leu His Thr Asp Gly Ala His Glu Arg Pro Gly Gly
    210                 215                 220
Asp Ala Thr Val Thr Ile Ser His Arg Tyr Thr Pro Lys Glu Gln Leu
225                 230                 235                 240
Lys Lys His Thr Ile Leu Ala Asp Ile Val Ile Ser Ala Ala Gly Ile
                245                 250                 255
Pro Asn Leu Ile Thr Ala Asp Met Ile Lys Glu Gly Ala Ala Val Ile
            260                 265                 270
Asp Val Gly Ile Asn Arg Val His Asp Pro Val Thr Ala Lys Pro Lys
        275                 280                 285
Leu Val Gly Asp Val Asp Phe Glu Gly Val Arg Gln Lys Ala Gly Tyr
    290                 295                 300
Ile Thr Pro Val Pro Gly Gly Val Gly Pro Met Thr Val Ala Met Leu
305                 310                 315                 320
Met Lys Asn Thr Ile Ile Ala Ala Lys Lys Val Leu Arg Leu Glu Glu
                325                 330                 335
Arg Glu Val Leu Lys Ser Lys Glu Leu Gly Val Ala Thr Asn
            340                 345                 350
```

<210> 5
<211> 21
<212> DNA
<213> Artificial Sequence

<220>
<223> shRNA-encoding sequences

<400> 5

gaggtgtgtg atgaagtcaa a 21

<210> 6
<211> 21
<212> DNA
<213> Artificial Sequence

<220>
<223> shRNA-encoding sequences

<400> 6
acaagtactc ggagggttat c 21

<210> 7
<211> 21
<212> DNA
<213> Artificial Sequence

<220>
<223> shRNA-encoding sequences

<400> 7
gtctgacgtc aagcggatat c 21

<210> 8
<211> 21
<212> DNA
<213> Artificial Sequence

<220>
<223> shRNA-encoding sequences

<400> 8
cggagagttg tggactttat a 21

<210> 9
<211> 21
<212> DNA
<213> Artificial Sequence

<220>
<223> shRNA-encoding sequences

<400> 9
acaacagcca caacgtctat a 21

<210> 10
<211> 21
<212> DNA
<213> Artificial Sequence

<220>
<223> shRNA-encoding sequences

<400> 10
cgaatgtgtt tggatcagta t 21

<210> 11

<211> 21
<212> DNA
<213> Artificial Sequence

<220>
<223> shRNA-encoding sequences

<400> 11
gcagttgaag aaacatacaa t 21

## Description

### FEDERALLY SPONSORED RESEARCH OR DEVELOPMENT

This invention was made with Government support under Grant Nos. R01DK081457, 5R01GM099683, and 5K08HL107451 awarded by the National Institutes of Health. The Government has certain rights in the invention.

### TECHNICAL FIELD

Methods of treatment and diagnosis of subjects with cancer, generally comprising determining levels of SHMT2 protein, transcript, or activity, and administering an antifolate that targets SHMT2, wherein the antifolate is methotrexate or any one of the antifolate compounds recited in Table 1.

### BACKGROUND

Malignant transformation typically results from mutations that alter cellular physiology to confer a proliferative advantage (Nowell, Science 194, 23 (1976); Hanahan and Weinberg, Cell 144, 646 (2011)). Despite the genetic heterogeneity and complexity of cancer (Stratton et al., Nature 458, 719 (2009)), transformed cells exhibit a number of proposed common hallmarks, including metabolic reprogramming, which manifests as altered nutrient uptake and utilization (Hanahan and Weinberg, Cell 144, 646 (2011); Hsu and Sabatini, Cell 134, 703 (2008)). Although metabolic reprogramming is thought to be essential for rapid cancer cell proliferation, a systematic characterization of the metabolic pathways active in transformed cells is lacking, and the contribution of these pathways in promoting rapid cancer cell proliferation remains unclear (Hsu and Sabatini, Cell 134, 703 (2008)). Existing studies of cancer metabolism have only examined relatively few cell lines, and have largely focused on measurement of intracellular metabolite pools (Sreekumar et al., Nature 457, 910 (2009)) from which it is difficult to infer metabolic pathway activity, or have estimated metabolic flux through a limited number of reactions using isotope tracing (DeBerardinis et al., Proc Natl Acad Sci U S A 104, 19345 (2007)).

M. Jain et al., Science vol. 336, no. 6084, 2012, pages 1040-1044 discloses that glycine consumption and expression of the mitochondrial glycine biosynthetic pathway is correlated with rates of proliferation across cancer cells.

S. D. Selcuklu et al., Journal of Biological Chemistry, vol. 287, no.35, 2012, pages 29516-29528 discloses MTHFD2 as a miR-9 target gene that affects cell proliferation.

US 2010/203508 A1 discloses methods for analyzing genetic and/or metabolite biomarkers to individualize methotrexate therapy in patients who have been diagnosed with a disease such as an inflammatory disease, autoimmune disease or cancer.

Unhee Lim et al., Blood, vol. 109, no.7, 2007, pages 3050-3059 discloses a correlation between one-carbon metabolism enzymes and non Hodgkin lymphoma.

Stéphane Puyo et al., Cancer Research, vol. 70, no. 8, supplement, 2010, pages 2692-2692 discloses that oxaliplatin could be used as an alternative therapy for high grade prostate cancer overexpressing SHMT2.

Vazquez A et al., Cancer Research, vol. 73, 2012, pages 478-482 discloses that methotrexate could be used to target cancers with high rates of proliferation.

### SUMMARY

As demonstrated herein, mitochondrial glycine (and one-carbon, or 1-C, metabolism) is important in cancer cell metabolism. Glycine consumption is altered in some cancers, and is a unique predictor of antifolate sensitivity as well as of cancer cell proliferation. Glycine starvation can reduce the proliferation of sensitive cancers.

In addition, MTHFD2, which is a part of the mitochondrial 1-C pathway, is one of the strongest differentially expressed genes and, in the present analysis, is the most differentially expressed metabolic enzyme. MTHFD2 appears to be an embryonic enzyme that is resurrected in cancer; MTHFD2 is highly upregulated across many cancers relative to normal tissues. Genetic silencing of MTHFD2 and inhibition with small molecules slows proliferation across a number of cancer cell lines.

In a first aspect, the invention provides an antifolate drug for use in treating a cancer in a subject. The methods include; determining a level of SHMT2 protein, SHMT2 mRNA, or SHMT2 activity in a sample comprising tumour cells from a subject; comparing the level ofSHMT2 protein, SHMT2 mRNA, or SHMT2 activity in the sample to a reference level of SHMT2 protein, SHMT2 mRNA, or SHMT2 activity; selecting a subject who has a level of SHMT2 protein, SHMT2 mRNA, or SHMT2 activityabove the reference level; and administering a therapeutically effective amount of an antifolate drug, wherein the antifolate drug is methotrexate or any one of the antifolate compounds recited in Table 1.

In some embodiments, the antifolate drug is linked covalently to a mitochondrial targeting moiety.

In some embodiments, the agent is linked covalently to a mitochondrial-targeting moiety.

In some embodiments, wherein the agent is linked covalently to mitochondrial-targeting moiety tetraphenylphosphonium.

In a second aspect, the invention provides a method of diagnosing cancer in a subject. The methods include; determining a level of SHMT2 protein, mRNA, or activity in a sample suspected of comprising tumor cell from a subject; comparing the level of SHMT2 protein, mRNA, or activity in the sample to a reference level of SHMT2 protein, mRNA, or activity; and diagnosing the subject in which the sample has a level of SHMT2 above the reference level as having cancer, and selecting the treatment of an antifolate drug.

In some embodiments, the cancer or tumor is a carcinoma.

In some embodiments, the carcinoma is glioma, glioblastoma, breast, ovarian, renal cell, lung; melanoma, cervical, adrenal, brain, esophagus, gastric, germ cell, head / neck, prostate, melanoma, liver, pancreas, testicular, or colon cancer;

more preferably wherein the carcinoma is not breast or bladder cancer.

In another aspect, the disclosure provides methods for treating a cancer in a subject. The methods of the disclosure include obtaining a sample comprising tumor cells from the subject; determining a level of one or more of SHMT2, MTHFD2, and/or MTHFD1L protein, mRNA, or activity in the sample; comparing the level of SHMT2, MTHFD2, and/or MTHFD1L protein, mRNA, or activity in the sample to a reference level of SHMT2, MTHFD2, and/or MTHFD1L protein, mRNA, or activity; selecting a subject who has a level of SHMT2, MTHFD2, and/or MTHFD1L protein, mRNA, or activity above the reference level; and treating the subject by administering a therapeutically effective amount of one or both of an antifolate drug and an agent that inhibits a mitochondrial 1-carbon (1-C) pathway enzyme, e.g., an agent that inhibits SHMT2 or MTHFD2.

In some aspects of the disclosure, the antifolate drug is methotrexate.

In some aspects of the disclosure, the agent that inhibits MTHFD2 is 6-hydroxy-DL DOPA, calmidazolium chloride, CDOO, ebselen, celestrol, GW5074, iodoacetamide, para-benzoquinone, or protoporphyrin IX disodium.

In some aspects of the disclosure, the agent that inhibits a mitochondrial 1-carbon (1-C) pathway enzyme is an inhibitory nucleic acid that inhibits SHMT2 or MTHFD2, preferably SHMT2.

In some aspects of the disclosure, the inhibitory nucleic acid is an siRNA, shRNA, or antisense oligonucleotide.

In another aspect, the disclosure provides methods for treating a cancer in a subject. The methods include administering to the subject a therapeutically effective amount of a composition comprising an active agent that inhibits MTHFD2, or a composition thereof.

In some aspects of the disclosure, the active agent is selected from the group consisting of 6-hydroxy-DL DOPA, calmidazolium chloride, CDOO, ebselen, celestrol, GW5074, iodoacetamide, para-benzoquinone, protoporphyrin IX disodium, methotrexate, pemetrexed, or 5-fluorouracil, optionally.

In some aspects of the disclosure, the active agent is ebselen.

In some aspects of the disclosure, the inhibitor covalently modifies MTHFD2.

In some aspects of the disclosure, the inhibitor covalently or non-covalently modifies one or both of Cys145 or Cys166 of MTHFD2, preferably Cys145.

In some aspects of the disclosure, the active agent is linked covalently to a mitochondrial-targeting moiety, e.g., tetraphenylphosphonium.

In some aspects, the method of the disclosure includes identifying the cancer in the subject as having a level of SHMT2, MTHFD2, and/or MTHFD1L protein, mRNA, or activity above a reference level of SHMT2, MTHFD2, and/or MTHFD1L protein, mRNA, or activity.

In another aspect, the disclosure provides methods for predicting likelihood of survival in a subject who has cancer. The methods of the disclosure include obtaining a sample comprising tumor cells from the subject; determining a level of SHMT2, MTHFD2, and/or MTHFD1L protein, mRNA, or activity in the sample; comparing the level of SHMT2, MTHFD2, and/or MTHFD1L protein, mRNA, or activity in the sample to a reference level of SHMT2, MTHFD2, and/or MTHFD1L protein, mRNA, or activity; assigning a high predicted likelihood of survival to a subject who has a level of SHMT2, MTHFD2, and/or MTHFD1L above the reference level, or assigning a low predicted likelihood of survival to a subject who has a level of SHMT2, MTHFD2, and/or MTHFD1L below the reference level.

In another aspect, the disclosure provides methods for diagnosing cancer in a subject. The methods of the disclosure include obtaining a sample suspected of comprising tumor cells from the subject; determining a level of SHMT2, MTHFD2, and/or MTHFD1L protein, mRNA, or activity in the sample; comparing the level of SHMT2, MTHFD2, and/or MTHFD1L protein, mRNA, or activity in the sample to a reference level of SHMT2, MTHFD2, and/or MTHFD1L protein, mRNA, or activity; and diagnosing a subject who has a level of SHMT2, MTHFD2, and/or MTHFD1L above the reference level as having cancer.

In some aspects of the methods of the disclosure described herein, the MTHFD2 activity is NAD-dependent methylenetetrahydrofolate dehydrogenase / cyclohydrolase activity.

In another aspect, the disclosure provides methods for identifying a candidate compound for the treatment of cancer. The methods of the disclosure include providing a sample comprising a cell, e.g., a tumor cell, expressing MTHFD2; contacting the sample with a test compound; determining a level of NAD-dependent methylenetetrahydrofolate dehydrogenase / cyclohydrolase activity in the sample in the presence of the test compound in the absence of reducing agents, e.g., DTT or mercaptoethanol; comparing the level of activity in the presence of the test compound to a reference level of activity; and selecting a compound that is associated with reduced activity as a candidate compound.

In some aspects of the disclosure, the reference level is a level of NAD-dependent methylenetetrahydrofolate dehydrogenase / cyclohydrolase activity in the absence of the test compound, e.g., in a control sample.

In some aspects of the disclosure, the test compound is a cysteine modifying agent.

In some aspects of the disclosure, the method include contacting a cancer cell with the candidate compound; determining a rate of proliferation or viability in the presence of the candidate compound; comparing the rate of proliferation or viability in the presence of the candidate compound to a reference rate of proliferation or viability; and selecting as a candidate therapeutic compound a candidate compound that decreases the rate of proliferation or viability.

In some aspects of the disclosure, the reference rate of proliferation or viability is a level of rate of proliferation or viability in the absence of the candidate compound, e.g., in a control sample.

In some aspects of the disclosure, the method include administering the candidate therapeutic compound to an animal model of cancer, e.g., a xenograft model; determining an effect of the candidate therapeutic compound on a parameter of the cancer in the animal model; and selecting as a therapeutic compound a candidate therapeutic compound that improves one or more parameters of cancer in the animal model. In some aspects of the disclosure, the parameter is tumor size, tumor growth, time to tumor development (or average age of tumor development), or metastasis, and an improvement is a reduction in tumor size, tumor growth rate, or metastasis, or an increase in time to tumor development (or average age of tumor development).

In another aspect, the disclosure provides methods for treating or identifying a subject for treatment with a low-glycine diet and/or administration of sodium benzoate. The methods of the disclosure include determining levels of glycine uptake in a sample comprising cancer cells from the subject, comparing the levels of glycine uptake to reference levels of glycine uptake, selecting a subject who has levels of levels of glycine uptake above the reference levels for treatment with a low-glycine diet or administration of sodium benzoate, and optionally administering the treatment to the subject.

In another aspect, the disclosure provides methods for predicting aggressiveness or growth rate of a tumor in a subject. The methods of the disclosure include determining a level of glycine uptake in a sample comprising cells from the tumor; comparing the level of glycine uptake in the sample to a reference level of glycine uptake; assigning a high likelihood of aggressiveness and high growth rate to a subject who has a tumor with a level of glycine uptake above the reference level, or assigning a low likelihood of aggressiveness and high growth rate to a subject who has a tumor with a level of glycine uptake below the reference level.

In another aspect, the disclosure provides methods for predicting aggressiveness or growth rate of a tumor in a subject. The methods of the disclosure include determining a level of one or more of SHMT2, MTHFD2, and/or MTHFD1L mRNA, protein, or activity in a sample comprising cells from the tumor; comparing the level of SHMT2, MTHFD2, and/or MTHFD1L protein, mRNA, or activity in the sample to a reference level of SHMT2, MTHFD2, and/or MTHFD1L protein, mRNA, or activity; assigning a high likelihood of aggressiveness and high growth rate to a subject who has a tumor with a level of HMT2, MTHFD2, and/or MTHFD1L above the reference level, or assigning a low likelihood of aggressiveness and high growth rate to a subject who has a tumor with a level of SHMT2, MTHFD2, and/or MTHFD1L below the reference level.

In some aspects , the present disclosure provides a method for treating a cancer, e.g., a carcinoma, in a patient, comprising administering to the patient an inhibitor of MTHFD2, or pharmaceutically acceptable composition thereof.

In some aspects of the methods of the disclosure described herein, the cancer is a carcinoma, e.g., glioma, glioblastoma, breast, ovarian, renal cell, lung; melanoma, cervical, adrenal, brain, esophagus, gastric, germ cell, head / neck, prostate, melanoma, liver, pancreas, testicular, or colon cancer.

In some aspects of the disclosure, the carcinoma is not breast or bladder cancer.

In another aspect, the disclosure provides methods for of inhibiting proliferation of glycine consuming cells, the method comprising contacting the cells with an antifolate agent.

In another aspect, the disclosure provides methods for treating cancer by inhibiting proliferation of glycine consuming cells.

In certain aspects, the present disclosure provides a method of inhibiting MTHFD2 in a biological sample, comprising contacting said sample with a compound that inhibits MTHFD2.

In certain aspects, the present disclosure provides a method of inhibiting MTHFD2 in a patient, comprising administering to the patient a compound that inhibits MTHFD2, or a pharmaceutically acceptable composition thereof

In another aspect, the disclosure provides methods for inhibiting MTHFD2 in a patient or a biological sample comprising administering to the patient, or contacting the biological sample with, an inhibitor of MTHFD2.

In another aspect, the disclosure provides methods for inhibiting glycine metabolism or NAD-dependent methylenetetrahydrofolate dehydrogenase activity in a cell, comprising contacting the cell with an antifolate agent.

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. In case of conflict, the present specification, including definitions, will control.

Other features and advantages of the invention will be apparent from the following detailed description and figures, and from the claims.

## DESCRIPTION OF DRAWINGS

FIGs. 1A-G: Glycine consumption and synthesis are correlated with rapid cancer cell proliferation. (1A) Distribution of Spearman correlations between 111 metabolite CORE profiles and proliferation rate across 60 cancer cell lines. Only metabolites highlighted in red are significant at P < 0.05, Bonferroni-corrected. (1B) Glycine CORE versus proliferation rate across 60 cancer cell lines (left) and selected solid tumor types (right). Cell lines selected for follow-up experiments are highlighted in red. Joined data points represent replicate cultures. P values are Bonferroni-corrected for 111 tested metabolites. (1C) Distribution of Spearman correlations between gene expression of 1425 metabolic enzymes and proliferation rates across 60 cancer cell lines. Highlighted are mitochondrial (MTHFD2, SHMT2, MTHFD1L) and cytosolic (MTHFD1, SHMT1) glycine metabolism enzymes. (ID) Schematic of cytosolic and mitochondrial glycine metabolism. (IE) Abundance of unlabeled (0) and labeled (+1) intracellular glycine and serine in LOX IMVI cells grown on 100% extracellular (13C)glycine. (1F) Growth of A498 and LOXIMVI cells expressing shRNAs targeting SHMT2 (sh1-4) or control shRNA (shCtrl), cultured in the absence (solid bars) or presence (open bars) of glycine (gly). (1G) Growth of 10 cancer cell lines expressing shRNA targeting SHMT2 (sh4) after 3 days, cultured in the absence (-gly, solid bars) or presence (+gly, open bars) of glycine. Cell number is presented as a ratio relative to +gly cells. Error bars in (E), (F), and (G) denote SD. (1H) RT-PCR analysis of SHMT2 transcript levels following silencing by the sh4 hairpin in 10 cancer cell lines, as in Fig. 1G.

FIGs. 2A-B. Expression of the mitochondrial glycine biosynthesis pathway is associated with mortality in breast cancer patients. (2A) Kaplan-Meier survival analysis of six independent breast cancer patient cohorts (22-27). Patients were separated into above-median (bottom line) and below-median (top line) expression of mitochondrial glycine metabolism enzymes (SHMT2, MTHFD2, and MTHFD1L, Fig. 1D). Dashes denote censored events. (2B) Meta-analysis of Cox hazard ratios for the six studies. Solid lines denote 95% confidence intervals; boxes denote the relative influence of each study over the results (inverse squared SE); diamond marks the overall 95% confidence interval.

FIG. 3. Glycine consumption is strongly correlated to and predictive of cellular sensitivity to antifolates. Cells that consume glycine were uniquely sensitive to multiple antifolate agents (grey dots) but were not more sensitive to other chemotherapy agents (black dots), including those agents that target rapidly proliferating cancer cells, including 5-fluorouracil.

FIG. 4 is a graph showing a meta-analysis of tumor gene expression. The distribution of Z scores for 20,450 genes is shown across 20 diverse cancer types indicating the degree of expression change in cancer versus corresponding normal tissue. Among the 20,450 genes measured, the top 50 genes most consistently upregulated genes (defined as the number of datasets in which the gene appears within the top 5% of upregulated genes) are shown in Table 2 below. This gene list includes known drug targets, including TYMS, RRM2, TOP2A, and AURKA, as well as the higher ranking gene MTHDF2 (gene rank 8). In contrast the cytosolic paralogue MTHFD1 (gene rank 548) or the adult paralogue MTHFD2L (rank score 11912), were not highly upregulated in cancer relative to normal counterparts. Those cancer datasets in which MTHFD2 appears in the top 5% of upregulated genes (by false discovery rate) are indicated by black dots in FIG. 4.

FIG. 5 is a pair of bar graphs showing that MTHFD2 is strongly expressed by immunohistochemistry analysis in tumor cells, with limited expression in the surrounding normal stroma.

FIG. 6 is a set of graphs showing that in breast, renal, melanoma, and colon cancer studies, above median expression of MTHFD2 (lower, grey line) was associated with worse survival than below median expression of MTHFD2 (upper, black line).

FIG. 7A is a set of seven graphs showing expression of MTHFD2, MTHFD1, MTHFD2L, RRM2, DHFR, TOP2A, and TYMS in 1) normal, non-proliferating tissues (grey bars), 2) normal proliferating tissues (black bars) including colon epithelium and leukocytes, and 3) cancer tissues included in this tissue atlas dataset (white bars).

FIG. 7B shows that among all 20,000 genes evaluated, MTHFD2 had the highest min/max ratio.

FIG. 7C is a set of three line graphs showing that known chemotherapeutic targets, including DHFR, RRM2 and TOP2A, were strongly induced in normal tissue when stimulated to proliferate, but MTHD2 was not induced in normal proliferating cells (see Fig. 7C, left and middle panels). The exception was activated T cells, which do upregulate MTHFD2 expression when activated (Fig. 7C, right panel).

FIG. 8 is a set of graphs showing the effects of genetic knockdown of MTHFD2 using shRNA on cell proliferation in the following cell lines:

| Cell Line | Tumor Type |
|---|---|
| U251 | Glioma |
| HCT116 | Colon |
| SNB75 | Glioblastoma |
| HS-578T | Breast |
| SW620 | Colon |
| OVCAR8 | Ovarian |
| A498 | Renal cell |
| HOP92 | Lung |
| MCF7 | Breast |
| EKVX | Lung |
| HT29 | Colon |
| H460 | Lung |
| SF295 | Glio blastoma |
| LOXIMVI | Melanoma |
| HeLa | Cervical |
| HCT115 | Colon |

FIGs. 9A-C are line graphs showing the results of analysis of wild type MTHFD2 protein or MTHFD2 C145S, C166S or both C145S/C166S mutants in the presence of 6-hydroxy-DL-DOPA (9A), Celastrol (9B), and Ebselen (9C).

## DETAILED DESCRIPTION

Liquid chromatography-tandem mass spectrometry was used to profile the cellular consumption and release (CORE) of 219 metabolites spanning the major pathways of intermediary metabolism, to probe the relation between metabolism and proliferation in cancer cells. The results demonstrated that glycine uptake/consumption, and genes related to mitochondrial 1-carbon metabolism, are correlated with proliferation across a diverse set of cancers, and can be targeted to alter proliferation.

### Glycine Consumption

As described herein, there was an unexpected increased reliance on glycine metabolism in rapidly proliferating cancer cells, a phenotype that was not observed in rapidly proliferating nontransformed cells. Glycine uptake can therefore be used as a predictor of cancer cell proliferation rates (see, e.g., Figs. 1a, b). Cell proliferation rates are related to cancer aggressiveness and growth rates, so monitoring glycine uptake activity can be used to determine how aggressive or rapidly proliferating a tumor might be. Glycine uptake also represents a unique metabolic vulnerability in rapidly proliferating cancer cells that can be targeted for therapeutic benefit.

Glycine is utilized for de novo purine nucleotide biosynthesis in rapidly proliferating cancer cells; mechanisms including utilization of one-carbon groups derived from glycine for cellular methylation reactions (Zhang et al., Cell 148, 259 (2012)) may be critical in linking glycine to cancer proliferation.

### Assays for Glycine Consumption

A number of in vitro assays are known in the art for quantifying glycine uptake in cells, e.g., in tumor cells from a subject. Traditional assays measure uptake of radio labelled substrate (e.g. [$^3$H]glycine) into cells; typically, after incubation of the cells in the presence of the substrate, the cells are washed and solubilized, and the amount of radioactivity taken up into the cells measured by scintillation counting (Morrow et al., FEBS Lett., 1998, 439(3), 334-340; Williams et al., Anal. Biochem., 2003, 321(1), 31-37). Other methods can also be used, e.g., mass spectrometry methods such as liquid chromatography-tandem mass spectrometry, or HPLC, e.g., hydrophilic interaction chromatography (HILIC) or UPLC, e.g., as described herein, as well as other methods known in the art, e.g., as described in Allan et al.., Combinatorial Chemistry & High Throughput Screening, 9:9-14 (2006) (a homogenous cell-based assay using the FLIPR membrane potential blue dye (Molecular Devices) and FLEXstation); and Kopek et al., J Biomol Screen. 14(10):1185-94 (2009).

To measure glycine metabolism *in vivo,* e.g., in living subjects with cancer, one of skill in the art could non-invasively monitor glycine consumption in tumors in live subjects, e.g., using PET imaging and labeled glycine (synthesis of $^{11}$C-labelled glycine PET probe and its use in tumors is known in the art, e.g., as reported in Bolster et al., Int J Rad Appl Instrum Part A, 37(9):985-7 (1986) and Johnstrom et al., Int J Rad Appl Instrum A. 38(9):729-34 (1987)). Alternatively, glycine uptake in patients with cancer could be monitored through infusion of glycine labeled with a stable carbon-13 isotope, and upon excision of the cancer, the labeled glycine could be measured in the tumor specimen via mass spectrometric analysis.

**Mitochondrial 1-Carbon Metabolism**

Intracellular nucleotide metabolism is essential for cancer cell proliferation. De novo biosynthesis of nucleotides involves a number of metabolic enzymes, spanning the mitochondrion, the cytosol and the nucleus. A number of the metabolic enzymes in this pathway are actually targets of currently employed chemotherapy agents, including methotrexate (targets DHFR), 5-fluorouracil (targets TYMS), as well as a number of antimetabolite chemotherapy agents. These drugs are highly effective against cancer, but are limited by their *on target* side effects which occur in rapidly proliferating normal cells. Recently it was discovered that a number of these drug targets, including DHFR and TYMS, have dual localized or paralogous enzymes present in the mitochondria, including the paralogue DHFRL1 (Anderson et al., Proc Natl Acad Sci U S A. 2011;108:15163-15168; McEntee et al., Proc Natl Acad Sci U S A. 2011;108:15157-15162) or the alternatively mitochondrial localized TYMS (Samsonoff et al., The Journal of biological chemistry. 1997;272:13281-13285). In principle, targeting of these agents specifically to the mitochondria, e.g., by linkage to a mitochondrial targeting moiety as known in the art or described herein, may limit on-target drug toxicity while maintaining or increasing drug efficacy. In addition, for a number of additional enzymatic reactions within this critical pathway, there exists paralogous mitochondrial and cytosolic enzymes, or paralogous cancer and adult isoforms.

*Mitochondrial 1-Carbon Pathway Enzymes*

One striking example of paralogous enzymes includes those required for the methylenetetrahydrofolate dehydrogenase/cyclohydrolase reactions in the mitochondrial 1-carbon pathway. There are three enzymes which catalyze this reaction, methylenetetrahydrofolate dehydrogenase (NAD+ dependent), methenyltetrahydrofolate cyclohydrolase 2 (MTHFD2), MTHFD1 and MTHFD2L, further described below:

MTHFD2 is a bifunctional enzyme, localized to the mitochondria, that catalyzes both the dehydrogenase and cyclohydrolase reactions. Cofactors for MTHFD2 include NAD+, Mg2+, and inorganic phosphate. MTHFD2 is expressed in embryonic growth and in the transformed state (Mejia et al., The Journal of biological chemistry. 1985;260:14616-14620).

MTHFD1 is a trifunctional enzyme, localized to the cytosol, that catalyzes the dehydrogenase, cyclohydrolase and formyl-THF synthetase reactions. Cofactors for MTHFD1 include NADP+. MTHFD1 is ubiquitously expressed (Tibbetts and Appling, Annu Rev Nutr. 2010;30:57-81).

MTHFD2L is a bifunctional enzyme, localized to the mitochondria, that catalyzes both the dehydrogenase and cyclohydrolase reactions. Cofactors for MTHFD2L include NADP+. MTHFD2L is ubiquitously expressed in adult tissue; see Bolusani et al., The Journal of biological chemistry. 2011;286:5166-5174.

As mentioned, MTHFD2 has differential cofactor requirements and subcellular localization relative to its cytosolic paralog MTHFD1 and its adult paralog MTHFD2L. The different properties of MTHFD2 vs MTHFD1 vs MTHFD2L may be potentially exploited to inhibit MTHFD2 while sparing MTHFD1 and MTHFD2L. Methods to inhibit MTHFD2 selectively include delivery of small molecule agents specifically to the mitochondria through conjugation with tetraphenylphosphonium or related chemical moieties, and/or through identification of small molecule agents that may specially bind within the NAD+ versus NADP+ enzymatic pocket. The differential cofactor coupling suggests that the paralogous enzymes differ enough in their biology that selective inhibition of one enzyme should be possible. One such difference in biology between MTHFD2 and MTHFD1 is highlighted herein, i.e., the non-catalytic cysteine residues in MTHFD2 that are required for enzyme activity.

The sequence of human MTHFD2 is available in GenBank at Accession Nos. NM_006636.3 (nucleic acid) and NP_006627.2 (protein); MTHFD1 NM_005956.3 (nucleic acid) and NP_005947.3 (protein); MTHFD2L NM_001144978.1 (nucleic acid) and NP_001138450.1 (protein).

MTHFD1L is another member of this family; unlike the other bi- and trifunctional members, MTHFD1L only has formyltetrahydrofolate synthetase activity (Christensen et al., J. Biol. Chem. 280 (9): 7597-602 (2005)). There are four amino acid sequences for human MTHFD1L is NP_001229696.1, NP_001229697.1, NP_001229698.1, and NP_056255.2, which are expressed from four alternative transcripts, NM_001242767.1, NM_001242768.1, NM_001242769.1, and NM_015440.4.

Serine hydroxymethyltransferase 2 (mitochondrial), or SHMT2, is another enzyme involved in glycine synthesis. SHMT2 plays an important role in cellular one-carbon pathways by catalyzing the reversible, simultaneous conversions of L-serine to glycine (retro-aldol cleavage) and tetrahydrofolate to 5,10-methylenetetrahydrofolate (hydrolysis) (Fig. ID and Appaji Rao et al., Biochim Biophys Acta. 2003 Apr 11;1647(1-2):24-9). This reaction provides the majority of the one-carbon units available to the cell (Stover et al., J. Biol. Chem. 265 (24): 14227-33).

There are five isoforms of human SHMT2, with the sequences of NP_001159828.1, NP_001159829.1, NP_001159830.1, NP_001159831.1, or NP_005403.2 , encoded by five alternative transcripts: NM_001166356.1, NM_001166357.1, NM_001166358.1, NM_001166359.1, or NM_005412.5.

**Assays for Mitochondrial 1-Carbon Enzymes**

A number of methods known in the art can be used to detect levels of a protein, mRNA, or enzyme activity for the purposes of the present disclosure. For example, in some of the methods of the disclosure described herein, the level, presence or absence of protein, mRNA, or activity of one, two or all three of the mitochondrial glycine synthesis enzymes, SHMT2, MTHFD2 and/or MTHFD1L, is determined in a sample from the subject.

In some embodiments, the level of mRNA (transcript) can be evaluated using methods known in the art, e.g., Northern blot, RNA in situ hybridization (RNA-ISH), RNA expression assays, e.g., microarray analysis, RT-PCR, RNA sequencing (e.g., using random primers or oligoT primers), deep sequencing, cloning, Northern blot, and amplifying the transcript, e.g., using quantitative real time polymerase chain reaction (qRT-PCR). Analytical techniques to determine RNA expression are known. See, e.g., Sambrook et al., Molecular Cloning: A Laboratory Manual, 3rd Ed., Cold Spring Harbor Press, Cold Spring Harbor, NY (2001).

Any method known in the art can be used for detecting the presence of proteins (e.g., using one or more antibodies that specifically bind to a biomarker as described herein). For example, a sample can be contacted with one or more antibodies or antigenic portions thereof that specifically bind to SHMT2, MTHFD2 or MTHFD1L; the binding of the one or more antibodies to proteins present in the sample can be detected using methods known in the art. Antibodies that bind specifically to SHMT2, MTHFD2 or MTHFD1L are known in the art and commercially available, e.g., from AbD Serotec; Thermo Fisher Scientific, Inc.; Proteintech Group; Biorbyt; NovaTeinBio; Aviva Systems Biology; United States Biological; Creative Biomart; Fitzgerald; Novus Biologicals; R&D Systems; and Abcam.

Where desired, any protein isolation methods described herein or known in the art can be used before the sample is contacted with the antibody or antigenic portion thereof.

Methods for detecting binding of the antibodies to target proteins are known in the art, and can include the use of secondary antibodies. The secondary antibodies are generally modified to be detectable, e.g., labeled. The term "labeled" is intended to encompass direct labeling by coupling (i.e., physically linking) a detectable substance to the secondary antibody, as well as indirect labeling of the multimeric antigen by reactivity with a detectable substance. Examples of detectable substances include various enzymes, prosthetic groups, fluorescent materials, luminescent materials, bioluminescent materials, and radioactive materials. Examples of suitable enzymes include horseradish peroxidase (HRP), alkaline phosphatase, β-galactosidase, and acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, and quantum dots, dichlorotriazinylamine fluorescein, dansyl chloride, and phycoerythrin; an example of a luminescent material includes luminol; examples of bioluminescent materials include green fluorescent protein and variants thereof, luciferase, luciferin, and aequorin; and examples of suitable radioactive material include $^{121}I$, $^{131}I$, $^{35}S$, or $^{3}H$. Methods for producing such labeled antibodies are known in the art, and many are commercially available.

Any method of detecting proteins present in a sample can be used, including but not limited to radioimmunoassays (RIA), enzyme-linked immunosorbent assays (ELISA), Western blotting, surface plasmon resonance, microfluidic devices, protein array, protein purification (e.g., chromatography, such as affinity chromatography), mass spectrometry, two-dimensional gel electrophoresis, or other assays as known in the art.

In some aspects of the methods of the disclosure described herein, an assay comprises providing one or more antibodies that specifically bind to SHMT2, MTHFD2 or MTHFD1L, contacting the antibodies with a sample comprising proteins from a tumor cell from the subject, and the binding of the antibodies to any SHMT2, MTHFD2 or MTHFD1L proteins present in the sample can be detected. Alternatively, an assay can comprises providing one or more nucleic acid probes that specifically bind to SHMT2, MTHFD2 or MTHFD1L, contacting the antibodies with the sample comprising nucleic acids from a tumor cell from the subject, and the binding of the probes to any SHMT2, MTHFD2 or MTHFD1L mRNA present in the sample can be detected.

In some embodiments, high throughput methods, e.g., protein or gene chips as are known in the art (see, e.g., Ch. 12, "Genomics," in Griffiths et al., Eds. Modern genetic Analysis, 1999,W. H. Freeman and Company; Ekins and Chu, Trends in Biotechnology, 1999;17:217-218; MacBeath and Schreiber, Science 2000, 289(5485):1760-1763; Simpson, Proteins and Proteomics: A Laboratory Manual, Cold Spring Harbor Laboratory Press; 2002; Hardiman, Microarrays Methods and Applications: Nuts & Bolts, DNA Press, 2003), can be used to detect the presence and/or level of satellites or SCG.

In some aspects of the disclosure, assays that detect SHMT2, MTHFD2 or MTHFD1L activity, e.g., in tumor samples, can be used. Any assay known in the art or described herein can be used. For example, to measure MTHFD2 activity *in vitro,* enzyme immunohistochemistry, e.g., an assay of NAD-dependent methylenetetrahydrofolate dehydrogenase activity as known in the art or described herein (see, e.g., Example 12), can be used. To measure SHMT2 activity, an assay as described in Hebbring et al., e.g., a modification of that published by Taylor and Weissbach (Anal. 1 Biochem. 1965;13:80-84) as modified by Zhang et al (Anal Biochem. 2008 Apr 15; 375(2):367-9), can be used.

**Samples**

The present methods can be performed using samples obtained from a subject, e.g., a mammalian subject, preferably a human subject. A sample (e.g., a sample containing a cancer or tumor cell, or a cell suspected to be a cancer or tumor cell) can be collected from a subject (e.g., subject who is known to or suspected to have cancer) at any time, e.g., during a routine annual physical, during an evaluation specifically to detect possible malignancy, or during an evaluation to stage a previously identified malignancy. In some embodiments, the sample includes known or suspected tumor cells, e.g., is a biopsy sample, e.g., a fine needle aspirate (FNA), endoscopic biopsy, or core needle biopsy; in some embodiments the sample comprises cells from a pancreatic, lung, breast, prostate, renal, ovarian or colon tumor of the subject. In some embodiments, the sample comprises lung cells obtained from a sputum sample or from the lung of the subject by brushing, washing, bronchoscopic biopsy, transbronchial biopsy, or FNA, e.g., bronchoscopic, fluoroscopic, or CT-guided FNA (such methods can also be used to obtain samples from other tissues as well). In some embodiments, the sample is frozen, fixed and/or permeabilized, e.g., is a formalin-fixed paraffin-embedded (FFPE) sample. Samples can be used immediately or frozen or stored for a period of time (e.g., at least one day, two days, three days, four days, five days, six days, 1 week or several months) prior to use, e.g., prior to detecting/determining the presence or absence of one or more biomarkers (e.g., MTHFD2 levels or glycine consumption levels) as described herein.

**Diagnostic and Prognostic Biomarkers of Cancer**

As described herein, glycine uptake is a predictor of cancer cell proliferation rates (Figs. 1A-B); determining or monitoring glycine uptake activity in subject in vivo, or in a sample comprising tumor cells from the subject, can be used to determine how aggressive or rapidly proliferating a tumor might be. To predict whether a subject's tumor is likely to be aggressive or rapidly growing, glycine uptake levels are measured and compared to reference levels; uptake levels above the reference level indicate that the tumor is likely to be aggressive.

In addition, the three gene expression signature (SHMT2, MTHFD2, and MTHFD1L), corresponding to mitochondrial 1-carbon (1-C), is a predictor of cancer cell proliferation (Figs. 1C-D). Measurement of the expression of the three genes sample, e.g., comprising biopsy material, can thus be used to predict how aggressive or rapidly proliferating a tumor might be. To predict whether a subject's tumor is likely to be aggressive or rapidly growing, expression levels of the three genes are measured and compared to reference levels; levels of the three genes above the reference level indicate that the tumor is likely to be aggressive.

The three gene expression signature (SHMT2, MTHFD2, and MTHFD1L) is also a predictor of breast cancer survival (Figs. 2A-B). Measurement of the expression levels of the three genes in a sample, e.g., comprising biopsy material, can be used to predict survival. To predict whether a subject's is likely to survive longer than a predetermined period, e.g., six months, or one, two, three, four, or five years, expression levels of the three genes are measured and compared to reference levels; levels of the three genes above the reference level indicate that the subject is less likely to survive longer than the predetermined period than is a subject who has a level at or below the reference level.

In addition, as demonstrated herein, MTHFD2 is highly differentially expressed in cancer versus normal cells. This differential expression makes MTHFD2 by itself an excellent biomarker. Furthermore, expression of MTHFD2 may be used as prognostic marker in cancer, e.g., in carcinoma, e.g., in breast, colon and renal cell cancer. MTHFD2 RNA levels, protein levels, or enzyme activity can be measured as described above, e.g., in biopsy specimens to serve as predictors of survival. To determine whether a subject has cancer, or is at risk of developing cancer, or to provide a prognosis, the level or activity of MTHFD2 in the sample from a subject can be compared to a reference level of MTHFD2. The presence of a level of MTHFD2 above the reference level indicates that the subject has or is at an increased risk of developing cancer, or has a low likelihood of survival beyond a predetermined period, as compared to a subject who has a level at or below the reference level.

Thus, in some aspects of the methods of the disclosure described herein, the methods include comparing a detected level of a SHMT2, MTHFD2, and/or MTHFD1L protein, transcript, or activity (e.g., SHMT2, MTHFD2, and MTHFD1L enzyme activity or glycine uptake activity) to a reference level.

In some embodiments, the reference represents levels of the protein, transcript, or activity in a non-cancerous cell of the same type in the subject from whom the test sample is taken. In some embodiments, the reference represents levels of protein, transcript, or activity in a healthy control, i.e., a subject who has not been diagnosed with or is not at risk of developing a cancer, or who has a good likelihood of survival past a predetermined period. In some embodiments of the methods described herein, the reference level represents levels of protein, transcript, or activity in a cancer control subject, i.e., a subject diagnosed with a cancer, e.g., a carcinoma, adenocarcinoma, lung cancer or ovarian cancer. In certain embodiments, the cancer control is from a subject having lung cancer or ovarian cancer.

In some embodiments, the reference level is a threshold level in a subject who has cancer and who has a predetermined likelihood of survival, and the presence of a level above that threshold indicates that the subject has less than that predetermined likelihood of survival, e.g., survival for 6 months, 1 year, 2 years, 5 years, or more.

In some embodiments, the reference level is a median or cutoff level in a reference cohort, e.g., a cutoff defining a statistically significantly distinct group, e.g., a top or bottom tertile, quartile, quintile, or other percentile of a reference cohort. Levels above the reference level indicate the presence of disease or increased risk.

In some embodiments, levels above a reference level are statistically significant increased, or by at least 5%, 10%, 15%, 20%, 25%, 30%, 35%, 40%, 45%, 50%, 55%, 60%, 65%, 70%, 75%, 80%, 85%, 90%, 95%, 100%, 200$, 300%, 400%, 500%, or 1000%. An increase, as described herein, can be determined by comparison to a threshold or baseline value (e.g., a threshold detection level of an assay for determining the presence or absence of a protein, or a reference level of protein in a reference subject (e.g., healthy reference or a subject who has cancer, e.g., a known stage of cancer). In some embodiments, levels below a reference level are statistically significant decreased, or by at least 5%, 10%, 15%, 20%, 25%, 30%, 35%, 40%, 45%, 55%, 60%, 65%, 70%, 75%, 80%, 85%, 90%, or 95%. A decrease, as described herein, can be determined by comparison to a threshold or baseline value (e.g., a threshold detection level of an assay for determining the presence or absence of a protein, or a level of protein in a reference subject (e.g., a healthy reference subject or a subject who does not have cancer, e.g., does not have lung or ovarian cancer).

In some embodiments, the methods include calculating a ratio of the level of protein, transcript, or activity in the subject sample to a reference level, and if the ratio is greater than a threshold ratio, determining that the subject has or is at risk of developing a carcinoma as described herein, e.g., adenocarcinoma, e.g., lung or ovarian cancer. In some embodiments, whether the ratio is positive or negative is determined and the presence of a positive ratio indicates that the subject has or is at risk of developing a carcinoma as described herein, e.g., adenocarcinoma, e.g., lung or ovarian cancer.

**Predicting Drug Sensitivity**

As demonstrated herein, expression/activity of MTHFD2 or glycine consumption can be used as a predictor of toxicity (and thus efficacy) from methotrexate or other anti-folates, allowing selection of subjects for treatment with these drugs. Thus the methods of the disclosure described herein can include measuring glycine consumption levels, or MTHFD2 levels or activity, and comparing the levels to a reference or threshold level as described above, and in doing so, identify those patients that would benefit from therapy with an antifolate agent or an agent that inhibits MTHFD2. In these methods of the disclosure, the presence of glycine consumption levels, or levels of MTHFD2 expression and/or activity, above a selected reference or threshold level indicate that the subject is likely to benefit from therapy with an antifolate agent or an agent that inhibits MTHFD2; the methods can further include selecting the treatment and/or administering a therapeutically effective dose of the treatment to the subject.

Thus, in some aspects, the methods of the disclosure described herein include administering a therapeutically effective dose of an antifolate agent or an agent that inhibits MTHFD2. A number of antifolate agents are known in the art, including but not limited to methotrexate, pemetrexed, pralatrexate, raltitrexed, among others (e.g., those listed in Table 1). See, e.g., McGuire, Curr Pharm Des. 2003;9(31):2593-613; Gangjee et al., Anticancer Agents Med Chem. 2007 Sep;7(5):524-42; and Gonen and Assaraf, Drug Resist Updat. 2012 Aug;15(4):183-210.

**Use of Antifolate Drugs in Methods of Treating Cancer**

The invention provides antifolate drugs for use in methods for the treatment of cancer in a subject comprising (a) determining a level of SHMT2 protein, SHMT2 mRNA, or SHMT2 activity in a sample comprising tumor cells from a subject;

comparing the level of SHMT2 protein, SHMT2mRNA, or SHMT2 activity in the sample to a reference level of SHMT2 protein, SHMT2 mRNA, or SHMT2 activity;

selecting a subject who has a level of SHMT2 protein, SHMT2 mRNA, or SHMT2 activity above the reference level, and administering a therapeutically effective amount of an antifolate drug, wherein the antifolate drug is methotrexate,

or any one of the compounds recited in Table 1. As used in this context, to "treat" means to ameliorate or improve at least one symptom or clinical parameter of the cancer. For example, a treatment can result in a reduction in tumor size or growth rate. A treatment need not cure the cancer or cause remission 100% of the time, in all subjects.

As described herein as part of the disclosure, the application of agents, e.g., inhibitory nucleic acids or small molecules, that inhibit SHMT2 or MTHFD2 reduces cancer cell proliferation and thus treat cancer in subjects. Thus, in some aspects of the disclosure, the methods described herein include administering a therapeutically effective dose of one or more agents that inhibit a mitochondrial 1-carbon (1-C) pathway enzyme, e.g., SHMT2, MTHFD2, and/or MTHFD1L. Such drugs include those identified herein, e.g., those small molecules that target MTHFD2 as described herein, e.g., 6-hydroxy-DL DOPA, calmidazolium chloride, CDOO, ebselen, celastrol, GW5074, iodoacetamide, para-benzoquinone, and protoporphyrin IX disodium, as well as inhibitory nucleic acids that inhibit SHMT2, MTHFD2, and/or MTHFD1L, e.g., preferably SHMT2 or MTHFD2.

*Small Molecule Inhibitors and Targeting the Mitochondrial Compartment*

In some aspects of the disclosure, the drugs are targeted for delivery to the mitochondria, e.g., by conjugation to a mitochondrial penetrating moiety. Such moieties are known in the art and include a mitochondria penetrating peptide, e.g., a mitofusin peptide, a mitochondrial targeting signal peptide, Antennapedia helix III homeodomain cell-penetrating peptide (ANT), HIV-1 Tat basic domain; VP22 peptide, or Pep-1 peptide; an RNA mitochondrial penetrating signal; guanidine-rich peptoids, guanidine-rich polycarbamates, beta-oligoarginines, proline-rich dendrimers, and phosphonium salts, e.g., methyltriphenylphosphonium and tetraphenylphosphonium.

Thus, MTHFD2 may be selectively inhibited relative to its cytosolic counterpart MTHFD1 or to its adult mitochondrial counterpart MTHFD2L, e.g., using drugs such as small molecules that are conjugated to accumulate within mitochondria [13], as with the example of the recently described tetraphenylphosphonium-conjugated ebselen[14].

Other methods of the disclosure for treating subjects with cancer, e.g., subjects who have levels of glycine uptake, or expression of mitochondrial 1-carbon (1-C) pathway enzymes (SHMT2, MTHFD2, and MTHFD1L), above a reference level, include the administration of drugs that affect other mitochondrial enzymes that have been modified to be targeted to the mitochondria. For example, antifolate inhibitors of DHFR (methotrexate, pemetrexed, etc) or thymidylate synthetase (TYMS) inhibitors (5-fluorouracil) can be targeted to the mitochondria as described herein, e.g., through coupling with a mitochondrial-localizing moiety including tetraphenylphosphonium or related chemical moieties, given that the mitochondria contains the DHFR paralog DHFRL1 or a mitochondrial localized TYMS. Peptide conjugates of methotrexate, which result in localization of the agent to the mitochondria, have previously been described; a mitochondria-specific version of Mtx (mt-Mtx) for use as an antimicrobial was generated by coupling the drug to the N-terminus of a peptide consisting of 3 repeating units of cyclohexylalanine and d-arginine, see Figure 1B of Pereira et al., J Am Chem Soc. 133(10):3260-3 (2011).

Alternatively, MTHFD2 can be selectively targeted using drugs that antagonize the selective NADH, Mg, or phosphate cofactor requirement of MTHFD2; or selectively modifying the non-catalytic cysteine residues, as described for other enzyme inhibitors [15,16]. Drug screening efforts are currently underway to identify inhibitors of enzymes, notably kinases, through reversible and irreversible covalent inhibitors of noncatalytic cysteines. Two recent publications [15,16] highlight these efforts in cancer chemotherapeutics. The methods exemplified by these two papers, in combination with the insights from the cysteine mutagenesis studies and evaluation in the presence of cysteine-modifying agents described herein, could be exploited to design new drugs that specifically inhibit MTHFD2 with greater potency and specificity.

Two non-catalytic cysteine residues were identified in MTHFD2: Cys 145 and Cys166. Experiments described herein have identified the cysteine residue at 145 as a critical cysteine. With catalytic cysteine residues, mutation of the cysteine to serine results in complete loss of enzymatic activity, as has previously been shown (Ziegler et al., Biochemistry. 2007;46(10):2674-83; Parker et al., Mamm Genome. 2010; 21(11-12):565-76). In the C145S MTHFD2 mutant protein, activity is retained albeit at a slightly lower value (Kcat: wild type MTHFD2 $2.57 \pm 0.07$; C145S mutant MTHFD2 $1.31 \pm 0.07$ 1/sec), confirming at C145 is a non-catalytic cysteine. In addition, the C145S mutant protein is resistant to inhibition by cysteine modifying agents, including ebselen and celastrol (Figs. 9B-9C), suggesting that this non-catalytic cysteine can be targeted as a means for inhibiting MTHFD2 activity. The non-catalytic nature of C145 is quite advantageous from a drug development perspective, as non-catalytic cysteine residues are typically not conserved among related enzymes within a given enzyme class; this is indeed the case with MTHFD2 and MTHFD1, allowing for selective targeting of particular enzymes through these non-catalytic cysteines, as has been described previously (Singh et al., Nature reviews. Drug discovery. 2011;10:307-317).

Therefore, without wishing to be bound by any particular theory, it is believed that covalent modification of MTHFD2 at one or both of Cys145 or Cys166 will result in selective inhibition of MTHFD2 as compared with other related enzymes (e.g., MTHFD1). Thus, in certain aspects, the present disclosure provides a method of covalently binding to one or both of Cys145 or Cys166 of MTHFD2 thereby irreversibly inhibiting MTHFD2. In some aspects, the present disclosure provides a method of selectively inhibiting MTHFD2 as compared to MTHFD1 comprising covalently binding to one or both of Cys145 or Cys166 of MTHFD2 thereby irreversibly inhibiting MTHFD2. Exemplary methods are described at Example 13, *infra.*

In certain aspects, the present disclosure provides a conjugate of the formula **A:**

**Cys145-modifier-inhibitor moiety** **A**

wherein:

Cys145 is Cys145 of MTHFD2;
the inhibitor moiety is a moiety that binds in the binding site of MTHFD2;
the modifier is a bivalent group resulting from covalent bonding of a an inhibitor moiety with the Cys145 of MTHFD2; and
the inhibitor moiety comprises a functional group capable of covalently or non-covalently binding to Cys145.

In certain aspects, the present disclosure provides a conjugate of the formula **B:**

**Cys166-modifier-inhibitor moiety** **B**

wherein:

Cys166 is Cys166 of MTHFD2;
the inhibitor moiety is a moiety that binds in the binding site of MTHFD2;
the modifier is a bivalent group resulting from covalent or non-covalent bonding of a an inhibitor moiety with the Cys166 of MTHFD2; and
the inhibitor moiety comprises a functional group capable of covalently or non covalently binding to Cys166.

Given that C145 is an essential non-catalytic cysteine residue, this allows for design of a targeted inhibitor of MTHFD2. Such a compound could be designed to bind non-covalently to MTHFD2, including through a natural ligand analogue, with a moderately reactive electrophile optimally placed at a mutual distance and orientation to be highly favorable for non-covalent or covalent interaction with the cysteine 145 residue on MTHFD2 and formation of an inhibited complex. An agent that interacts with MTHFD2 can be identified or designed by a method that includes using a representation of the MTHFD2 or a fragment thereof, or a complex of MTHFD2 bound to a test compound or a fragment of either one of these complexes.

Various software programs allow for the graphical representation of a set of structural coordinates to obtain a representation of a complex of the MTHFD2 bound to a test compound, or a fragment of one of these complexes. In general, such a representation should accurately reflect (relatively and/or absolutely) structural coordinates, or information derived from structural coordinates, such as distances or angles between features. In some aspects of the disclosure, the representation is a two-dimensional figure, such as a stereoscopic two-dimensional figure. In certain aspects of the disclosure, the representation is an interactive two-dimensional display, such as an interactive stereoscopic two-dimensional display. An interactive two-dimensional display can be, for example, a computer display that can be rotated to show different faces of a polypeptide, a fragment of a polypeptide, a complex and/or a fragment of a complex. In some aspects of the disclosure, the representation is a three-dimensional representation. As an example, a three-dimensional model can be a physical model of a molecular structure (*e.g.*, a ball-and-stick model). As another example, a three dimensional representation can be a graphical representation of a molecular structure (*e.g.*, a drawing or a figure presented on a computer display). A two-dimensional graphical representation (*e.g.*, a drawing) can correspond to a three-dimensional representation when the two-dimensional representation reflects three-dimensional information, for example, through the use of perspective, shading, or the obstruction of features more distant from the viewer by features closer to the viewer. In some aspects of the disclosure, a representation can be modeled at more than one level. As an example, when the three-dimensional representation includes a polypeptide, such as a complex of the MTHFD2 bound to a test compound, the polypeptide can be represented at one or more different levels of structure, such as primary (amino acid sequence), secondary (*e.g.*, I-helices and ϑ-sheets), tertiary (overall fold), and quaternary (oligomerization state) structure. A representation can include different levels of detail. For example, the representation can include the relative locations of secondary structural features of a protein without specifying the positions of atoms. A more detailed representation could, for example, include the positions of atoms.

In some aspects of the disclosure, a representation can include information in addition to the structural coordinates of the atoms in a complex of the MTHFD2 bound to a test compound. For example, a representation can provide information regarding the shape of a solvent accessible surface, the van der Waals radii of the atoms of the model, and the van der Waals radius of a solvent (*e.g.*, water). Other features that can be derived from a representation include, for example, electrostatic potential, the location of voids or pockets within a macromolecular structure, and the location of hydrogen bonds and salt bridges.

A software system can be designed and/or implemented to facilitate these steps. Software systems (e.g., computer programs) used to generate representations or perform the fitting analyses include, for example: MCSS, Ludi, QUANTA, Insight II, Cerius2, CHarMM, and Modeler from Accelrys, Inc. (San Diego, CA); SYBYL, Unity, FleXX, and LEAPFROG from TRIPOS, Inc. (St. Louis, MO); AUTODOCK (Scripps Research Institute, La Jolla, CA); GRID (Oxford University, Oxford, UK); DOCK (University of California, San Francisco, CA); and Flo+ and Flo99 (Thistlesoft, Morris Township, NJ). Other useful programs include ROCS, ZAP, FRED, Vida, and Szybki from Openeye Scientific Software (Santa Fe, NM); Maestro, Macromodel, and Glide from Schrodinger, LLC (Portland, OR); MOE (Chemical Computing Group, Montreal, Quebec), Allegrow (Boston De Novo, Boston, MA), and GOLD (Jones et al., J. Mol. Biol. 245:43-53, 1995). The structural coordinates can also be used to visualize the three-dimensional structure of an ERalpha polypeptide using MOLSCRIPT, RASTER3D, or PYMOLE (Kraulis, J. Appl. Crystallogr. 24: 946-950, 1991; Bacon and Anderson, J. Mol. Graph. 6: 219-220, 1998; DeLano, The PyMOL Molecular Graphics System (2002) DeLano Scientific, San Carlos, CA).

The agent can, for example, be selected by screening an appropriate database, can be designed de novo by analyzing the steric configurations and charge potentials of unbound MTHFD2 in conjunction with the appropriate software systems, and/or can be designed using characteristics of known ligands of progesterone receptors or other hormone receptors. The method can be used to design or select agonists or antagonists of MTHFD2. A software system can be designed and/or implemented to facilitate database searching, and/or agent selection and design.

Once an agent has been designed or identified, it can be obtained or synthesized and further evaluated for its effect on MTHFD2 activity. For example, the agent can be evaluated by contacting it with MTHFD2 and measuring the effect of the agent on polypeptide activity. A method for evaluating the agent can include an activity assay performed in vitro or in vivo. An activity assay can be a cell-based assay, for example as described herein, and agents that inhibit MTHFD2 selected. A crystal containing MTHFD2 bound to the identified agent can be grown and the structure determined by X-ray crystallography. A second agent can be designed or identified based on the interaction of the first agent with MTHFD2.

Various molecular analysis and rational drug design techniques are further disclosed in, for example, U.S. Patent Nos. 5,834,228, 5,939,528 and 5,856,116, as well as in PCT Application No. PCT/US98/16879, published as WO 99/09148.

Alternatively, a mechanism based inhibitor can be designed in which the MTHFD2 reaction converts an unreactive ligand into a highly reactive ligand which then may target the cysteine 145 residue. These approaches allow for development of a highly potent and selective targeted inhibitor for MTHFD2, without antagonism of the paralog MTHFD1. Such approaches have been found to result in development of highly potent and selective enzymatic inhibitors (Wissner et al., J Med Chem. 2003;46(1):49-63; Ahn et al., Chem Biol. 2009;16(4):411-20).

*Reduction of Glycine Levels*

In addition, deprivation of the non-essential amino acid glycine blunts rapid cancer cell proliferation (Figs. 1F-G). Thus, the methods of the disclosure described herein can include prescribing a low-glycine diet or administration of sodium benzoate, e.g., to a subject with cancer, e.g., a subject who has levels of glycine uptake, or expression of mitochondrial 1-carbon (1-C) pathway enzymes (SHMT2, MTHFD2, and MTHFD1L), above a reference level. The methods of the disclosure can include treating or identifying a subject for treatment with a low-glycine diet or administration of sodium benzoate by determining levels of glycine uptake, or levels or expression of mitochondrial 1-carbon (1-C) pathway enzymes (SHMT2, MTHFD2, and MTHFD1L), comparing the levels to reference levels, selecting a subject who has levels of levels of glycine uptake, or expression of mitochondrial 1-carbon (1-C) pathway enzymes (SHMT2, MTHFD2, and MTHFD1L) above the reference levels, and optionally administering the treatment to the subject. Sodium benzoate or derivatives thereof can be administered, see, e.g., US8198328; Sun and Hai Liu, Cancer Lett. 2006 Sep 8;241(1):124-34; or Neto et al., Mol Nutr Food Res. 2008 Jun;52 Suppl 1:S18-27.

*Inhibitory Nucleic Acids*

Finally, the methods of treatment of the disclosure can include administration of compositions comprising inhibitory nucleic acid molecules that are designed to inhibit a target RNA, e.g., antisense, siRNA, ribozymes, and aptamers. The methods of the disclosure can include inhibiting any one or more of SHMT2, MTHFD2, and MTHFD1L; in preferred aspect of the disclosure, the inhibitory nucleic acids inhibit SHMT2.

*siRNA Molecules*

RNAi is a process whereby double-stranded RNA (dsRNA) induces the sequence-specific degradation of homologous mRNA in mammalian cells.

The nucleic acid molecules or constructs can include dsRNA molecules comprising 16-30, e.g., 16, 17, 18, 19, 20, 21, 22, 23, 24, 25, 26, 27, 28, 29, or 30 nucleotides in each strand, wherein one of the strands is substantially identical, e.g., at least 80% (or more, e.g., 85%, 90%, 95%, or 100%) identical, e.g., having 3, 2, 1, or 0 mismatched nucleotide(s), to a target region in the mRNA, and the other strand is complementary to the first strand. The dsRNA molecules can be chemically synthesized, or can be transcribed *in vitro* from a DNA template, or *in vivo* from, e.g., small hairpin RNAs (shRNAs). The dsRNA molecules can be designed using any method known in the art; a number of algorithms are known, and are commercially available. Gene walk methods can be used to optimize the inhibitory activity of the siRNA.

The nucleic acid compositions can include both siRNA and modified siRNA derivatives, e.g., siRNAs modified to alter a property such as the pharmacokinetics of the composition, for example, to increase half-life in the body, as well as engineered RNAi precursors.

siRNAs can be delivered into cells by methods known in the art, e.g., cationic liposome transfection and electroporation. siRNA duplexes can be expressed within cells from engineered RNAi precursors, e.g., recombinant DNA constructs using mammalian Pol III promoter systems (e.g., HI or U6/snRNA promoter systems (Tuschl (2002), *supra)* capable of expressing functional double-stranded siRNAs; (Bagella et al., J. Cell. Physiol. 177:206-213 (1998); Lee *et al.* (2002), *supra;* Miyagishi *et al.* (2002), *supra;* Paul *et al.* (2002), *supra;* Yu *et al.* (2002), *supra;* Sui *et al.* (2002), *supra).* Transcriptional termination by RNA Pol III occurs at runs of four consecutive T residues in the DNA template, providing a mechanism to end the siRNA transcript at a specific sequence. The siRNA is complementary to the sequence of the target gene in 5'-3' and 3'-5' orientations, and the two strands of the siRNA can be expressed in the same construct or in separate constructs. Hairpin siRNAs, driven by HI or U6 snRNA promoter and expressed in cells, can inhibit target gene expression (Bagella *et al.* (1998), *supra;* Lee *et al.* (2002), *supra;* Miyagishi *et al.* (2002), *supra;* Paul *et al.* (2002), *supra;* Yu *et al.* (2002), *supra;* Sui *et al.* (2002) *supra).* Constructs containing siRNA sequence under the control of T7 promoter also make functional siRNAs when cotransfected into the cells with a vector expression T7 RNA polymerase (Jacque (2002), *supra).*

*Antisense*

An "antisense" nucleic acid can include a nucleotide sequence that is complementary to a "sense" nucleic acid encoding a protein, e.g., complementary to the coding strand of a double-stranded cDNA molecule or complementary to a target mRNA sequence. The antisense nucleic acid can be complementary to an entire coding strand of a target sequence, or to only a portion thereof. In another aspect of the dislcousre, the antisense nucleic acid molecule is antisense to a "noncoding region" of the coding strand of a nucleotide sequence (e.g., the 5' and 3' untranslated regions).

An antisense nucleic acid can be designed such that it is complementary to the entire coding region of a target mRNA, but can also be an oligonucleotide that is antisense to only a portion of the coding or noncoding region of the target mRNA. For example, the antisense oligonucleotide can be complementary to the region surrounding the translation start site of the target mRNA, e.g., between the -10 and +10 regions of the target gene nucleotide sequence of interest. An antisense oligonucleotide can be, for example, about 7, 10, 15, 20, 25, 30, 35, 40, 45, 50, 55, 60, 65, 70, 75, 80, or more nucleotides in length.

An antisense nucleic acid can be constructed using chemical synthesis and enzymatic ligation reactions using procedures known in the art. For example, an antisense nucleic acid (e.g., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives and acridine substituted nucleotides can be used. The antisense nucleic acid also can be produced biologically using an expression vector into which a nucleic acid has been subcloned in an antisense orientation (i.e., RNA transcribed from the inserted nucleic acid will be of an antisense orientation to a target nucleic acid of interest, described further in the following subsection).

Based upon the sequences disclosed herein, one of skill in the art can easily choose and synthesize any of a number of appropriate antisense molecules for use in accordance with the present disclosure. For example, a "gene walk" comprising a series of oligonucleotides of 15-30 nucleotides spanning the length of a target nucleic acid can be prepared, followed by testing for inhibition of target gene expression. Optionally, gaps of 5-10 nucleotides can be left between the oligonucleotides to reduce the number of oligonucleotides synthesized and tested.

In some aspects of the disclosure, the antisense nucleic acid molecule is an alpha-anomeric nucleic acid molecule. An alpha-anomeric nucleic acid molecule forms specific double-stranded hybrids with complementary RNA in which, contrary to the usual $\vartheta$-units, the strands run parallel to each other (Gaultier et al., Nucleic Acids. Res.

15:6625-6641 (1987)). The antisense nucleic acid molecule can also comprise a 2'-o-methylribonucleotide (Inoue et al. Nucleic Acids Res. 15:6131-6148 (1987)) or a chimeric RNA-DNA analogue (Inoue et al. FEBS Lett., 215:327-330 (1987)).

In some aspects of the disclosure, the antisense nucleic acid is a morpholino oligonucleotide (see, e.g., Heasman, Dev. Biol. 243:209-14 (2002); Iversen, Curr. Opin. Mol. Ther. 3:235-8 (2001); Summerton, Biochim. Biophys. Acta. 1489:141-58 (1999).

Target gene expression can be inhibited using nucleotide sequences complementary to a regulatory region (e.g., promoters and/or enhancers) to form triple helical structures that prevent transcription of the Spt5 gene in cells. See generally, Helene, C. Anticancer Drug Des. 6:569-84 (1991); Helene, C. Ann. N.Y. Acad. Sci. 660:27-36 (1992); and Maher, Bioassays 14:807-15 (1992). The potential sequences that can be targeted for triple helix formation can be increased by creating a so called "switchback" nucleic acid molecule. Switchback molecules are synthesized in an alternating 5'-3', 3'-5' manner, such that they base pair with first one strand of a duplex and then the other, eliminating the necessity for a sizeable stretch of either purines or pyrimidines to be present on one strand of a duplex.

*Ribozymes*

Ribozymes are a type of RNA that can be engineered to enzymatically cleave and inactivate other RNAs in a specific, sequence-dependent fashion. By cleaving the target RNA, ribozymes inhibit translation, thus preventing the expression of the encoded gene. Ribozymes can be chemically synthesized in the laboratory and structurally modified to increase their stability and catalytic activity using methods known in the art. Alternatively, ribozyme genes can be introduced into cells through gene-delivery mechanisms known in the art. A ribozyme having specificity for a target nucleic acid can include one or more sequences complementary to the nucleotide sequence of a cDNA described herein, and a sequence having known catalytic sequence responsible for mRNA cleavage (see U.S. Pat. No. 5,093,246 or Haselhoff and Gerlach Nature 334:585-591 (1988)). For example, a derivative of a Tetrahymena L-19 IVS RNA can be constructed in which the nucleotide sequence of the active site is complementary to the nucleotide sequence to be cleaved in a target mRNA. See, e.g., Cech et al. U.S. Patent No. 4,987,071; and Cech et al. U.S. Patent No. 5,116,742. Alternatively, a target mRNA can be used to select a catalytic RNA having a specific ribonuclease activity from a pool of RNA molecules. See, e.g., Bartel and Szostak, Science 261:1411-1418 (1993).

*Aptamers*

Aptamers are short oligonucleotide sequences which can specifically bind specific proteins. It has been demonstrated that different aptameric sequences can bind specifically to different proteins, for example, the sequence GGNNGG where N=guanosine (G), cytosine (C), adenosine (A) or thymidine (T) binds specifically to thrombin (Bock et al (1992) Nature 355: 564 566 and U.S. Pat. No. 5,582,981 (1996) Toole et al). Methods for selection and preparation of such RNA aptamers are knotn in the art (see, e.g., Famulok, Curr. Opin. Struct. Biol. 9:324 (1999); Herman and Patel, J. Science 287:820-825 (2000)); Kelly et al., J. Mol. Biol. 256:417 (1996); and Feigon et al., Chem. Biol. 3: 611 (1996)).

*Administration of Inhibitory Nucleic Acid Molecules*

The inhibitory nucleic acid molecules of the disclosure directed against MTHFD2 or SHMT2 described herein can be administered to a subject (e.g., by direct injection at a tissue site), or generated *in situ* such that they hybridize with or bind to cellular mRNA and/or genomic DNA encoding an MTHFD2 or SHMT2 protein to thereby inhibit expression of the protein, e.g., by inhibiting transcription and/or translation. Alternatively, inhibitory nucleic acid molecules can be modified to target selected cells and then administered systemically. For systemic administration, inhibitory nucleic acid molecules can be modified such that they specifically bind to receptors or antigens expressed on a selected cell surface, e.g., by linking the inhibitory nucleic acid nucleic acid molecules to peptides or antibodies that bind to cell surface receptors or antigens. The inhibitory nucleic acid nucleic acid molecules can also be delivered to cells using the vectors described herein. To achieve sufficient intracellular concentrations of the inhibitory nucleic acid molecules, vector constructs in which the inhibitory nucleic acid nucleic acid molecule is placed under the control of a strong promoter can be used.

*Combination Treatments*

The methods of the disclosure described herein can also include administration of combinations of the treatments described herein, e.g., a combination of a glycine-reducing treatment such as low glycine diet or sodium benzoate or derivatives thereof, plus another treatment such as administration of an inhibitory nucleic acid as described herein, e.g., siRNA or antisense oligonucleotides that inhibit a mitochondrial 1-carbon (1-C) pathway enzyme, e.g., SHMT2 or MTHFD2.

## Cancer

As used herein, the term "cancer" refers to cells having the capacity for autonomous growth, i.e., an abnormal state or condition characterized by rapidly proliferating cell growth. The term is meant to include all types of cancerous growths or oncogenic processes, metastatic tissues or malignantly transformed cells, tissues, or organs, irrespective of histopathologic type or stage of invasiveness. The term "tumor" as used herein refers to cancerous cells, e.g., a mass of cancer cells.

Cancers that can be treated or diagnoses using the methods described herein include malignancies of the various organ systems, such as affecting lung, breast, thyroid, lymphoid, gastrointestinal, and genito-urinary tract, as well as adenocarcinomas which include malignancies such as most colon cancers, renal-cell carcinoma, prostate cancer and/or testicular tumors, non-small cell carcinoma of the lung, cancer of the small intestine and cancer of the esophagus.

In some aspects, the methods of the disclosure described herein are used for treating or diagnosing a carcinoma in a subject. The term "carcinoma" is art recognized and refers to malignancies of epithelial or endocrine tissues including respiratory system carcinomas, gastrointestinal system carcinomas, genitourinary system carcinomas, testicular carcinomas, breast carcinomas, prostatic carcinomas, endocrine system carcinomas, and melanomas. In some embodiments, the cancer is renal carcinoma or melanoma. Exemplary carcinomas include those forming from tissue of the cervix, lung, prostate, breast, head and neck, colon and ovary. The term also includes carcinosarcomas, e.g., which include malignant tumors composed of carcinomatous and sarcomatous tissues. An "adenocarcinoma" refers to a carcinoma derived from glandular tissue or in which the tumor cells form recognizable glandular structures.

The term "sarcoma" is art recognized and refers to malignant tumors of mesenchymal derivation.

In some aspects of the disclosure, the cancers that are treated by the antifolate drug described herein are cancers that have increased levels of glycine uptake or an increased expression or activity of a mitochondrial 1-c enzyme (e.g., SHMT2, MTHFD2, and/or MTHFD1L) relative to normal tissues or to other cancers of the same tissues; methods known in the art and described herein can be used to identify those cancers. In some aspects, the methods of the disclosure include obtaining a sample comprising cells of the cancer, determining the level of glycine uptake or protein, mRNA, or activity of one or more mitochondrial 1-c enzymes (e.g., SHMT2, MTHFD2, and/or MTHFD1L) in the sample, and administering a treatment as described herein (e.g., an antifolate or an agent that inhibits MTHFD2, e.g., ebselen). In some embodiments, the cancer is one that is shown herein to have increased levels of glycine uptake.

In some embodiments, the cancer is not breast cancer, or is not bladder cancer.

## Use of Pharmaceutical Compositions

In some embodiments, the use of an antifolate drug as defined in the claims include the administration of an antifolate, in a pharmaceutical composition. Pharmaceutical compositions typically include the active agent plus a pharmaceutically acceptable carrier. As used herein the language "pharmaceutically acceptable carrier" includes saline, solvents, dispersion media, coatings, antibacterial and antifungal agents, isotonic and absorption delaying agents, and the like, compatible with pharmaceutical administration.

Pharmaceutical compositions are typically formulated to be compatible with its intended route of administration. Examples of routes of administration include parenteral, e.g., intravenous, intradermal, subcutaneous, oral (e.g., inhalation), transdermal (topical), transmucosal, and rectal administration.

Methods of formulating suitable pharmaceutical compositions are known in the art, see, e.g., Remington: The Science and Practice of Pharmacy, 21st ed., 2005; and the books in the series Drugs and the Pharmaceutical Sciences: a Series of Textbooks and Monographs (Dekker, NY). For example, solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use can include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, NJ) or phosphate buffered saline (PBS). In all cases, the composition must be sterile and should be fluid to the extent that easy syringability exists. It should be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, sodium chloride in the composition. Prolonged absorption of the injectable compositions can be brought about by including in the composition an agent that delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle, which contains a basic dispersion medium and the required other ingredients from those enumerated above. In the case of sterile powders for the preparation of sterile injectable solutions, the preferred methods of preparation are vacuum drying and freeze-drying, which yield a powder of the active ingredient plus any additional desired ingredient from a previously sterile-filtered solution thereof.

Oral compositions generally include an inert diluent or an edible carrier. For the purpose of oral therapeutic administration, the active compound can be incorporated with excipients and used in the form of tablets, troches, or capsules, e.g., gelatin capsules. Oral compositions can also be prepared using a fluid carrier for use as a mouthwash. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds can be delivered in the form of an aerosol spray from a pressured container or dispenser that contains a suitable propellant, e.g., a gas such as carbon dioxide, or a nebulizer. Such methods include those described in U.S. Patent No. 6,468,798.

In some embodiments, the therapeutic compounds are prepared with carriers that will protect the therapeutic compounds against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Such formulations can be prepared using standard techniques, or obtained commercially, e.g., from Alza Corporation and Nova Pharmaceuticals, Inc. Liposomal suspensions (including liposomes targeted to selected cells with monoclonal antibodies to cellular antigens) can also be used as pharmaceutically acceptable carriers. These can be prepared according to methods known to those skilled in the art, for example, as described in U.S. Patent No. 4,522,811.

*Dosage*

The use of the antifolate drug as recited in the claims can include administration of an effective amount of an antifolate. An "effective amount" is an amount sufficient to effect beneficial or desired results. For example, a therapeutic amount is one that achieves the desired therapeutic effect. This amount can be the same or different from a prophylactically effective amount, which is an amount necessary to prevent onset of disease or disease symptoms. An effective amount can be administered in one or more administrations, applications or dosages. A therapeutically effective amount of a therapeutic compound (i.e., an effective dosage) depends on the therapeutic compounds selected. The compositions can be administered one from one or more times per day to one or more times per week; including once every other day. The skilled artisan will appreciate that certain factors may influence the dosage and timing required to effectively treat a subject, including but not limited to the severity of the disease or disorder, previous treatments, the general health and/or age of the subject, and other diseases present. Moreover, treatment of a subject with a therapeutically effective amount of the therapeutic compounds described herein can include a single treatment or a series of treatments.

Dosage, toxicity and therapeutic efficacy of the therapeutic compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit high therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC50 (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

**Methods of Screening Test Compounds**

Included herein as part of the disclosure are methods for screening test compounds, e.g., polypeptides, polynucleotides, inorganic or organic large or small molecule test compounds, to identify agents useful in the treatment of cancer, e.g., cancers associated with increased levels of MTHFD2.

As used herein, "small molecules" refers to small organic or inorganic molecules of molecular weight below about 3,000 Daltons. In general, small molecules useful for the invention have a molecular weight of less than 3,000 Daltons (Da). The small molecules can be, e.g., from at least about 100 Da to about 3,000 Da (e.g., between about 100 to about 3,000 Da, about 100 to about 2500 Da, about 100 to about 2,000 Da, about 100 to about 1,750 Da, about 100 to about 1,500 Da, about 100 to about 1,250 Da, about 100 to about 1,000 Da, about 100 to about 750 Da, about 100 to about 500 Da, about 200 to about 1500, about 500 to about 1000, about 300 to about 1000 Da, or about 100 to about 250 Da).

The test compounds can be, e.g., natural products or members of a combinatorial chemistry library. A set of diverse molecules should be used to cover a variety of functions such as charge, aromaticity, hydrogen bonding, flexibility, size, length of side chain, hydrophobicity, and rigidity. Combinatorial techniques suitable for synthesizing small molecules are known in the art, e.g., as exemplified by Obrecht and Villalgordo, Solid-Supported Combinatorial and Parallel Synthesis of Small-Molecular-Weight Compound Libraries, Pergamon-Elsevier Science Limited (1998), and include those such as the "split and pool" or "parallel" synthesis techniques, solid-phase and solution-phase techniques, and encoding techniques (see, for example, Czarnik, Curr. Opin. Chem. Bio. 1:60-6 (1997)). In addition, a number of small molecule libraries are commercially available. A number of suitable small molecule test compounds are listed in U.S. Patent No. 6,503,713.

Libraries screened using the methods of the present disclosure can comprise a variety of types of test compounds. A given library can comprise a set of structurally related or unrelated test compounds. In some aspects of the disclosure, the test compounds are peptide or peptidomimetic molecules. In some aspects of the disclosure, the test compounds are nucleic acids.

In some aspects of the disclosure, the test compounds and libraries thereof can be obtained by systematically altering the structure of a first test compound, e.g., a first test compound that is structurally similar to a known natural binding partner of the target polypeptide, or a first small molecule identified as capable of binding the target polypeptide, e.g., using methods known in the art or the methods described herein, and correlating that structure to a resulting biological activity, e.g., a structure-activity relationship study. As one of skill in the art will appreciate, there are a variety of standard methods for creating such a structure-activity relationship. Thus, in some instances, the work may be largely empirical, and in others, the three-dimensional structure of an endogenous polypeptide or portion thereof can be used as a starting point for the rational design of a small molecule compound or compounds. For example, in one aspects of the disclosure, a general library of small molecules is screened, e.g., using the methods of the disclosure described herein.

In some aspects of the disclosure, a test compound is applied to a test sample, e.g., a cell expressing MTHFD2, e.g., a normal cell or a cancer cell, and one or more effects of the test compound is evaluated, e.g., using a glycine metabolism assay or, preferably, an NAD-dependent methylenetetrahydrofolate dehydrogenase / cyclohydrolase activity assay in the absence of reducing agents including DTT or mercaptoethanol, which allows for detection of inhibition by cysteine modifying agents.

A test compound that has been screened by a method of the disclosure described herein and determined to decrease glycine metabolism or NAD-dependent methylenetetrahydrofolate dehydrogenase activity, can optionally be further tested, e.g., to determine whether the compound has effects on cancer cells (e.g., on viability or proliferation), and those that reduce proliferation or viability of cancer cells can be considered a candidate compound. A candidate compound that has been screened, e.g., in an in vivo model of a disorder, e.g., a tumor model (e.g., de novo or xenografted tumor model), and determined to have a desirable effect on the disorder, e.g., on one or more symptoms of the disorder (e.g., tumor size or growth rate), can be considered a candidate therapeutic agent. Candidate therapeutic agents, once screened in a clinical setting, are therapeutic agents. Candidate compounds, candidate therapeutic agents, and therapeutic agents can be optionally optimized and/or derivatized, and formulated with physiologically acceptable excipients to form pharmaceutical compositions.

Thus, test compounds identified as "hits" (e.g., test compounds that decrease glycine metabolism or NAD-dependent methylenetetrahydrofolate dehydrogenase activity, and that reduce cancer cell proliferation or viability, and optionally that have activity in an in vivo model) can be selected and systematically altered, e.g., using rational design, to optimize binding affinity, avidity, specificity, or other parameter. Such optimization can also be screened for using the methods described herein. Thus, in one aspect, the disclosure includes screening a first library of compounds using a method known in the art and/or described herein, identifying one or more hits in that library, subjecting those hits to systematic structural alteration to create a second library of compounds structurally related to the hit, and screening the second library using the methods described herein. Test compounds identified as hits can be considered candidate therapeutic compounds, useful in treating cancer, e.g., cancers associated with increased levels of MTHFD2. A variety of techniques useful for determining the structures of "hits" can be used in the methods described herein, e.g., NMR, mass spectrometry, gas chromatography equipped with electron capture detectors, fluorescence and absorption spectroscopy.

## EXAMPLES

The invention is further described in the following examples. Any examples falling outside the scope of the claims are provided for comparative purposes only.

### Materials and Methods

The following materials and methods were used in the Examples set forth below.

Cell Culture

NCI-60 low-passage cancer cell lines (Shoemaker, Nat Rev Cancer. 2006;6:813-823) were cultured in biological duplicates according to prior specifications (Shoemaker, Nat Rev Cancer. 2006;6:813-823) and under standard operating protocol. All 60 cell lines were grown in T-162 culture flasks (Costar) in 35 mL complete medium containing RPMI-1640 (GIBCO) with 2 mM L-glutamine and 5% fetal bovine serum (HyClone Laboratories), with the exception of the non-adherent cell lines SR, MOLT-4, HL-60(TB), K562, RPMI 8226, and CCRF-CEM, which were cultured in 50 mL of medium; and the cell lines NCI-H460, HCC-2998, and SW620, which were grown in T-75 flasks with 25mL of medium. Cells were maintained at 37° C, 5% $CO_2$, 95% air and 100% relative humidity for 4 or 5 days, with the culture duration selected to maintain cells under exponential growth and reach ~80% confluency.

For each cell line, 5 mL of spent medium was carefully aspirated from flasks to avoid contamination with cellular material, centrifuged at 1200 RPM x 10 minutes, the supernatant placed in cryovials and rapidly frozen at -80°C for subsequent metabolite analysis. Fresh medium was collected prior to addition to cells. Following aspiration of medium at the time of harvest, cells were trypsinized and counted using an automated cellometer to provide a final cell number. For non-adherent cell lines (SR, MOLT-4, HL-60(TB), K562, RPMI 8226, and CCRF-CEM), cultured cells was gently centrifuged at 1500 RPM x 5 minutes to pellet cells and medium aspirated and rapidly frozen as described above. Cells were subsequently resuspended and counted using a cellometer as described above. *In vitro* doubling times were reported for each cell line by the NCI (Shoemaker, Nat Rev Cancer. 2006;6:813-823)and confirmed by an independent study (O'Connor et al., Cancer Res. 1997;57:4285-4300). For all cell lines, tumor type annotations (tissue of origin) were provided by the NCI (Shoemaker, Nat Rev Cancer. 2006;6:813-823).

To confirm doubling times, HCT116, LOX IMVI, SF295, MCF7, A498, and HOP92 cells from the NCI-60 panel were cultured as described above and plated in 96-well microtiter plates at a plating density of 5,000 cells / well in 200 μL of medium. At selected time points, cells were washed with PBS and fixed using 4% paraformaldehyde for 20 minutes at room temperature. Cells were stained using Hoechst 33342 dye (Invitrogen) according to manufacturer's specifications, imaged using ImageXpress Micro (Molecular Devices), and counted using the "count nuclei" module of MetaXpress (Molecular Devices). To estimate doubling times, the exponential phase of the growth curve was analyzed by linear regression against the logarithm of cell number.

Metabolite CORE Profiling

Metabolites were profiled in medium samples using high performance liquid chromatography coupled to tandem mass spectrometry (LC-MS/MS). Two separate HPLC methods were employed, a hydrophilic interaction chromatography (HILIC) method (Alpert, J Chromatogr. 1990;499:177-196) to assess metabolites under positive ion MS conditions, including amino acids and biogenic amines, and a modified ion paring chromatography (IPR) method (Luo et al., J Chromatogr A. 2007;1147:153-164) to assess metabolites under negative ion MS conditions, including central metabolites and organic acids. For HILIC, medium samples were prepared using nine volumes (1/9 v/v) of extraction solution containing 75% acetonitrile, 25% methanol, and 0.2% formic acid, and vortexed. Samples were then centrifuged at 10,000 RPM x 10 minutes at 4°C and the supernatant separated for LC-MS/MS analysis as described below. For IPR method, medium samples were prepared using three volumes (1/3 v/v) of 100% methanol, and vortexed. Samples were then centrifuged at 10,000 RPM x 10 minutes at 4°C and the supernatant separated, nitrogen dried, and resuspended (9/1 v/v) in water. Resuspended samples were vortexed, centrifuged at 10,000 RPM x 10 minutes at 4°C and the supernatant separated for LC-MS/MS analysis as described below.

MS data were acquired using a 4000 QTRAP triple quadrupole mass spectrometer (AB SCIEX, Foster City, CA) equipped with an HTS PAL autosampler (Leap Technologies, Carrboro, NC) and an Agilent 1200 Series binary HPLC pump (Santa Clara, CA). HILIC separations were achieved using an Atlantis HILIC column (150 x 2.1 mm; Waters, Milford, MA) that was eluted at 250 μL/minute with a 10 minute linear gradient, initiated with 95% mobile phase B (acetonitrile with 0.1% formic acid, v/v) and concluding with 60% mobile phase A (10 mM ammonium formate and 0.1% formic acid, v/v). The modified IPR method was performed using an Atlantis T3 column (150 x 2.1 mm; Waters, Milford, MA). IPR mobile phase consisted of 10 mM tributylamine/15 mM acetic acid (mobile phase A) and methanol (mobile phase B), and the column was eluted at a flow rate of 300 μL/minute using the following program: 100% mobile phase A at initiation, 100% A at 4.0 minutes, 2% A at 34 minutes, and held at 2% mobile phase A to 39.0 minutes. Multiple reaction monitoring (MRM) was used to acquire targeted MS data for specific metabolites in the positive (HILIC method) and negative (IPR method) ion modes. Declustering potentials and collision energies were optimized for each metabolite by infusion of reference standards prior to sample analysis. The scheduled MRM algorithm in the Analyst 1.5 software (AB SCIEX; Foster City, CA) was used to automatically set dwell times for each transition.

MultiQuant software (Version 1.1; AB SCIEX; Foster City, CA) was used for manual review of chromatograms and peak area integration. For quality measures, all peaks were compared to known standards to confirm the metabolite identity. For metabolites assessed under both HILIC and IPR methods, only data from the method with best signal-to-noise characteristic was used in our analyses. Cell lines were analyzed in randomized order and peaks were integrated in a blinded fashion. Drift in MS peak area over the run order was normalized out for each metabolite by fitting a linear trend line to fresh medium samples (analyzed at regular intervals) using robust regression (minimizing the $L_1$ norm of residuals) and subtracting this trend line from all data points. The median coefficient of variation across all measured metabolites, as estimated from biological duplicates, was 5.5%. Quantitative measures of glucose and lactate were obtained for all samples using a calibrated commercial blood gas analyzer (Nova Biomedical; model *pHOx Plus L*). In total, 219 metabolites were monitored (Supplemental Table 1), of which 140 were present in either fresh medium or in spent medium from at least one cancer cell line, where "present" was defined as intensity greater than 3 times the background signal intensity. Of these 140 metabolites, 111 demonstrated reproducible variation across the 60 cell lines, defined as

$$\text{(standard deviation over all cell lines)} > 3 * \text{(pooled standard deviation of replicates)}.$$

Wherever possible, normalized MS data was calibrated against serial dilutions of standard analytes at known concentrations in buffer to determine absolute concentrations. To validate this calibration technique, we compared estimated concentrations with known concentrations of medium components, which revealed a median relative error of <10%. For 14 of the 111 metabolites, calibration data was unavailable; these were retained in arbitrary units for the purpose of metabolite clustering and correlative analyses, which are unaffected by the scale of measurement. For each spent medium sample and each calibrated metabolite, the measured concentration $c_{spent}$ was converted to consumption/release (CORE) data $\nu$ (molar amounts per cell per unit time) by subtracting the fresh medium concentration $c_{fresh}$, multiplying by the culture volume $V$ and normalizing to the area under the growth curve A for the corresponding cell,

$$\nu = \frac{V(c_{spent} - c_{fresh})}{A},$$

where the area under the growth curve A is given by

$$A = \int_0^T N(t)dt = \frac{N(T)\tau}{\ln 2}\left(1 - 2^{-T/\tau}\right),$$

here expressed as a function of the culture time *T,* the final cell count *N(T)*, and the doubling time $\tau$. Carbon consumption and release was calculated for each metabolite as the number of carbons per metabolite times the consumption/release of that metabolite (in molar amounts / time / cell).

The full CORE profiling dataset is available as Supplementary Data (sciencemag.org/content/suppl/2012/05/23/336.6084.1040.DC1/1218595databases1_ Corrected.xls) as well as through the NCI website (dtp.nci.nih.gov/index.html).

Cluster analysis of CORE data

Hierarchical agglomerative clustering of the metabolite CORE data was performed using the Pearson correlation distance with average linkage (Luo et al., Proc Natl Acad Sci USA. 1998;95:14863-14868). To avoid numerically large fluxes dominating the correlation coefficients, the metabolite data was scaled prior to clustering so that the maximum absolute value for each metabolite equals 1. For multi-dimensional scaling analysis, the Pearson correlation distances were projected into the 2-dimensional plane using the nonlinear stress minimization algorithm implemented in the R package SMACOF (de Leeuw and Mair, Journal of Statistical Software. 2009;31).

Isotope tracing

For isotope tracing studies 1.5 x $10^6$ LOX IMVI cells were grown in a 6 cm dish in RPMI 1640 medium (without unlabeled glycine) containing 2 mM glutamine, 5% dialyzed FBS (Hyclone) and 140 μM 1-$^{13}$C-glycine or 2-$^{13}$C-glycine (Cambridge Isotope Laboratories). At 18h, medium was rapidly removed and intracellular metabolites rapidly extracted by the addition of -80°C 100% methanol. For measurement of amino acids, samples were centrifuged at 10,000 RPM x 10 minutes at 4°C and the supernatant extracted using nine volumes (1/9 v/v) of an extraction solution containing 75% acetonitrile, 25% methanol, and 0.2% formic acid, recentrifuged at 10,000 RPM x 10 minutes at 4°C, and the supernatant separated and analyzed using HILIC LC-MS/MS, as described above. For assessment of labeled nucleotides and folates, metabolites were extracted with 100% methanol, samples were centrifuged at 10,000 RPM x 10 minutes at 4°C and the supernatant directly injected onto a ACQUITY UPLC (Waters Corp) equipped with a Luna NH2 column (5μm, 150 x 2 mm; Phenomenex). Initial mobile phase composition was 10% mobile phase A (aqueous 20 mM ammnoium acetate and 20 mM ammonium hydroxide) and 90% mobile phase B (10 mM ammonium hydroxide in 25% methanol/75% acetonitrile). The column was eluted at 0.4 mL/minute using a linear gradient to 100% mobile phase A over 10 minutes followed by isocratic flow of 100% mobile phase A for 2 minutes. MS/MS analysis was performed using a 5500 QTRAP triple quadrupole mass spectrometer in negative ion mode, utilizing an electrospray ionization source (AB SCIEX, Foster City, CA) with an ion spray voltage of -4.5 kV and a source temperature of 500°C. Declustering potentials and collision energies were tuned using unlabeled standards and data were collected using multiple reaction monitoring (MRM) scans.

Observed raw isotope spectra (MS/MS peak areas) were deconvoluted into relative isotope abundances by calculating theoretical spectra for MS/MS transitions based on the known natural abundances of the elements C,H,N and O and modeling the observed spectra as a mixture of unlabeled and labeled molecules, and fitting this model to the observed MS/MS intensities to obtain the proportions of labeled and unlabeled molecules, as previously described (Rantanen et al., Metab Eng. 2002;4:285-294). To estimate fluxes between serine and glycine, the model depicted in the figure below was used.

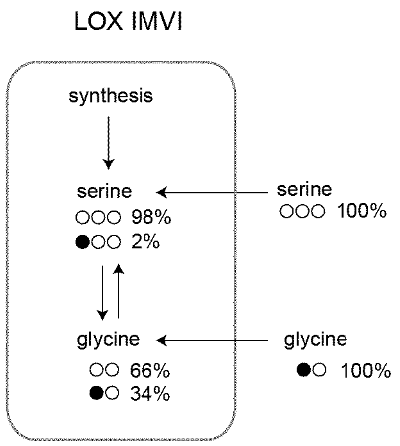


Using flux ratio analysis (Zamboni et al., Nat Protoc. 2009;4:878-892), this model yields the equations

$$x_{gly} = \frac{v_{\rightarrow gly} + v_{ser\rightarrow gly} x_{ser}}{v_{\rightarrow gly} + v_{ser\rightarrow gly}}$$

$$x_{ser} = \frac{v_{gly\rightarrow ser} x_{gly}}{v_{\rightarrow ser} + v_{gly\rightarrow ser}},$$

Where $x_{gly}$ is the fraction +1 isotope of intracellular glycine and $x_{ser}$ is the fraction +1 isotope of intracellular serine; $v_{ser\rightarrow gly}$ is the flux from serine to glycine; $v_{gly\rightarrow ser}$ is the flux from glycine to serine; $v_{\rightarrow gly}$ is the uptake rate of glycine; and $v_{\rightarrow ser}$ is the endogenous synthesis rate plus uptake rate of serine. Given the isotope data, we solve this equation for the fraction of glycine derived from serine,

$$\frac{v_{ser\rightarrow gly}}{v_{\rightarrow gly} + v_{ser\rightarrow gly}} = \frac{1 - x_{gly}}{1 - x_{set}}$$

and conversely, the fraction of serine derived from glycine,

$$\frac{v_{gly\rightarrow ser}}{v_{\rightarrow ser} + v_{gly\rightarrow ser}} = \frac{x_{ser}}{x_{gly}}.$$

RNAi silencing of SHMT2

Cells were cultured according to standard techniques as described above. Lentiviral vectors (pLKO.1) expressing shRNA clones were generated by the Broad Institute RNAi platform, as previously described (Moffatt et al., Cell. 2006; 124:1283-1298). Four sequence-independent shRNA's were generated against human SHMT2 using the following target gene sequences (RNAi Platform ID#):

sh1 (TRCN0000034808): GAGGTGTGTGATGAAGTCAAA (SEQ ID NO:5)
sh2 (TRCN0000234656): ACAAGTACTCGGAGGGTTATC (SEQ ID NO:6)
sh3 (TRCN0000234657): GTCTGACGTCAAGCGGATATC (SEQ ID NO:7)
sh4 (TRCN0000238795): CGGAGAGTTGTGGACTTTATA (SEQ ID NO:8)
Control shRNA (shCtrl = TRCN0000072181) was generated with a target sequence not matching any human gene: ACAACAGCCACAACGTCTATA (SEQ ID NO:9).

For lentiviral infection, 100,000 cells were seeded in a 6-well dish in 2 mL medium containing 8 μg/ml Polybrene and 100 μl viral supernatant added. Plates were centrifuged at 800 x g for 30 minutes at 37 °C, and the medium replaced. Twenty-four hours later, cells were selected for infection by the addition of 2 μg/ml puromycin. Uninfected control cells demonstrated 100% cell death with puromycin within 24 hours. Cells were passaged for >10 cell divisions to ensure stable expression of the shRNA construct. For assessment of SHMT2 knockdown, mRNA was isolated from cells using RNeasy kit (Qiagen), and qRT-PCR was performed for SHMT2 and HPRT1 using the Taqman assay (Applied Biosystems assay ID Hs00193658_m1* and Hs01003267_m1*, respectively), according to manufacturer's instructions. For experiments using A498 and LOX IMVI, cells were infected with either sh1-4 or shCtrl lentivirus and stable expressing cells selected as above. For experiments utilizing NCI-H226, HS-578T, TK10, EKVX, OVCAR-8, U251, A549, HT29, NCI-460 and HCT-116, cells were infected with either sh4 or shCtrl lentivirus and stable expressing cells selected as above. One additional cell line, HCT-15 was removed from the analysis since effective knockdown of SHMT2 could not be achieved. Following generation of stable knockdown cell lines, cells were plated in 96-well microtiter plates in 200 μL of RPMI 1640 medium containing 2 mM glutamine and either 140 μM (+gly) or 0 μM (-gly) glycine, supplemented with 5% dialyzed FBS. For rescue experiments (Example 4) LOX IMVI cells expressing sh4 were grow in the absence of glycine and rescue attempted with vehicle (PBS), glycine (140 μM), sarcosine (140 μM) or formate (140 μM). For all experiments, cells were counted and cell counts expressed as fold change over time. For glycine dropout experiments (Example 4), A498 and LOX IMVI cells were cultured in RPMI 1640 medium containing 2 mM glutamine and either 140

μM (+gly) or 0 μM (-gly) glycine, supplemented with 5% dialyzed FBS. Cells were counted at regular time intervals as described above. All experiments were performed using at least 10 independent cell cultures.

Culture of Non-transformed Cells

Human breast epithelial (CC-2551, Lonza), human lung bronchial epithelial cells (CC-2540, Lonza) and human umbilical vein endothelial cells (Lonza), were cultured in MEGM, BEGM and EGM2 media respectively, according to manufacturer instructions. Doubling times for all cells were confirmed using cell counting, as described above. Cells were cultured in 10 cm dishes for five days and fresh and spent medium collected for measurement of glycine CORE, as described above. For CD4+ experiments, peripheral blood lymphocytes were isolated from whole blood using Ficoll (Sigma) gradient centrifugation, and resting CD4+ T cells were purified (>95% purity) using magnetic negative separation (Dynal, Invitrogen), according to the manufacturer's instructions. Purified cells were plated in Dulbecco's modified Eagle's medium (DMEM) supplemented with 10% heat-inactivated fetal calf serum, 2 mM L-glutamine, penicillin-streptomycin, nonessential amino acids, sodium pyruvate, vitamins, 10 mM HEPES, and 50 mM 2-mercaptoethanol supplemented with 20 U/mL recombinant human IL-2, at a concentration of 1 x $10^6$ cells/mL in 12-well plates pre-coated with goat anti-mouse IgG (ICN Biomedical). Cells were stimulated with 1 ug/mL anti-human CD3 (eBioscience, clone UCHT1) and 1 ug/mL anti-human CD28 (eBioscience, clone CD28.2). After 48 hours, stimulated cells were removed from the TCR signal and re-cultured at a concentration of 1 x $10^6$ cells/mL, and stimulated cell supernatants were harvested after 24 hours in culture. For each condition, triplicate wells were prepared and analyzed.

Compound sensitivity analysis

Compound sensitivity data for glutathione biosynthesis and *de novo* purine biosynthesis inhibitors was obtained from the NCI data repository (October 2009 release). Compound sensitivity was quantified using the GI50 measure (Shoemaker, Nat Rev Cancer. 2006;6:813-823), defined as the compound concentration inhibiting cell growth by 50%. For those compounds in which multiple experiments with different concentration ranges were available, the experiment with the least degree of saturation was used.

Gene expression analysis

Gene expression data for the 60 cell lines was previously generated by Chiron Corporation (Emeryville, CA) using Affymetrix U133A and U133B arrays and normalized probeset-level data obtained from the NCI data repository. In cases where several probesets matched a gene of interest, we selected the probeset with maximum average intensity across all cell lines. The MDA-MB-468 and RXF 393 cell lines did not have associated gene expression data and were ignored for the purpose of gene expression analysis. Enrichment analysis for gene expression was evaluated using the GSEA-P statistic (Subramanian et al., Proc Natl Acad Sci USA. 2005;102:15545-15550) with p = 1, using the Spearman correlation as the underlying measure. P-values and false discovery rates were calculated by randomly permuting cell lines as previously described (Subramanian et al., Proc Natl Acad Sci USA. 2005;102:15545-15550).

Cell cycle analysis

HeLa cells were transfected with a geminin reporter construct as previously described (Sakaue-Sawano et al., Cell. 2008;132:487-498) and subsequently infected with lentivirus containing the shSHMT2 (sh4) hairpin or shCtrl hairpin as described above, and selected using puromycin. Cells were cultured in using identical conditions to NCI-60 cells, as described above, in the presence of 140 μM glycine (+gly) or absence of glycine (-gly), and doubling times determined from growth curves, as described above. For cell cycle analysis, cells were plated in the presence of 140 μM glycine (+gly) or absence of glycine (-gly) in 10 cm dishes containing adhered glass cover slips. After 48h, cells were fixed and stained with DAPI to quantify DNA content and a succinimidyl-linked Alexa SE-A647 dye (Invitrogen) to quantify protein content, according to manufacturer instructions. Cells were imaged using a Nikon Ti-E Microscope with Ti-ND6-PFS Perfect Focus, and image analysis was performed with custom written software (EnsembleThresher). The algorithm identified cell boundaries by two complementary approaches: (i) cells were separated from background by thresholding a Top-Hat transform of the original image. Top-Hat transformation was used to remove trends that are spatially wider than cell diameters; and (ii) boundaries between adjacent, touching cells were identified by seed-based watershedding. Seeds were calculated as the regional maxima of the Gaussian-smoothed image. Imaging analysis resulted in a single intensity value per cell. Geminin data was log-transformed, and density plots were generated by bin counting on a 50 x 50 grid. Gates were set manually to optimally separate G1, G1/S and G2 populations, and used to calculate fractions of cells in each phase. From these data, fractional lengths of each cell cycle phase was estimated as previously described (Toettcher et al., Proc Natl Acad Sci USA. 2009;106:785-790) and multiplied by the measured doubling time for each cell line and culture condition to obtain absolute cell cycle phase lengths.

Survival analysis

Six independent large cohorts of patients with early stage cancer for which survival data for at least a decade was available were examined. Microarray data from Chin et al. (Cancer Cell. 2006;10:529-541) and van de Vijver et al. (N Engl J Med. 2002;347:1999-2009), were downloaded from the Lawrence Berkeley National Laboratory (cancer.lbl.gov/breastcancer/list_data.php?id=9) and Rosetta Inpharmatics (rii.com/publications/2002/nejm.html), respectively. Microarray data from the Desmedt et al. (Clin Cancer Res. 2007;13:3207-3214), Pawitan et al. (Breast Cancer Res. 2005;7:R953-964), Miller et al. (Proc Natl Acad Sci U S A 2005 Sep 20;102(38):13550-5) and Kao et al. (BMC Cancer. 2011;11:143) studies are available in the NCBI Gene Expression Omnibus, accessions GSE7390, GSE1456, GSE3494, and GSE20685, respectively. Survival data and clinical parameters were obtained from the original reports. Patients were split into "positive" or "negative" groups based on the centroid $t_i$ of expression of the mitochondrial glycine metabolic pathway (consisting of the enzymes SHMT2, MTHFD2 and MTHFD1L),

$$t_i = \frac{1}{|G|} \sum_{j \in G} x_{ij} / \bar{x}_j ,$$

where

$$\bar{x}_j = \sum_i x_{ij} / n$$

where $i$ ranges over patients (arrays) and $j$ over genes in the glycine pathway G. Individuals $i$ with $t_i$ above its median were assigned to the "positive" group, and Kaplan-Meier curves were derived for these groups. Hazard ratios were estimated using Cox's proportional hazard model (Cox, Journal of the Royal Statistical Society. Series B. 1972;34:187-220), as implemented in the R package "survival" (cran.r-project.org/web/packages/survival/index.html). Groups were tested for significant differences using the logrank test (Bland and Altman, BMJ. 2004;328:1073). Meta-analysis was performed using DerSimonian & Laird's weighted estimator (DerSimonian and Laird, Control Clin Trials. 1986;7:177-188), with the Cox hazard ratio as the effect size measure. No significant heterogeneity between studies was detected (P = 0.34).

**Example 1. Cancer Cell Metabolism**

To systematically characterize cancer cell metabolism, liquid chromatography-tandem mass spectrometry was used to profile the cellular consumption and release (CORE) of 219 metabolites spanning the major pathways of intermediary metabolism in the NCI-60 panel, a collection of sixty well-characterized primary human cancer cell lines established from nine common tumor types [17]. CORE profiling builds upon metabolic footprinting or exometabolomics [18,19], and provides a systematic and quantitative assessment of cellular metabolic activity by relating metabolite concentrations in medium from cultured cells to baseline medium, resulting in a time-averaged consumption and release (CORE) profile for each metabolite on a per cell basis over a period of exponential growth. Using CORE profiling 140 metabolites were identified that were either present in fresh medium or released by at least one cancer cell line, of which 111 metabolites demonstrated appreciable variation across the 60 cell lines, with excellent reproducibility between biological replicates. Approximately one third of the 111 metabolites were consumed by all cell lines, whereas most of the remaining two thirds of metabolites were consistently released into the medium; only a handful of metabolites exhibited consumption in certain cell lines and release by others.

This CORE atlas of cancer metabolism can be used to explore metabolic phenotypes of cancer cells and to discover relationships between metabolites. For example, ornithine was released from leukemia cells and adenosine and inosine were released from melanoma cells, reflecting metabolic activities that may be unique to these cancers. Unsupervised cluster analysis of metabolite CORE data identified leukemia cells as a distinct group, but did not more generally distinguish between tumor cell lines based on tissue of origin. Functionally related metabolites demonstrated similar patterns of consumption and release across the 60 cell lines. For example, major nutrients including glucose, essential amino acids, and choline formed a single cluster, as did metabolites representing glycolysis, the citric acid cycle, nucleotides, and polyamines. Consumption of major nutrients also correlated with release of their byproducts: for example, glucose consumption correlated to lactate release, consistent with the well-documented Warburg effect in

transformed cells [4]. A similar pattern of nutrient consumption and byproduct release was also observed with other nutrients. Glutamine consumption, quantitatively the greatest among amino acids, was closely mirrored by glutamate release. An analysis of all monitored metabolites revealed that total measured carbon consumption was also closely correlated to total measured carbon release, suggesting that transformed cells share a common metabolic phenotype of incomplete catabolism of major nutrients followed by byproduct release.

**Example 2. Metabolism of Glycine Correlates with Proliferation**

The next experiments were performed to determine whether any metabolite CORE profiles were associated with cancer cell proliferation. Previously reported doubling times across the 60 cancer cell lines ranged from 17.0 to 79.5 hours and were highly reproducible [20]. From the 111 metabolite CORE profiles, two metabolites, phosphocholine and glycine, were significantly correlated (Bonferroni-corrected $P < 0.01$) with proliferation rate across the 60 cell lines (Fig. 1A). Phosphocholine, which was released from all cells, correlated with consumption of the essential nutrient choline, and has been reported to accumulate in transformed cells as a substrate for phospholipid biosynthesis [21]. In contrast, the relation between glycine consumption and proliferation rate was unanticipated, since glycine is a non-essential amino acid that can be endogenously synthesized. Glycine exhibited an unusual CORE profile, being consumed by rapidly proliferating cells and released by slowly proliferating cells (Fig. 1B), suggesting that glycine demand may exceed endogenous synthesis capacity in rapidly proliferating cancer cells, whereas in slowly proliferating cells, glycine synthesis may exceed demand. Increasing glycine consumption with faster proliferating rate was observed across all 60 cell lines (Fig. 1B), and was even more pronounced within specific tumor types, including ovarian, colon, and melanoma cells (Fig. 2B), but not evident in non-adherent leukemia cells. To determine whether glycine consumption is specific to transformed cells or a general feature of rapid proliferation, glycine consumption was measured in cultured primary human mammary epithelial cells (HMEC), human bronchial epithelial cells (HBE), human umbilical vein endothelial cells (HUVEC) and human activated CD4+ T lymphocytes. These nontransformed cells had doubling times between 8 and 18 hours, comparable to the most rapidly dividing cancer cells, yet each of these cell types released rather than consumed glycine (HMEC: $3.5 \pm 0.8$; HBE $17.5 \pm 3.2$; HUVEC $8.4 \pm 1.4$; lymphocytes $1.9 \pm 0.3$ fmol/cell/h). Thus, glycine consumption appears to be a feature specific to rapidly proliferating transformed cells.

**Example 3. Expression of Glycine Metabolic Enzymes**

To complement the metabolite CORE analysis, the gene expression of 1,425 metabolic enzymes [22] was examined in a previously generated microarray dataset across these 60 cell lines [23]. This independent analysis revealed that glycine biosynthesis enzymes are more highly expressed in rapidly proliferating cancer cell lines (Fig. 1C). Intracellular glycine synthesis is compartmentalized between the cytosol and mitochondria [11], providing two separate enzymatic pathways (Fig. 1D). The mitochondrial glycine synthesis pathway consists of the glycine-synthesizing enzyme serine hydroxymethyltransferase 2, *SHMT2,* a target of the oncogene c-Myc [24], as well as methylenetetrahydrofolate dehydrogenase (NADP+ dependent) 2 *(MTHFD2)* and methylenetetrahydrofolate dehydrogenase (NADP+ dependent) 1-like (*MTHFD1L*)*,* which regenerate the cofactor tetrahydrofolate (THF) for the SHMT2 reaction (Fig. 1D). Only the mitochondrial pathway exhibited significant correlation with proliferation, whereas the corresponding cytosolic enzymes did not (Fig. 1C), suggesting a key role for mitochondria in supporting rapid cancer cell proliferation. To assess the relative contributions of glycine consumption *vs*. endogenous synthesis to intracellular glycine pools, tracer analysis with $^{13}C$-labeled glycine in rapidly dividing LOX IMVI cells was utilized. Assuming a simple steady state model [23], from labeling of intracellular glycine and serine pools it was estimated that approximately one-third of intracellular glycine originates from extracellular consumption, whereas the remainder is synthesized endogenously. Thus, both metabolite CORE profiling and gene expression analysis independently identify glycine metabolism as closely related to rapid proliferation in cancer cells.

**Example 4. Glycine Metabolism is Crucial in Cancer Cells**

To directly evaluate the contribution of glycine metabolism to rapid cancer cell proliferation, a combination of genetic silencing and nutrient deprivation was used. Expression of the glycine-synthesizing enzyme *SHMT2* was stably silenced in slowly proliferating A498 cells and in rapidly proliferating LOX IMVI cells (Fig. IE) with four distinct shRNA hairpins. CHO strains mutant in SHMT2 have previously been shown to be auxotrophic for glycine [25]. Silencing of SHMT2 in the absence of extracellular glycine halted proliferation of LOX IMVI cells (Fig. 2F), and was rescued by the addition of glycine to the medium, indicating that glycine itself, rather than one-carbon units derived from the SHMT2 reaction (Fig. 1D), is critical to proliferation in these cells (Fig. IF). Supplementation of medium with sarcosine, a glycine-related metabolite [26], or formate, a source of cellular one-carbon units [27], failed to rescue LOX IMVI cells. In contrast, slowly proliferating A498 cells (Fig. 2F) were not impaired by *SHMT2* depletion and extracellular glycine deprivation, indicating that other means of glycine synthesis can satisfy the requirements in these cells. Withdrawal of extracellular glycine alone also reduced the proliferation of LOX IMVI cells but not A498 cells, although this effect was more subtle.

Collectively, this data suggest that mitochondrial production of glycine is critical specifically in rapidly proliferating cancer cells.

To determine whether this reliance on glycine for rapid proliferation extends to other cancer cells, silencing of *SHMT2* (Fig. 1H) and extracellular glycine deprivation was tested in 10 additional primary cancer cell lines from the NCI-60 panel (Fig. 1G). Rapidly proliferating cancer cells exhibited slower proliferation with antagonism of glycine metabolism and were rescued with addition of extracellular glycine, whereas slowly proliferating cells were less sensitive to these perturbation (Fig. 1G), even when assessed at later time points to allow for a comparable number of cellular divisions relative to rapidly proliferating cells.

We next sought to explore the potential mechanisms by which glycine metabolism contributes to rapid cancer cell proliferation. The results suggested that consumed glycine is utilized in part for *de novo* purine nucleotide biosynthesis in rapidly proliferating in these cells, and antagonism of glycine metabolism results in prolongation of Gl, thus slowing proliferation.

**Example 5. Expression of Glycine Synthesis Enzymes in Cancer Patients**

To explore the potential relevance of glycine metabolism to cancer, the expression of the mitochondrial glycine synthesis enzymes, *SHMT2*, *MTHFD2* and *MTHFD1L* (Fig. 2D), was examined in previously generated microarray datasets across six independent large cohorts totaling over 1300 patients with early stage breast cancer followed for survival [28-33]. Two groups of individuals were defined: those with above-median gene expression of the mitochondrial glycine biosynthesis pathway, and those with below-median gene expression. Above-median expression of the mitochondrial glycine biosynthesis pathway was associated with greater mortality (Figs. 2A-B), and a formal meta-analysis of all six datasets indicated an overall hazard ratio of 1.82 (95% CI: 1.43 - 2.31; Fig. 2B), comparable to that of other established factors such as lymph node status and tumor grade, that contribute to poor cancer prognosis [31]. The mitochondrial glycine synthesis enzyme *SHMT2* alone was also significantly associated with mortality, whereas its cytosolic paralog *SHMT1* was not. These data highlight the potential importance of mitochondrial glycine metabolism in human breast cancer.

**Example 6. Glycine-Consuming Cells are Sensitive to Antifolate Drugs**

Given the observation that glycine consumption is correlated to rapid cancer cell proliferation (see Examples 1-5, above), the present inventors sought to determine whether glycine consumption predicts sensitivity to particular chemotherapeutic agents. The association between glycine update and sensitivity to 3851 annotated small molecules (data provided to the public by NCI) was examined bioinformatically across the 60 cell lines.

The results showed that cells that consume glycine were uniquely sensitive to multiple antifolate agents (grey dots on Fig. 3, 15 antifolate agents listed below with NCI / NSC identifiers) including the commonly used agent methotrexate (NCI# 740) that target tetrahydrofolate metabolism through DHFR. It is notable that although many chemotherapy agents will kill rapidly proliferating cancer cells, glycine consuming cells were not more sensitive to other chemotherapy agents (black dots), including 5-fluorouracil, which targets the related metabolic enzyme TYMS. Hence, the fact that glycine consumption is strongly correlated to and predictive of cellular sensitivity to antifolates, including methotrexate, is nonobvious.

**TABLE 1**

| NCI (NSC) # | Chemical names |
|---|---|
| 133072 | Ethanesulfonic acid, compd. with 4-(4,6-diamino-2,2-dimethyl-1,3, 5-triazin-1(2H)-yl)-N-(3-methylphenyl) benzenepropanamide (1:1)\|Ethanesulfonic acid, compd. with 4-(4,6-diamino-2,2-dimethyl-s-triazin-1 (2H)-yl)-m-hydrocinnamotoluidide (1:1) |
| 132275 | Ethanesulfonic acid, compd. with 2-[2-chloro-4-(4,6-diamino-2, 2-dimethyl-1,3,5-triazin-1(2H)-yl) phenoxy]-N-phenylacetamide (1:1)\|Ethanesulfonic acid, compd. with 2-(2-chloro-4-(4,6-diamino-2, 2-dimethyl-s-triazin-1(2H)-yl)phenoxy)acetanilide (1:1) |
| 225112 | 2,4,6-Quinazolinetriamine, N(6)-[(3, 4-dichlorophenyl)methyl]-N(6)-ethyl-5-methyl- |

(continued)

| TABLE 1 | |
|---|---|
| NCI (NSC) # | Chemical names |
| 173516 | 1,3,5-Triazine-2,4-diamine, 1-[4-fluoro-3-(trifluoromethyl)phenyl]-1,6-dihydro-6,6-dimethyl-, monohydrochloride\|s-Triazine, 4,6-diamino-1,2-dihydro-2,2-dimethyl-1-(.alpha., .alpha.,.alpha.,4-tetrafluoro-m-tolyl)-, monohydrochloride (8Cl) |
| 123463 | Ethanesulfonic acid, compd. with 4-[3-[p-(4,6-diamino-2,2-dimethyl-s-triazin-1(2H)-yl)benzyl]ureido]-o-toluenesulfonyl fluoride (1:1) (8Cl) |
| 132277 | Ethanesulfonic acid, compd. with 1,6-dihydro-6,6-dimethyl-1-[4-(4-phenylbutyl)phenyl]-1,3,5-triazine-2,4-diamine (1:1)\|Ethanesulfonic acid, compd. with 4,6-diamino-1,2-dihydro-2, 2-dimethyl-1-[p-(4-phenylbutyl)phenyl]-s-triazine (1:1) |
| 3077 | D 54\|s-Triazine, 4,6-diamino-1-(3,4-dichlorophenyl)-1,2-dihydro-2, 2-dimethyl-, monohydrochloride (8Cl) \|X 69 |
| 3074 | 1,3,5-Triazine-2,4-diamine, 1-(4-chlorophenyl)-1,6-dihydro-6, 6-dimethyl-, monohydrochloride (9Cl)\|4, 6-Diamino-1-(p-chlorophenyl)-1,2-dihydro-2,2-dimethyl-s-triazine hydrochloride\|Chlorazin\|Cycloguanil hydrochloride\|NSC 3074\|s-Triazine, 4,6-diamino-1-(p-chlorophenyl)-1,2-dihydro-2, 2-dimethyl-, monohydrochloride (8Cl)\|WLN: T6N CN EN BHJ AR DG & B1B1 DZ FZ & GH |
| 173552 | L-Aspartic acid, N-[4-[[(2-amino-4-hydroxy-6-quinazolinyl)methyl]amino]benzoyl]-, monohydrate |
| 123461 | Benzenesulfonyl fluoride, 4-[[3-[2-chloro-4-(4,6-diamino-2,2-dimethyl-1,3,5-triazin-1(2H)-yl)phenyl]-1-oxopropyl]amino]-, monoethanesulfonate (9Cl)\|Ethanesulfonic acid, compd. with 4-[[3-[2-chloro-4-(4,6-diamino-2,2-dimethyl-1,3,5-triazin-1(2H)-yl)phenyl]-1-oxopropyl]amino]benzenesulfonyl fluoride (1:1) (9Cl)\|Ethanesulfonic acid, compd. with N-[2-chloro-4-(4,6-diamino-2,2-dimethyl-s-triazin-1(2H)-yl) hydrocinnamoyl]sulfanilyl fluoride (1:1) (8Cl)\|NSC-123461 |
| 302325 | 2,4-Pyrimidinediamine, 5-(4-chloro-3-nitrophenyl)-6-ethyl-\|Pyrimidine, 2,4-diamino-5-(4-chloro-3 -nitrophenyl)-6-ethyl- (8Cl) |
| 112846 | Aspartic acid, N-[p-[[(2, 4-diamino-6-quinazolinyl)methyl]amino]benzoyl]-, L-(8Cl)\|L-Aspartic acid, N-[4-[[(2, 4-diamino-6-quinazolinyl)methyl]amino]benzoyl]- (9Cl)\|N-[p-[[(2, 4-Diamino-6-quinazolinyl) methyl]amino]benzoyl]-L-aspartic acid\|Quinaspar |
| 382034 | 2,4-Pyrimidinediamine, 6-ethyl-5-[4-(methylamino)- 3-nitrophenyl]- |
| 740 | Amethopterin\|CL 14377\|EMT 25,299\|Glutamic acid, N-[p-[[(2, 4-diamino-6-pteridinyl)methyl] methylamino]benzoyl]-, L-(+)-(8Cl)\|HDMTX\|L-Glutamic acid, N-[4-[[2, 4-diamino-6-pteridinyl) methyl]-methylamino]benzoyl]-(9Cl)\|Metatrexan\|Methopterin\|Methotrexate(USAN) \|Methylaminopterin\|MTX\|NCI-C04671\|R 9985\|WLN: T66 BN DN GN JNJ CZ EZ H1N1&R DVMYVQ2VQ |

**Example 7. MTHFD2 mRNA and Protein are Increased in Cancer Cells**

To identify potential gene targets for cancer chemotherapeutics, a large dataset of tumor samples and corresponding normal counterparts was generated (dataset derived from [34] and analyzed to identify genes that were consistently upregulated in 20 diverse cancers relative to normal tissue counterparts (Fig. 4). Among the 20,450 genes measured, the top 50 genes most consistently upregulated genes (defined as the number of datasets in which the gene appears within the top 5% of upregulated genes) are shown in Table 2 below. This gene list includes known drug targets, including TYMS, RRM2, TOP2A, and AURKA, as well as the higher ranking gene MTHDF2 (gene rank 8), all of which are in bold font in Table 2. In contrast, the cytosolic paralogue MTHFD1 (gene rank 548) or the adult paralogue MTHFD2L (rank score 11912), were not highly upregulated in cancer relative to normal counterparts.

TABLE 2

| Rank | Score | Symbol | Description |
|---|---|---|---|
| 1 | 30 | KIAA0101 RNASEH2 | KIAA0101 |

(continued)

| Rank | Score | Symbol | Description |
|---|---|---|---|
| 2 | 29 | A | ribonuclease H2, subunit A |
| 3 | 29 | MELK | maternal embryonic leucine zipper kinase |
| 4 | 28 | TPX2 | TPX2, microtubule-associated, homolog (Xenopus laevis) |
| 5 | 28 | UBE2C | ubiquitin-conjugating enzyme E2C |
| 6 | 28 | CCNB1 | cyclin B1 |
| 7 | 27 | CBX3 | chromobox homolog 3 |
| **8** | **27** | **MTHFD2** | **methylenetetrahydrofolate dehydrogenase** |
| 9 | 27 | TRIP 13 | thyroid hormone receptor interactor 13 |
| 10 | 27 | CCNB2 | cyclin B2 |
| **11** | **27** | **TYMS** | **thymidylate synthetase** |
| **12** | **27** | **RRM2** | **ribonucleotide reductase M2** |
| 13 | 26 | LMNB2 | lamin B2 |
| 14 | 26 | GGCT | gamma-glutamylcyclotransferase |
| 15 | 26 | GMPS | guanine monphosphate synthetase |
| 16 | 26 | UCK2 | uridine-cytidine kinase 2 |
| **17** | **26** | **AURKA** | **aurora kinase A** |
| 18 | 26 | STIL | SCL/TAL1 interrupting locus |
| 19 | 26 | NME1 | non-metastatic cells 1, protein (NM23A) expressed in |
| 20 | 26 | CKS2 | CDC28 protein kinase regulatory subunit 2 |
| 21 | 26 | CCNA2 | cyclin A2 |
| 22 | 26 | BUB1B | budding uninhibited by benzimidazoles 1 homolog beta (yeast) |
| 23 | 25 | ZWINT | ZW10 interactor |
| 24 | 25 | KIF14 | kinesin family member 14 |
| 25 | 25 | PTTG1 | pituitary tumor-transforming 1 |
| 26 | 25 | AURKB | aurora kinase B |
| 27 | 25 | PLOD3 | procollagen-lysine, 2-oxoglutarate 5-dioxygenase 3 |
| 28 | 25 | RUVBL1 | RuvB-like 1 (E. coli) |
| 29 | 25 | TTK | TTK protein kinase |
| **30** | **25** | **TOP2A** | **topoisomerase (DNA) II alpha 170kDa** |
| 31 | 25 | SHMT2 | serine hydroxymethyltransferase 2 (mitochondrial) |
| 32 | 25 | MCM2 | minichromosome maintenance complex component 2 |
| 33 | 25 | GARS | glycyl-tRNA synthetase |
| 34 | 25 | FOXM1 | forkhead box M1 |
| 35 | 25 | CDKN3 | cyclin-dependent kinase inhibitor 3 |
| 36 | 25 | CDK4 | cyclin-dependent kinase 4 |
| 37 | 25 | CDC20 | cell division cycle 20 homolog (S. cerevisiae) |
| 38 | 24 | XPOT | exportin, tRNA (nuclear export receptor for tRNAs) |
| 39 | 24 | KIF2C | kinesin family member 2C NDC80 kinetochore complex component homolog (S. |
| 40 | 24 | NDC80 | cerevisiae) |
| 41 | 24 | GINS1 | GINS complex subunit 1 (Psfl homolog) |
| 42 | 24 | DLGAP5 | discs, large (Drosophila) homolog-associated protein 5 |
| 43 | 24 | TK1 | thymidine kinase 1, soluble |
| 44 | 24 | TARS | threonyl-tRNA synthetase |
| 45 | 24 | MKI67 | antigen identified by monoclonal antibody Ki-67 |
| 46 | 24 | MCM7 | minichromosome maintenance complex component 7 |
| 47 | 24 | KIF11 | kinesin family member 11 |
| 48 | 24 | FEN1 | flap structure-specific endonuclease 1 |
| 49 | 24 | CENPF | centromere protein F, 350/400kDa (mitosin) |

(continued)

| Rank | Score | Symbol | Description |
|---|---|---|---|
| 50 | 24 | CDK1 | cyclin-dependent kinase 1 |

Prior meta-analyses of tumor microarrays have similarly identified MTHFD2 mRNA as elevated across multiple human tumors types, including lung adenocarcinoma, small cell lung cancer, colon cancer, prostate cancer, salivary carcinoma, glioma, and medulloblastoma [35], as well as in human breast cancer [36, 37]. Moreover, MTHFD2 *activity* has been found to be increased in embryonic tissue as well as in a number of transformed cell lines, including murine Ehrlich mastocytoma ascites cells, MCF-7 breast cancer cells, M4 cutaneous melanoma cells, EL4 murine T cells lymphoma cells, K562 CML cells, Raji Burkitt lymphoma cells, L murine fibroblast cells, CCRF-CEM leukemia cells, MG-63 osteosarcoma cells, MNNG/HOS osteosarcoma cells, YAC lymphoma cells, HA-Py embryo fibroblast cells, BeWo choriocarcinoma cells, HCT-8R intestinal carcinoma cells, as well as in transformed human leukocytes, CHO cells and mouse embyo cells, but MTHFD2 activity was not present in adult tissues, with the exception of adult bone marrow [10, 38].

However, the degree of MTHFD2 upregulation in cancer relative to all other genes is not disclosed in these prior studies, and in particular, none of the previous studies showed how MTHFD2 compares to known chemotherapy drug targets or to paralogous enzymes (MTHFD1 and MTHFD2L). Moreover, it was not clear whether upregulation of MTHFD2 is a generalizable feature of cancer or specific to several cancer types. The present meta-analysis suggests that MTHFD2 is a generalizable feature of cancer and present in many cancer types.

To determine if MTHFD2 protein is indeed increased in various cancers, immunohistochemical analysis for MTHFD2 protein was performed in over 100 cancer biopsies, spanning 16 cancer types, with methods as previously described [39]. MTHFD2 was found to be strongly expressed in tumor cells, with limited expression in the normal stroma (Fig. 5). Collectively, this data suggests that MTHFD2 represents a new cancer drug target, one that that is highly upregulated, more so than even existing drug targets, in a variety of cancers relative to normal counterparts.

**Example 8. MTHFD2 Expression Levels Predict Survival**

Whether expression of MTHFD2 may be used as a predictor of cancer survival in patients was also examined. Patients with breast cancer, colon cancer and renal cancer selected for which microarray datasets and corresponding survival data were publically available.

As shown in Fig. 6, across these three cancer types, above median expression of MTHFD2 (grey line) was associated with worse survival than below median expression of MTHFD2 (black line). This not only suggests the importance of MTHFD2 in driving cancer progression, but also suggests that expression of MTHFD2 may be used as prognostic marker in breast, colon and renal cell cancer.

**Example 9. MTHFD2 is Differentially Expressed in Cancer v Normal Cells**

As mentioned above, many current chemotherapy agents have on-target side effects which stem from affecting genes in normal proliferating cells. A microarray tissue atlas was used to examine expression of various genes in 1) normal, non-proliferating tissues (Fig. 7A, grey bars), 2) normal proliferating tissues (Fig. 7A, black bars) including colon epithelium and leukocytes, and 3) cancer tissues included in this tissue atlas dataset (Fig. 7A, white bars). A minimum / maximum ratio for all >20,000 genes was calculated based on the minimum expression among the cancer samples relative to maximum expression in all normal tissues. An ideal anti-cancer agent would have a high min/max ratio and be high among all cancer samples, and relatively low in normal tissues.

Among all 20,000 genes, MTHFD2 had the highest min/max ratio (Fig. 7B). Expression of MTHFD2 in normal non proliferating, normal proliferating and cancer samples is shown on the upper left. In contrast to MTHFD2, the cytosolic paralogue MTHFD1 was elevated in several proliferating normal tissues and was low in many transformed cells. The adult paralogue MTHFD2L was ubiquitously expressed without upregulation in cancer. Similar to MTHFD1, many other known cancer chemotherapeutic drug targets, such as RRM2, DHFR, TOP2A, TYMS, were increased in proliferating normal tissues to comparable levels to cancer, which may contribute to the side effects associated with use of these chemotherapeutic agents.

Induction of these genes was also evaluated in additional datasets of activated and proliferating normal cells. Whereas many known chemotherapeutic drug targets, including DHFR, RRM2 and TOP2A, were strongly induced in normal tissue when stimulated to proliferate, MTHD2 was not induced in normal proliferating cells (see Fig. 7C, left and middle panels). The exception was activated T cells, which do upregulate MTHFD2 expression when activated (Fig. 7C, right panel). Collectively, these data suggest that MTHFD2 may represent an excellent chemotherapy drug target given its low levels or lack of expression in non-cancerous tissues.

**Example 10. shRNA Silencing of MTHFD2 Slows Cancer Cell Proliferation**

To determine whether MTHFD2 is indeed essential for cancer cell proliferation, MTHFD2 expression was silenced in 16 cancer cell lines using 2 sequence independent shRNA reagents (sh50 sequence CGAATGTGTTTGGATCAGTAT (SEQ ID NO:10); sh53 sequence: GCAGTTGAAGAAACATACAAT (SEQ ID NO:11)).

As shown in Fig. 8, in 15 of the 16 cell lines tested, shRNA mediated silencing of MTHFD2 by at least one shRNA reagent significantly slowed cancer cell proliferation over 7 days.

**Example 11. Bacterial Expression and Purification of human MTHFD2**

Based on previous papers [40], a construct was designed that allowed expression and purification of large quantities of human MTHFD2. Starting with the full-length 350 amino acid sequence, the 35 amino acid mitochondrial targeting sequence (which is cleaved to produce the active protein in mammalian cells) was removed, and a methionine was added to initiate translation. 12 amino acids were removed from the unstructured C-terminus of the protein, and a 6-histidine tag added to facilitate purification. This construct was previously reported in the literature, but without the following modifications: the transcript was codon-optimized for bacterial expression (so the human *protein* was produced, even if the nucleotide sequence was not identical to that found in humans). This construct was synthesized and cloned into the pET-30a(+) vector for bacterial expression by Genewiz. The codon optimized (for expression in *Escherichia coli*) sequence was as follows; the start and stop codons are underlined:

CATATGGAGGCCGTGGTTATCAGTGGCCGCAAGCTGGCCCAGCAGATCAAGCAGG
AGGTGCGCCAAGAAGTGGAAGAATGGGTTGCCAGCGGCAACAAGCGCCCGCATC
TGAGCGTGATCCTGGTGGGCGAGAACCCGGCAAGCCACAGCTACGTGCTGAACA
AAACACGCGCAGCAGCAGTGGTGGGCATCAACAGCGAGACAATCATGAAACCGG
CCAGCATCAGCGAGGAAGAGTTACTGAACTTAATTAACAAGCTGAATAACGACG
ACAACGTGGACGGCCTGCTGGTGCAGTTACCGCTGCCGGAACATATCGACGAACG
CCGCATCTGCAACGCCGTGAGTCCTGATAAGGACGTGGACGGCTTTCACGTGATC
AATGTTGGCCGCATGTGCTTAGACCAGTACAGCATGCTGCCGGCAACCCCTTGGG
GCGTTTGGGAGATCATCAAGCGCACCGGTATCCCGACCCTGGGTAAGAACGTTGT
GGTGGCCGGCCGTAGCAAGAACGTGGGCATGCCTATCGCAATGTTACTGCACACC
GACGGCGCACATGAACGTCCTGGTGGCGATGCAACCGTTACCATCAGTCACCGTT
ACACCCCGAAGGAGCAACTGAAAAAGCACACCATCCTGGCCGACATTGTGATCA
GCGCAGCCGGCATTCCGAACCTGATCACCGCAGACATGATCAAAGAGGGCGCCG
CCGTGATCGACGTGGGCATTAACCGCGTGCACGATCCGGTGACAGCCAAACCGAA
GCTGGTGGGTGACGTGGACTTCGAGGGCGTGCGTCAAAAAGCCGGCTACATCACC
CCGGTTCCTGGTGGCGTTGGCCCTATGACCGTGGCCATGCTGATGAAGAACACCA
TCATTGCCGCCAAGAAGGTGCTGCGTCTGGAGGAGCGCGAGCACCATCATCACCA
CCACTAAGGTACC (SEQ ID NO:1)

The amino acid sequence of the expressed construct was:

MEAVVISGRKLAQQIKQEVRQEVEEWVASGNKRPHLSVILVGENPASHSYVLNKTRA
AAVVGINSETIMKPASISEEELLNLINKLNNDDNVDGLLVQLPLPEHIDERRICNAVSP
DKDVDGFHVINVGRMCLDQYSMLPATPWGVWEIIKRTGIPTLGKNVVVAGRSKNVG
MPIAMLLHTDGAHERPGGDATVTISHRYTPKEQLKKHTILADIVISAAGIPNLITADMI
KEGAAVIDVGINRVHDPVTAKPKLVGDVDFEGVRQKAGYITPVPGGVGPMTVAMLM
KNTIIAAKKVLRLEEREHHHHHH (SEQ ID NO:2)

The Full DNA Sequence of Expression Construct codon-optimized MTHFD2 sequence in pET-30a(+) was as follows:

TGGCGAATGGGACGCGCCCTGTAGCGGCGCATTAAGCGCGGCGGGTGTGGTGGTT
ACGCGCAGCGTGACCGCTACACTTGCCAGCGCCCTAGCGCCCGCTCCTTTCGCTTT
CTTCCCTTCCTTTCTCGCCACGTTCGCCGGCTTTCCCCGTCAAGCTCTAAATCGGG
GGCTCCCTTTAGGGTTCCGATTTAGTGCTTTACGGCACCTCGACCCCAAAAAACTT
GATTAGGGTGATGGTTCACGTAGTGGGCCATCGCCCTGATAGACGGTTTTTCGCCC
TTTGACGTTGGAGTCCACGTTCTTTAATAGTGGACTCTTGTTCCAAACTGGAACAA
CACTCAACCCTATCTCGGTCTATTCTTTTGATTTATAAGGGATTTTGCCGATTTCGG
CCTATTGGTTAAAAAATGAGCTGATTTAACAAAAATTTAACGCGAATTTTAACAA
AATATTAACGTTTACAATTTCAGGTGGCACTTTTCGGGGAAATGTGCGCGGAACC
CCTATTTGTTTATTTTTCTAAATACATTCAAATATGTATCCGCTCATGAATTAATTC
TTAGAAAAACTCATCGAGCATCAAATGAAACTGCAATTTATTCATATCAGGATTA
TCAATACCATATTTTTGAAAAAGCCGTTTCTGTAATGAAGGAGAAAACTCACCGA
GGCAGTTCCATAGGATGGCAAGATCCTGGTATCGGTCTGCGATTCCGACTCGTCC
AACATCAATACAACCTATTAATTTCCCCTCGTCAAAAATAAGGTTATCAAGTGAG
AAATCACCATGAGTGACGACTGAATCCGGTGAGAATGGCAAAAGTTTATGCATTT
CTTTCCAGACTTGTTCAACAGGCCAGCCATTACGCTCGTCATCAAAATCACTCGCA

TCAACCAAACCGTTATTCATTCGTGATTGCGCCTGAGCGAGACGAAATACGCGAT
CGCTGTTAAAAGGACAATTACAAACAGGAATCGAATGCAACCGGCGCAGGAACA
CTGCCAGCGCATCAACAATATTTTCACCTGAATCAGGATATTCTTCTAATACCTGG
AATGCTGTTTTCCCGGGGATCGCAGTGGTGAGTAACCATGCATCATCAGGAGTAC
GGATAAAATGCTTGATGGTCGGAAGAGGCATAAATTCCGTCAGCCAGTTTAGTCT
GACCATCTCATCTGTAACATCATTGGCAACGCTACCTTTGCCATGTTTCAGAAACA
ACTCTGGCGCATCGGGCTTCCCATACAATCGATAGATTGTCGCACCTGATTGCCCG
ACATTATCGCGAGCCCATTTATACCCATATAAATCAGCATCCATGTTGGAATTTAA
TCGCGGCCTAGAGCAAGACGTTTCCCGTTGAATATGGCTCATAACACCCCTTGTAT
TACTGTTTATGTAAGCAGACAGTTTTATTGTTCATGACCAAAATCCCTTAACGTGA
GTTTTCGTTCCACTGAGCGTCAGACCCCGTAGAAAAGATCAAAGGATCTTCTTGA
GATCCTTTTTTTCTGCGCGTAATCTGCTGCTTGCAAACAAAAAAACCACCGCTACC
AGCGGTGGTTTGTTTGCCGGATCAAGAGCTACCAACTCTTTTTCCGAAGGTAACTG
GCTTCAGCAGAGCGCAGATACCAAATACTGTCCTTCTAGTGTAGCCGTAGTTAGG
CCACCACTTCAAGAACTCTGTAGCACCGCCTACATACCTCGCTCTGCTAATCCTGT
TACCAGTGGCTGCTGCCAGTGGCGATAAGTCGTGTCTTACCGGGTTGGACTCAAG
ACGATAGTTACCGGATAAGGCGCAGCGGTCGGGCTGAACGGGGGGTTCGTGCAC
ACAGCCCAGCTTGGAGCGAACGACCTACACCGAACTGAGATACCTACAGCGTGA
GCTATGAGAAAGCGCCACGCTTCCCGAAGGGAGAAAGGCGGACAGGTATCCGGT
AAGCGGCAGGGTCGGAACAGGAGAGCGCACGAGGGAGCTTCCAGGGGGAAACG
CCTGGTATCTTTATAGTCCTGTCGGGTTTCGCCACCTCTGACTTGAGCGTCGATTTT
TGTGATGCTCGTCAGGGGGGCGGAGCCTATGGAAAAACGCCAGCAACGCGGCCTT
TTTACGGTTCCTGGCCTTTTGCTGGCCTTTTGCTCACATGTTCTTTCCTGCGTTATC
CCCTGATTCTGTGGATAACCGTATTACCGCCTTTGAGTGAGCTGATACCGCTCGCC
GCAGCCGAACGACCGAGCGCAGCGAGTCAGTGAGCGAGGAAGCGGAAGAGCGC
CTGATGCGGTATTTTCTCCTTACGCATCTGTGCGGTATTTCACACCGCATATATGG
TGCACTCTCAGTACAATCTGCTCTGATGCCGCATAGTTAAGCCAGTATACACTCCG
CTATCGCTACGTGACTGGGTCATGGCTGCGCCCCGACACCCGCCAACACCCGCTG
ACGCGCCCTGACGGGCTTGTCTGCTCCCGGCATCCGCTTACAGACAAGCTGTGAC
CGTCTCCGGGAGCTGCATGTGTCAGAGGTTTTCACCGTCATCACCGAAACGCGCG
AGGCAGCTGCGGTAAAGCTCATCAGCGTGGTCGTGAAGCGATTCACAGATGTCTG
CCTGTTCATCCGCGTCCAGCTCGTTGAGTTTCTCCAGAAGCGTTAATGTCTGGCTT
CTGATAAAGCGGGCCATGTTAAGGGCGGTTTTTTCCTGTTTGGTCACTGATGCCTC
CGTGTAAGGGGGATTTCTGTTCATGGGGGTAATGATACCGATGAAACGAGAGAGG
ATGCTCACGATACGGGTTACTGATGATGAACATGCCCGGTTACTGGAACGTTGTG
AGGGTAAACAACTGGCGGTATGGATGCGGCGGGACCAGAGAAAAATCACTCAGG
GTCAATGCCAGCGCTTCGTTAATACAGATGTAGGTGTTCCACAGGGTAGCCAGCA
GCATCCTGCGATGCAGATCCGGAACATAATGGTGCAGGGCGCTGACTTCCGCGTT
TCCAGACTTTACGAAACACGGAAACCGAAGACCATTCATGTTGTTGCTCAGGTCG
CAGACGTTTTGCAGCAGCAGTCGCTTCACGTTCGCTCGCGTATCGGTGATTCATTC
TGCTAACCAGTAAGGCAACCCCGCCAGCCTAGCCGGGTCCTCAACGACAGGAGC
ACGATCATGCGCACCCGTGGGGCCGCCATGCCGGCGATAATGGCCTGCTTCTCGC
CGAAACGTTTGGTGGCGGGACCAGTGACGAAGGCTTGAGCGAGGGCGTGCAAGA
TTCCGAATACCGCAAGCGACAGGCCGATCATCGTCGCGCTCCAGCGAAAGCGGTC
CTCGCCGAAAATGACCCAGAGCGCTGCCGGCACCTGTCCTACGAGTTGCATGATA
AAGAAGACAGTCATAAGTGCGGCGACGATAGTCATGCCCCGCGCCCACCGGAAG
GAGCTGACTGGGTTGAAGGCTCTCAAGGGCATCGGTCGAGATCCCGGTGCCTAAT
GAGTGAGCTAACTTACATTAATTGCGTTGCGCTCACTGCCCGCTTTCCAGTCGGGA
AACCTGTCGTGCCAGCTGCATTAATGAATCGGCCAACGCGCGGGGAGAGGCGGTT
TGCGTATTGGGCGCCAGGGTGGTTTTTCTTTTCACCAGTGAGACGGGCAACAGCT
GATTGCCCTTCACCGCCTGGCCCTGAGAGAGTTGCAGCAAGCGGTCCACGCTGGT
TTGCCCCAGCAGGCGAAAATCCTGTTTGATGGTGGTTAACGGCGGGATATAACAT
GAGCTGTCTTCGGTATCGTCGTATCCCACTACCGAGATGTCCGCACCAACGCGCA
GCCCGGACTCGGTAATGGCGCGCATTGCGCCCAGCGCCATCTGATCGTTGGCAAC
CAGCATCGCAGTGGGAACGATGCCCTCATTCAGCATTTGCATGGTTTGTTGAAAA
CCGGACATGGCACTCCAGTCGCCTTCCCGTTCCGCTATCGGCTGAATTTGATTGCG
AGTGAGATATTTATGCCAGCCAGCCAGACGCAGACGCGCCGAGACAGAACTTAAT
GGGCCCGCTAACAGCGCGATTTGCTGGTGACCCAATGCGACCAGATGCTCCACGC
CCAGTCGCGTACCGTCTTCATGGGAGAAAATAATACTGTTGATGGGTGTCTGGTC
AGAGACATCAAGAAATAACGCCGGAACATTAGTGCAGGCAGCTTCCACAGCAAT

GGCATCCTGGTCATCCAGCGGATAGTTAATGATCAGCCCACTGACGCGTTGCGCG
AGAAGATTGTGCACCGCCGCTTTACAGGCTTCGACGCCGCTTCGTTCTACCATCGA
CACCACCACGCTGGCACCCAGTTGATCGGCGCGAGATTTAATCGCCGCGACAATT
TGCGACGGCGCGTGCAGGGCCAGACTGGAGGTGGCAACGCCAATCAGCAACGAC
TGTTTGCCCGCCAGTTGTTGTGCCACGCGGTTGGGAATGTAATTCAGCTCCGCCAT
CGCCGCTTCCACTTTTTCCCGCGTTTTCGCAGAAACGTGGCTGGCCTGGTTCACCA
CGCGGGAAACGGTCTGATAAGAGACACCGGCATACTCTGCGACATCGTATAACGT
TACTGGTTTCACATTCACCACCCTGAATTGACTCTCTTCCGGGCGCTATCATGCCA
TACCGCGAAAGGTTTTGCGCCATTCGATGGTGTCCGGGATCTCGACGCTCTCCCTT
ATGCGACTCCTGCATTAGGAAGCAGCCCAGTAGTAGGTTGAGGCCGTTGAGCACC
GCCGCCGCAAGGAATGGTGCATGCAAGGAGATGGCGCCCAACAGTCCCCCGGCC
ACGGGGCCTGCCACCATACCCACGCCGAAACAAGCGCTCATGAGCCCGAAGTGG
CGAGCCCGATCTTCCCCATCGGTGATGTCGGCGATATAGGCGCCAGCAACCGCAC
CTGTGGCGCCGGTGATGCCGGCCACGATGCGTCCGGCGTAGAGGATCGAGATCGA
TCTCGATCCCGCGAAATTAATACGACTCACTATAGGGGAATTGTGAGCGGATAAC
AATTCCCCTCTAGAAATAATTTTGTTTAACTTTAAGAAGGAGATATACATATGGAG
GCCGTGGTTATCAGTGGCCGCAAGCTGGCCCAGCAGATCAAGCAGGAGGTGCGC
CAAGAAGTGGAAGAATGGGTTGCCAGCGGCAACAAGCGCCCGCATCTGAGCGTG
ATCCTGGTGGGCGAGAACCCGGCAAGCCACAGCTACGTGCTGAACAAAACACGC
GCAGCAGCAGTGGTGGGCATCAACAGCGAGACAATCATGAAACCGGCCAGCATC
AGCGAGGAAGAGTTACTGAACTTAATTAACAAGCTGAATAACGACGACAACGTG
GACGGCCTGCTGGTGCAGTTACCGCTGCCGGAACATATCGACGAACGCCGCATCT
GCAACGCCGTGAGTCCTGATAAGGACGTGGACGGCTTTCACGTGATCAATGTTGG
CCGCATGTGCTTAGACCAGTACAGCATGCTGCCGGCAACCCCTTGGGGCGTTTGG
GAGATCATCAAGCGCACCGGTATCCCGACCCTGGGTAAGAACGTTGTGGTGGCCG
GCCGTAGCAAGAACGTGGGCATGCCTATCGCAATGTTACTGCACACCGACGGCGC
ACATGAACGTCCTGGTGGCGATGCAACCGTTACCATCAGTCACCGTTACACCCCG
AAGGAGCAACTGAAAAAGCACACCATCCTGGCCGACATTGTGATCAGCGCAGCC
GGCATTCCGAACCTGATCACCGCAGACATGATCAAAGAGGGCGCCGCCGTGATCG
ACGTGGGCATTAACCGCGTGCACGATCCGGTGACAGCCAAACCGAAGCTGGTGG
GTGACGTGGACTTCGAGGGCGTGCGTCAAAAAGCCGGCTACATCACCCCGGTTCC
TGGTGGCGTTGGCCCTATGACCGTGGCCATGCTGATGAAGAACACCATCATTGCC
GCCAAGAAGGTGCTGCGTCTGGAGGAGCGCGAGCACCATCATCACCACCACTAA
GACGACGACGACAAGGCCATGGCTGATATCGGATCCGAATTCGAGCTCCGTCGAC
AAGCTTGCGGCCGCACTCGAGTGAGATCCGGCTGCTAACAAAGCCCGAAAGGAA
GCTGAGTTGGCTGCTGCCACCGCTGAGCAATAACTAGCATAACCCCTTGGGGCCT
CTAAACGGGTCTTGAGGGGTTTTTTGCTGAAAGGAGGAACTATATCCGGAT (SEQ ID NO:3)

MTHFD2 was expressed with Invitrogen One Shot BL21 Star (DE3) Chemically Competent *E. coli* cells as reported (Christensen et al., The Journal of biological chemistry. 2005; 280:34316-34323) with the modification that bacteria were grown in Luria-Bertani broth supplemented with kanamycin. Expression was induced with 1 mM IPTG and the cells were grown for 3 hours post-induction at 37°C before harvest. The bacteria were lysed with a sonicator, and MTHFD2 was nickel-purified using GE Ni-Sepharose 6 Fast Flow Resin and buffers modified from Christensen et al.

**Example 12: Assay for Inhibitors or MTHFD2**

The NAD-dependent methylenetetrahydrofolate dehydrogenase / cyclohydrolase activity assay employed in this microtiter well based screen is based on previously published enzymatic assays, with modifications as listed below. The assay was initially reported by Scrimgeour and Huennekens, Biochem. Biophys. Res. Commun. 1960, 2:230-233 and subsequently by Mejia and MacKenzie, The Journal of biological chemistry. 1985; 260:14616-14620). The assay was performed as described with slight modifications (reported assay concentrations in the Mejia et al. assay are provided in brackets below). The modified assay utilized 50 ng of recombinant MTHFD2 protein produced as described in Example 11 per well in 384 well format. In addition, the present assay utilized 30 uM potassium phosphate buffer (Mejia: 25 uM potassium phosphate buffer), at pH 7.5 (7.3), with 150 uM formaldehyde (2.5 mM), 150 uM tetrahydrofolate (250 uM) and 6 mM MgCl (5 mM). No 2-mercaptoethanol or reducing agents were used in the present assay, which is crucial for detection of cysteine modifying compounds as potential enzyme inhibitors. The assay employed for high-throughput screening for chemical inhibitors used the final endpoint measure of formation of 5,10-methenyltetrahydrofolate by spectrophotometric absorbance at 340 nm.

Using the recombinant protein and modified assay in microtiter 384 well plate format, the enzyme kinetics for MTHFD2 (Km for NAD and CH2-THF) were comparable to published values (published enzyme values provided in (Yang and MacKenzie Biochemistry. 1993;32:11118-11123)).

| | **Published** | **Measured** |
|---|---|---|
| Km of NAD+ (μM) | 56.4 (63±20) | 51.5 |
| Km of 5,10 CH2-THF (μM) | 6.7 (4.6±2.1) | 4.1 |
| kcat ($s^{-1}$) | 81 | ND |
| kcat/Km for 5,10 $CH_2$-THF($s^{-1}M^{-1}$) | 1.2 x $10^7$ | ND |
| ND: not determined | | |

**Example 13. High Throughput Screen Identifies Cysteine-Modifying Agents**

The assay described in Example 12 was used to screen a library of about 5,100 small molecule agents in the Prestwick chemical library, Known Bioactives Library and Library of Pharmacologically Active Compounds, in biological duplicate. A number of small molecular agents were found to inhibit the enzyme, including 6-hydroxy-DL DOPA, calmidazolium chloride, CDOO, ebselen, celastrol, GW5074, iodoacetamide, para-benzoquinone, and protoporphyrin IX disodium. Several of the high scoring inhibitors are known cysteine modifiers that could in principle inhibit enzyme activity by modification of cysteine residues.

In secondary testing, nine of the compounds identified in the initial screen were examined in an 8-concentration dose response assay, including 6-hydroxy-DL DOPA, calmidazolium chloride, CDOO*, ebselen*, celestrol*, GW5074, iodoacetamide*, para-benzoquinone*, and protoporphyrin IX disodium. Compounds with known capacity for cysteine modification are denoted by asterisk. The full protein sequence of human MTHFD2 has 3 cysteine amino acids:

```
MAATSLMSAL AARLLQPAHS CSLRLRPFHL AAVRNEAVVI SGRKLAQQIK QEVRQEVEEW 60
VASGNKRPHL SVILVGENPA SHSYVLNKTR AAAVVGINSE TIMKPASISE EELLNLINKL 130
NNDDNVDGLL VQLPLPEHID ERRICNAVSP DKDVDGFHVI NVGRMCLDQY SMLPATPWGV 190
WEIIKRTGIP TLGKNVVVAG RSKNVGMPIA MLLHTDGAHE RPGGDATVTI SHRYTPKEQL 250
KKHTILADIV ISAAGIPNLI TADMIKEGAA VIDVGINRVH DPVTAKPKLV GDVDFEGVRQ 310
KAGYITPVPG GVGPMTVAML MKNTIIAAKK VLRLEEREVL KSKELGVATN (SEQ ID NO:4)
```

The first cysteine (C21) is within the mitochondrial targeting sequence and is cleaved during the maturation process when the protein is imported into the mitochondria. This leaves two cysteines at C145 and C166 as candidate cysteines that may be modified by the small molecule agents identified herein.

Three mutant MTHFD2 constructs were generated with Agilent QuikChange Lightening, with cysteine to serine mutations at C145, C166 or both C145/C166. These mutants were retested with three of the compounds identified in the secondary screen (6-hydroxy-DL-DOPA, Celastrol, and Ebselen). 6-hydroxy-DL-DOPA is not a known cysteine modifying agent (negative control) and inhibited wild type, C145, C166 and C145/C166 proteins to a similar degree, and was not antagonized by the addition of the reducing agent DTT (Figure 9A). The cysteine modifying agent ebselen (Figure 9C) inhibited the wild type MTHFD2 and C166 mutant, whereas C145 and C145/C166 mutant proteins, or addition of the reducing agent DTT to wild type MTHFD2 protein, were resistant to ebselen, suggesting that C145 is the critical cysteine residue for ebselen. The cysteine modifying agent celestrol demonstrated partial inhibition in C145 or C166 mutant proteins, with loss of inhibition in C145/C166 double mutants or with addition of DTT, suggesting that celestrol may antagonize MTHFD2 additively through C145 and C166 (Figure 9B). Given the identification of these cysteine residues, additional structure/medicinal chemistry may be employed to specifically design small molecules that take advantage of these cysteine residues and inhibit MTHFD2 with greater selectivity.

In fact, examination of the sequence alignment between MTHFD2 relative to MTHFD1 revealed that the identified critical cysteine residues (C145 and C166) are present only in MTHFD2 and MTHFD2L, not MTHFD1, suggesting that these cysteine residues in MTHFD2 may be targeted in the development of novel enzyme inhibitors.

REFERENCES

1. Nowell PC. The clonal evolution of tumor cell populations. Science. 1976;194:23-28

2. Hanahan D, Weinberg RA. Hallmarks of cancer: The next generation. Cell. 2011;144:646-674

3. Stratton MR, Campbell PJ, Futreal PA. The cancer genome. Nature. 2009;458:719-724

4. Hsu PP, Sabatini DM. Cancer cell metabolism: Warburg and beyond. Cell. 2008;134:703-707

5. Sreekumar A, Poisson LM, Rajendiran TM, Khan AP, Cao Q, Yu J, Laxman B, Mehra R, Lonigro RJ, Li Y, Nyati MK, Ahsan A, Kalyana-Sundaram S, Han B, Cao X, Byun J, Omenn GS, Ghosh D, Pennathur S, Alexander DC, Berger A, Shuster JR, Wei JT, Varambally S, Beecher C, Chinnaiyan AM. Metabolomic profiles delineate potential role for sarcosine in prostate cancer progression. Nature. 2009;457:910-914

6. DeBerardinis RJ, Mancuso A, Daikhin E, Nissim I, Yudkoff M, Wehrli S, Thompson CB. Beyond aerobic glycolysis: Transformed cells can engage in glutamine metabolism that exceeds the requirement for protein and nucleotide synthesis. Proc Natl Acad Sci USA. 2007;104:19345-19350

7. Anderson DD, Quintero CM, Stover PJ. Identification of a de novo thymidylate biosynthesis pathway in mammalian mitochondria. Proc Natl Acad Sci USA. 2011;108:15163-15168

8. McEntee G, Minguzzi S, O'Brien K, Ben Larbi N, Loscher C, O'Fagain C, Parle-McDermott A. The former annotated human pseudogene dihydrofolate reductase-like 1 (dhfrll) is expressed and functional. Proc Natl Acad Sci USA. 2011;108:15157-15162

9. Samsonoff WA, Reston J, McKee M, O'Connor B, Galivan J, Maley G, Maley F. Intracellular location of thymidylate synthase and its state of phosphorylation. The Journal of biological chemistry. 1997;272:13281-13285

10. Mejia NR, MacKenzie RE. Nad-dependent methylenetetrahydrofolate dehydrogenase is expressed by immortal cells. The Journal of biological chemistry. 1985;260:14616-14620

11. Tibbetts AS, Appling DR. Compartmentalization of mammalian folate-mediated one-carbon metabolism. Annu Rev Nutr. 2010;30:57-81

12. Bolusani S, Young BA, Cole NA, Tibbetts AS, Momb J, Bryant JD, Solmonson A, Appling DR. Mammalian mthfd21 encodes a mitochondrial methylenetetrahydrofolate dehydrogenase isozyme expressed in adult tissues. The Journal of biological chemistry. 2011;286:5166-5174

13. Yousif LF, Stewart KM, Kelley SO. Targeting mitochondria with organelle-specific compounds: Strategies and applications. Chembiochem : a European journal of chemical biology. 2009;10:1939-1950

14. Filipovska A, Kelso GF, Brown SE, Beer SM, Smith RA, Murphy MP. Synthesis and characterization of a triphenylphosphonium-conjugated peroxidase mimetic. Insights into the interaction of ebselen with mitochondria. The Journal of biological chemistry. 2005;280:24113-24126

15. Serafimova IM, Pufall MA, Krishnan S, Duda K, Cohen MS, Maglathlin RL, McFarland JM, Miller RM, Frodin M, Taunton J. Reversible targeting of noncatalytic cysteines with chemically tuned electrophiles. Nature chemical biology. 2012;8:471-476

16. Singh J, Petter RC, Baillie TA, Whitty A. The resurgence of covalent drugs. Nature reviews. Drug discovery. 2011;10:307-317

17. Shoemaker RH. The nci60 human tumour cell line anticancer drug screen. Nat Rev Cancer. 2006;6:813-823

18. Allen J, Davey HM, Broadhurst D, Heald JK, Rowland JJ, Oliver SG, Kell DB. High-throughput classification of yeast mutants for functional genomics using metabolic footprinting. Nat Biotechnol. 2003;21:692-696

19. Shaham O, Slate NG, Goldberger O, Xu Q, Ramanathan A, Souza AL, Clish CB, Sims KB, Mootha VK. A plasma signature of human mitochondrial disease revealed through metabolic profiling of spent media from cultured muscle cells. Proc Natl AcadSci USA. 2010;107:1571-1575

20. O'Connor PM, Jackman J, Bae I, Myers TG, Fan S, Mutoh M, Scudiero DA, Monks A, Sausville EA, Weinstein JN, Friend S, Fornace AJ, Jr., Kohn KW. Characterization of the p53 tumor suppressor pathway in cell lines of the national cancer institute anticancer drug screen and correlations with the growth-inhibitory potency of 123 anticancer agents. Cancer Res. 1997;57:4285-4300

21. Katz-Brull R, Degani H. Kinetics of choline transport and phosphorylation in human breast cancer cells; nmr application of the zero trans method. Anticancer Res. 1996;16:1375-1380

22. Duarte NC, Becker SA, Jamshidi N, Thiele I, Mo ML, Vo TD, Srivas R, Palsson BO. Global reconstruction of the human metabolic network based on genomic and bibliomic data. Proc Natl Acad Sci USA. 2007;104:1777-1782

23. *Materials and methods are available as supplementary material on science online.*

24. Nikiforov MA, Chandriani S, O'Connell B, Petrenko O, Kotenko I, Beavis A, Sedivy JM, Cole MD. A functional screen for myc-responsive genes reveals serine hydroxymethyltransferase, a major source of the one-carbon unit for cell metabolism. Mol Cell Biol. 2002;22:5793-5800

25. Kao F, Chasin L, Puck TT. Genetics of somatic mammalian cells. X. Complementation analysis of glycine-requiring mutants. Proc Natl Acad Sci USA. 1969;64:1284-1291

26. Zhang WC, Shyh-Chang N, Yang H, Rai A, Umashankar S, Ma S, Soh BS, Sun LL, Tai BC, Nga ME, Bhakoo KK, Jayapal SR, Nichane M, Yu Q, Ahmed DA, Tan C, Sing WP, Tam J, Thiruganànam A, Noghabi MS, Huei Pang Y, Ang HS, Robson P, Kaldis P, Soo RA, Swarup S, Lim EH, Lim B. Glycine decarboxylase activity drives non-small cell lung cancer tumor-initiating cells and tumorigenesis. Cell. 2012;148:259-272

27. Patel H, Pietro ED, MacKenzie RE. Mammalian fibroblasts lacking mitochondrial nad+-dependent methylenetetrahydrofolate dehydrogenase-cyclohydrolase are glycine auxotrophs. The Journal of biological chemistry. 2003;278:19436-19441

28. Chin K, DeVries S, Fridlyand J, Spellman PT, Roydasgupta R, Kuo WL, Lapuk A, Neve RM, Qian Z, Ryder T, Chen F, Feiler H, Tokuyasu T, Kingsley C, Dairkee S, Meng Z, Chew K, Pinkel D, Jain A, Ljung BM, Esserman L, Albertson DG, Waldman FM, Gray JW. Genomic and transcriptional aberrations linked to breast cancer pathophysiologies. Cancer Cell. 2006;10:529-541

29. Desmedt C, Piette F, Loi S, Wang Y, Lallemand F, Haibe-Kains B, Viale G, Delorenzi M, Zhang Y, d'Assignies MS, Bergh J, Lidereau R, Ellis P, Harris AL, Klijn JG, Foekens JA, Cardoso F, Piccart MJ, Buyse M, Sotiriou C. Strong time dependence of the 76-gene prognostic signature for node-negative breast cancer patients in the transbig multicenter independent validation series. Clin Cancer Res. 2007;13:3207-3214

30. Pawitan Y, Bjohle J, Amler L, Borg AL, Egyhazi S, Hall P, Han X, Holmberg L, Huang F, Klaar S, Liu ET, Miller L, Nordgren H, Ploner A, Sandelin K, Shaw PM, Smeds J, Skoog L, Wedren S, Bergh J. Gene expression profiling spares early breast cancer patients from adjuvant therapy: Derived and validated in two population-based cohorts. Breast Cancer Res. 2005;7:R953-964

31. van de Vijver MJ, He YD, van't Veer LJ, Dai H, Hart AA, Voskuil DW, Schreiber GJ, Peterse JL, Roberts C, Marton MJ, Parrish M, Atsma D, Witteveen A, Glas A, Delahaye L, van der Velde T, Bartelink H, Rodenhuis S, Rutgers ET, Friend SH, Bernards R. A gene-expression signature as a predictor of survival in breast cancer. N Engl J Med. 2002;347:1999-2009

32. Kao KJ, Chang KM, Hsu HC, Huang AT. Correlation of microarray-based breast cancer molecular subtypes and clinical outcomes: Implications for treatment optimization. BMC Cancer. 2011;11:143

33. Miller LD, Smeds J, George J, Vega VB, Vergara L, Ploner A, Pawitan Y, Hall P, Klaar S, Liu ET, Bergh J. An expression signature for p53 status in human breast cancer predicts mutation status, transcriptional effects, and patient survival. Proc Natl Acad Sci U S A. 2005;102:13550-13555

34. Liu F, White JA, Antonescu C, Gusenleitner D, Quackenbush J. Gcod - genechip oncology database. BMC bioinformatics. 2011;12:46

35. Rhodes DR, Yu J, Shanker K, Deshpande N, Varambally R, Ghosh D, Barrette T, Pandey A, Chinnaiyan AM. Large-scale meta-analysis of cancer microarray data identifies common transcriptional profiles of neoplastic transformation and progression. Proc Natl Acad Sci U S A. 2004;101:9309-9314

36. Lehtinen L, Ketola K, Makela R, Mpindi JP, Viitala M, Kallioniemi O, Iljin K. High-throughput rnai screening for novel modulators of vimentin expression identifies mthfd2 as a regulator of breast cancer cell migration and invasion. Oncotarget. 2013;4:48-63

37. Selcuklu SD, Donoghue MT, Rehmet K, de Souza Gomes M, Fort A, Kovvuru P, Muniyappa MK, Kerin MJ, Enright AJ, Spillane C. Microrna-9 inhibition of cell proliferation and identification of novel mir-9 targets by transcriptome profiling in breast cancer cells. The Journal of biological chemistry. 2012;287:29516-29528

38. Smith GK, Banks SD, Monaco TJ, Rigual R, Duch DS, Mullin RJ, Huber BE. Activity of an nad-dependent 5,10-methylenetetrahydrofolate dehydrogenase in normal tissue, neoplastic cells, and oncogene-transformed cells. Archives of biochemistry and biophysics. 1990;283:367-371

39. Uhlen M, Bjorling E, Agaton C, Szigyarto CA, Amini B, Andersen E, Andersson AC, Angelidou P, Asplund A, Asplund C, Berglund L, Bergstrom K, Brumer H, Cerjan D, Ekstrom M, Elobeid A, Eriksson C, Fagerberg L, Falk R, Fall J, Forsberg M, Bjorklund MG, Gumbel K, Halimi A, Hallin I, Hamsten C, Hansson M, Hedhammar M, Hercules G, Kampf C, Larsson K, Lindskog M, Lodewyckx W, Lund J, Lundeberg J, Magnusson K, Malm E, Nilsson P, Odling J, Oksvold P, Olsson I, Oster E, Ottosson J, Paavilainen L, Persson A, Rimini R, Rockberg J, Runeson M, Sivertsson A, Skollermo A, Steen J, Stenvall M, Sterky F, Stromberg S, Sundberg M, Tegel H, Tourle S, Wahlund E, Walden A, Wan J, Wernerus H, Westberg J, Wester K, Wrethagen U, Xu LL, Hober S, Ponten F. A human protein atlas for normal and cancer tissues based on antibody proteomics. Molecular & cellular proteomics : MCP. 2005;4:1920-1932

40. Christensen KE, Mirza IA, Berghuis AM, Mackenzie RE. Magnesium and phosphate ions enable nad binding to methylenetetrahydrofolate dehydrogenase-methenyltetrahydrofolate cyclohydrolase. The Journal of biological chemistry. 2005;280:34316-34323

41. Yang XM, MacKenzie RE. Nad-dependent methylenetetrahydrofolate dehydrogenase-methenyltetrahydrofolate cyclohydrolase is the mammalian homolog of the mitochondrial enzyme encoded by the yeast mis1 gene. Biochemistry. 1993;32:11118-11123

## OTHER EMBODIMENTS

It is to be understood that while the invention has been described in conjunction with the detailed description thereof, the foregoing description is intended to illustrate and not limit the scope of the invention, which is defined by the scope of the appended claims.

## Claims

1. An antifolate drug for use in treating a cancer in a subject, comprising:

   (a) determining a level of SHMT2 protein, SHMT2 mRNA, or SHMT2 activity in a sample comprising tumor cells from a subject;

   comparing the level of SHMT2 protein, SHMT2mRNA, or SHMT2 activity in the sample to a reference level of SHMT2 protein, SHMT2 mRNA, or SHMT2 activity;
   selecting a subject who has a level of SHMT2 protein, SHMT2 mRNA, or SHMT2 activity above the reference level, and administering a therapeutically effective amount of an antifolate drug, wherein the antifolate drug is methotrexate, or any one of the compounds recited in Table 1.

2. The antifolate drug for the use of claim 1, wherein the antifolate drug is methotrexate.

3. The antifolate drug for the use of any one of claims 1 to 2, wherein the agent is linked covalently to a mitochondrial-targeting moiety.

4. The antifolate drug according to the use of claim 3, wherein the agent is linked covalently to mitochondrial-targeting moiety tetraphenylphosphonium.

5. A method of diagnosing cancer in a subject, the method comprising:

   determining a level of SHMT2 protein, SHMT2 mRNA, or SHMT2 activity in a sample suspected of comprising tumor cell from a subject;
   comparing the level of SHMT2 protein, SHMT2 mRNA, or SHMT2 activity in the sample to a reference level of SHMT2 protein, SHMT2 mRNA, or SHMT2 activity; and
   diagnosing the subject in which the sample has a level of SHMT2 above the reference level as having cancer, and selecting the treatment of an antifolate drug.

6. The antifolate drug for the use of any one of claims 1 to 4, or the method of claim 5, wherein the cancer or tumor is a carcinoma.

7. The antifolate drug for the use of claim 6, or the method of claim 6, wherein the carcinoma is glioma, glioblastoma, breast, ovarian, renal cell, lung; melanoma, cervical, adrenal, brain, esophagus, gastric, germ cell, head / neck, prostate, melanoma, liver, pancreas, testicular, or colon cancer;
   more preferably wherein the carcinoma is not breast or bladder cancer.

8. The antifolate drug for the use of claim 1, wherein the antifolate drug is any one of the compounds recited in Table 1.

## Patentansprüche

1. Antifolat-Arzneimittel zur Verwendung bei der Behandlung von Krebs in einem Subjekt, die Folgendes beinhaltet:

   (a) Bestimmen eines Niveaus von SHMT2-Protein, SHMT2 mRNA oder SHMT2-Aktivität in einer die Tumorzellen enthaltenden Probe von einem Subjekt;

Vergleichen des Niveaus von SHMT2-Protein, SHMT2 mRNA oder SHMT2-Aktivität in der Probe mit einem Referenzniveau von SHMT2-Protein, SHMT2 mRNA oder SHMT2-Aktivität;
Auswählen eines Subjekts, das ein SHMT2-Protein-, SHMT2 mRNA- oder SHMT2-Aktivitätsniveau über dem Referenzniveau hat, und Verabreichen einer therapeutisch wirksamen Menge eines Antifolat-Arzneimittels, wobei das Antifolat-Arzneimittel Methotrexat oder eine beliebige der in Tabelle 1 enthaltenen Verbindungen ist.

2. Antifolat-Arzneimittel zur Verwendung nach Anspruch 1, wobei das Antifolat-Arzneimittel Methotrexat ist.

3. Antifolat-Arzneimittel zur Verwendung nach einem der Ansprüche 1 bis 2, wobei das Mittel kovalent mit einem Mitochondrien-Targeting-Anteil verknüpft ist.

4. Antifolat-Arzneimittel gemäß der Verwendung nach Anspruch 3, wobei das Mittel kovalent mit dem Mitochondrien-Targeting-Anteil Tetraphenylphosphonium verknüpft ist.

5. Verfahren zum Diagnostizieren von Krebs in einem Subjekt, wobei das Verfahren Folgendes beinhaltet:

Bestimmen eines Niveaus von SHMT2-Protein, SHMT2 mRNA oder SHMT2-Aktivität in einer vermutlich Tumorzellen enthaltenden Probe von einem Subjekt;
Vergleichen des Niveaus von SHMT2-Protein, SHMT2 mRNA oder SHMT2-Aktivität in der Probe mit einem Referenzniveaus von SHMT2-Protein, SHMT2 mRNA oder SHMT2-Aktivität; und
Stellen einer Krebsdiagnose bei dem Subjekt, dessen Probe ein SHMT2-Niveau über dem Referenzniveau hat, und Auswählen einer Antifolat-Arzneimittelbehandlung.

6. Antifolat-Arzneimittel zur Verwendung nach einem der Ansprüche 1 bis 4 oder Verfahren nach Anspruch 5, wobei der Krebs oder Tumor ein Karzinom ist.

7. Antifolat-Arzneimittel zur Verwendung nach Anspruch 6 oder Verfahren nach Anspruch 6, wobei das Karzinom ein Gliom, Glioblastom, Brust-, Eierstock-, Nierenzell-, Lungenkrebs; ein Melanom, Gebärmutterhals-, Nebennieren-, Hirn-, Speiseröhren-, Magen-, Keimzellen-, Kopf/Hals-, Prostata-, ein Melanom, Leber-, Bauchspeicheldrüsen-, Hoden- oder Kolonkrebs ist;
wobei das Karzinom bevorzugter nicht Brust- oder Blasenkrebs ist.

8. Antifolat-Arzneimittel zur Verwendung nach Anspruch 1, wobei das Antifolat-Arzneimittel eine beliebige der in Tabelle 1 enthaltenen Verbindungen ist.

## Revendications

1. Médicament antifolique pour une utilisation dans le traitement d'un cancer chez un sujet, comprenant les étapes consistant à :

(a) déterminer un niveau d'activité d'une protéine SHMT2, SHMT2 ARNm ou SHMT2 dans un échantillon comprenant des cellules tumorales prélevées chez un sujet ;

comparer le niveau d'activité de la protéine SHMT2, SHMT2 ARNm, ou SHMT2 dans l'échantillon à un niveau d'activité de référence de la protéine SHMT2, SHMT2 ARNm ou SHMT2 ;
sélectionner un sujet qui a un niveau d'activité de la protéine SHMT2, SHMT2 ARNm ou SHMT2 supérieur au niveau de référence, et administrer une quantité thérapeutiquement efficace d'un médicament antifolique, le médicament antifolique étant le méthotrexate, ou l'un quelconque des composés mentionnés dans le Tableau 1.

2. Médicament antifolique pour une utilisation selon la revendication1, le médicament antifolique étant le méthotrexate.

3. Médicament antifolique pour une utilisation selon l'une quelconque des revendications 1 à 2, dans lequel l'agent est lié par covalence à une fraction de ciblage des mitochondries.

4. Médicament antifolique pour une utilisation selon la revendication 3, dans lequel l'agent est lié par covalence à un tétraphénylphosphonium d'une fraction de ciblage des mitochondries.

5. Procédé de diagnostic d'un cancer chez un sujet, le procédé comprenant les étapes consistant à :

déterminer un niveau d'activité de la protéine SHMT2, SHMT2 ARNm, ou SHMT2 dans un échantillon suspecté de contenir une cellule tumorale prélevé chez un sujet ;
comparer le niveau d'activité de la protéine SHMT2, SHMT2 ARNm ou SHMT2 dans l'échantillon par rapport à un niveau de référence de la protéine SHMT2, SHMT2 ARNm ou SHMT2 ; et
diagnostiquer le sujet dont l'échantillon qui a un niveau de SHMT2 supérieur au niveau de référence comme ayant un cancer et sélectionner le traitement par un médicament antifolique.

6. Médicament antifolique pour une utilisation selon l'une quelconque des revendications 1 à 4, ou procédé selon la revendication 5, le cancer ou la tumeur étant un carcinome.

7. Médicament antifolique pour utilisation selon la revendication 6, ou procédé selon la revendication 6, le carcinome étant un gliome, un glioblastome, un carcinome mammaire, un carcinome ovarien, un carcinome des cellules rénales, un cancer du poumon ; un mélanome, un cancer cervical, un cancer des glandes surrénales, un cancer du cerveau, un cancer de l'oesophage, un cancer gastrique, un cancer des cellules germinales, un cancer de la tête et du cou, un cancer de la prostate, un mélanome, un cancer du foie, un cancer du pancréas, un cancer des testicules ou un cancer du colon ;
plus préférablement, le carcinome n'étant pas un cancer du sein ou de la vessie.

8. Médicament antifolique selon la revendication 1, le médicament antifolique étant l'un quelconque des composés mentionnés dans le Tableau 1.

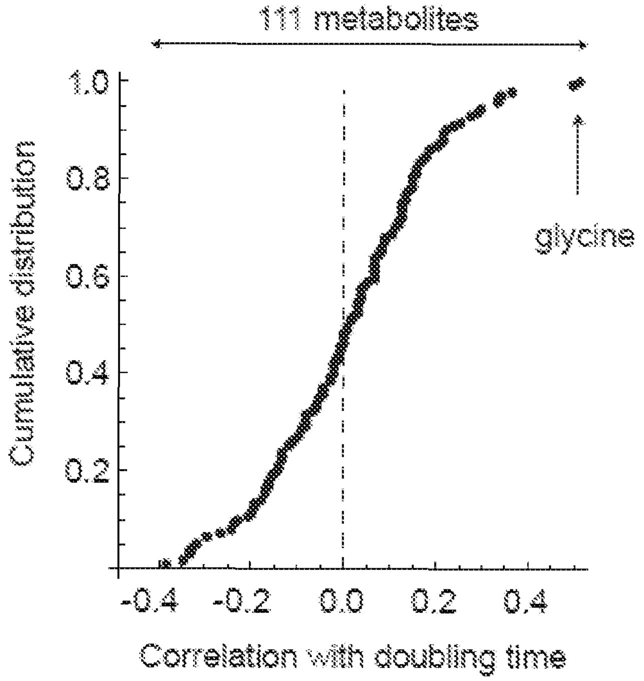
111 metabolites
1.0
0.8
0.6
0.4
0.2
Cumulative distribution
glycine
-0.4
-0.2
0.0
0.2
0.4
Correlation with doubling time

FIG. 1A

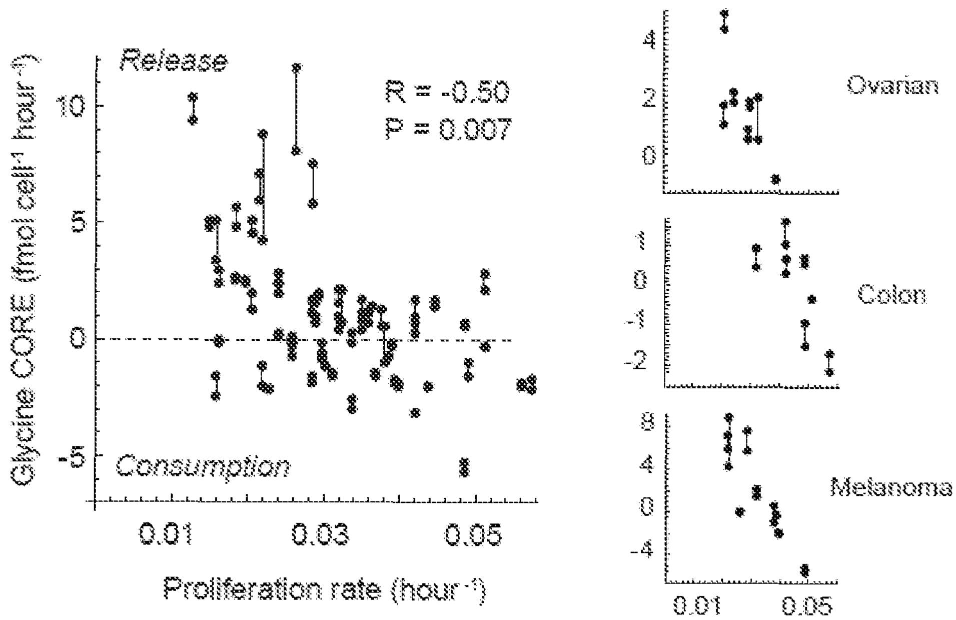
Release
R = -0.50
P = 0.007
10
5
0
-5
Glycine CORE (fmol cell-1 hour-1)
Consumption
0.01
0.03
0.05
Proliferation rate (hour-1)
4
2
0
Ovarian
1
0
-1
-2
Colon
8
4
0
-4
Melanoma
0.01
0.05

FIG. 1B

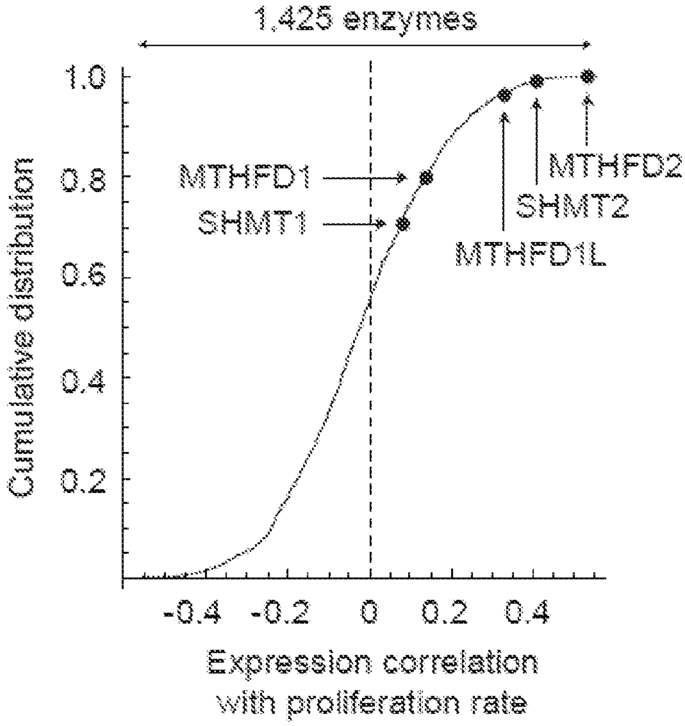
1,425 enzymes
1.0
0.8
0.6
0.4
0.2
Cumulative distribution
MTHFD1
SHMT1
MTHFD2
SHMT2
MTHFD1L
-0.4
-0.2
0
0.2
0.4
Expression correlation
with proliferation rate

FIG. 1C

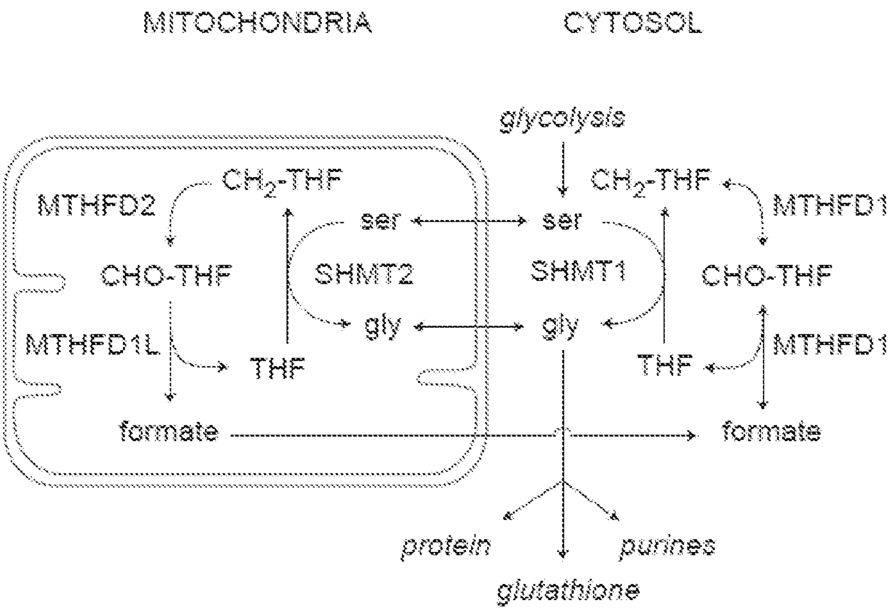
MITOCHONDRIA
CYTOSOL
glycolysis
MTHFD2
$CH_2$-THF
ser
ser
$CH_2$-THF
MTHFD1
CHO-THF
SHMT2
SHMT1
CHO-THF
MTHFD1L
gly
gly
MTHFD1
THF
THF
formate
formate
protein
purines
glutathione

FIG. 1D

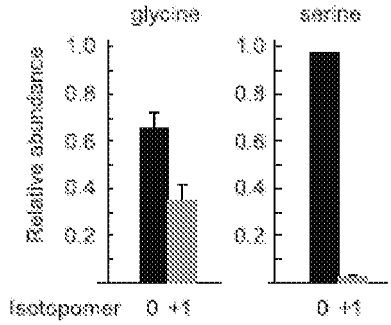


FIG. 1E

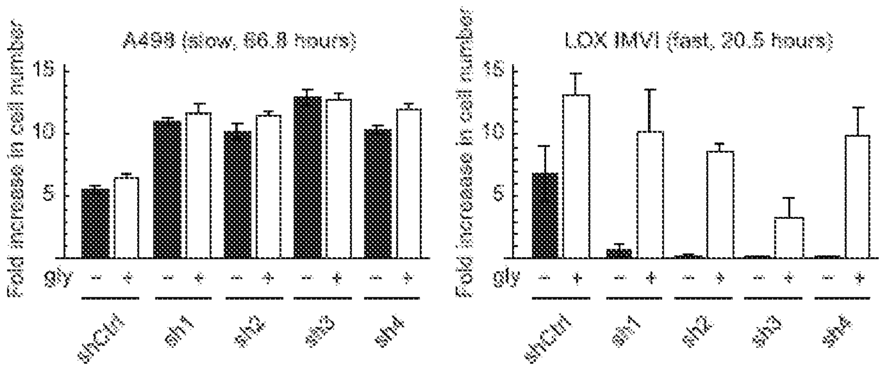


FIG. 1F

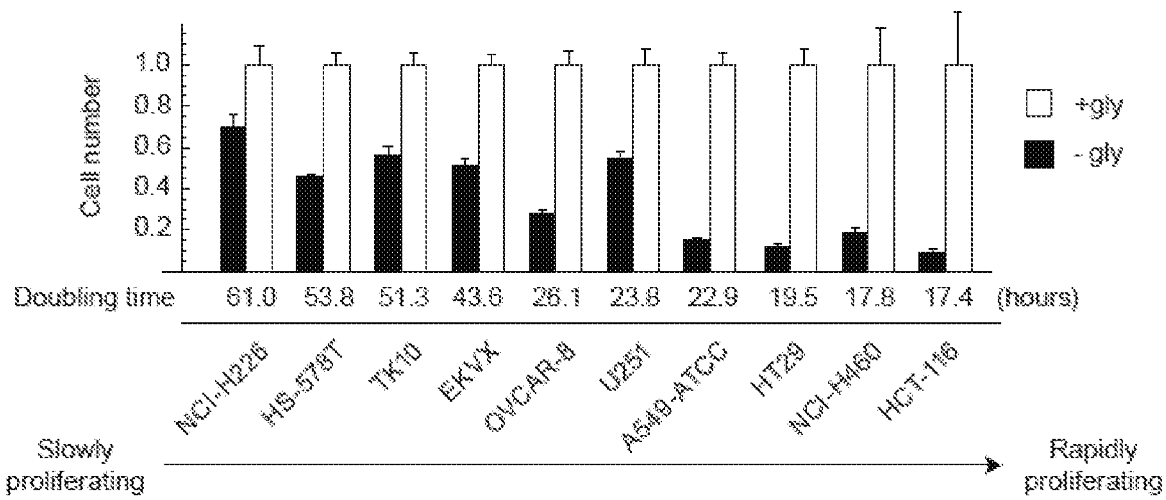


FIG. 1G

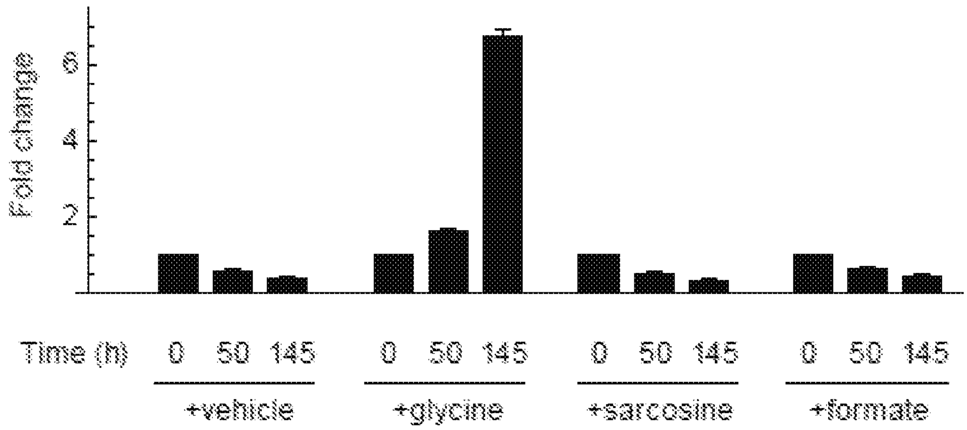
Fold change
6
4
2
Time (h)
0 50 145
0 50 145
0 50 145
0 50 145
+vehicle
+glycine
+sarcosine
+formate

FIG. 1H

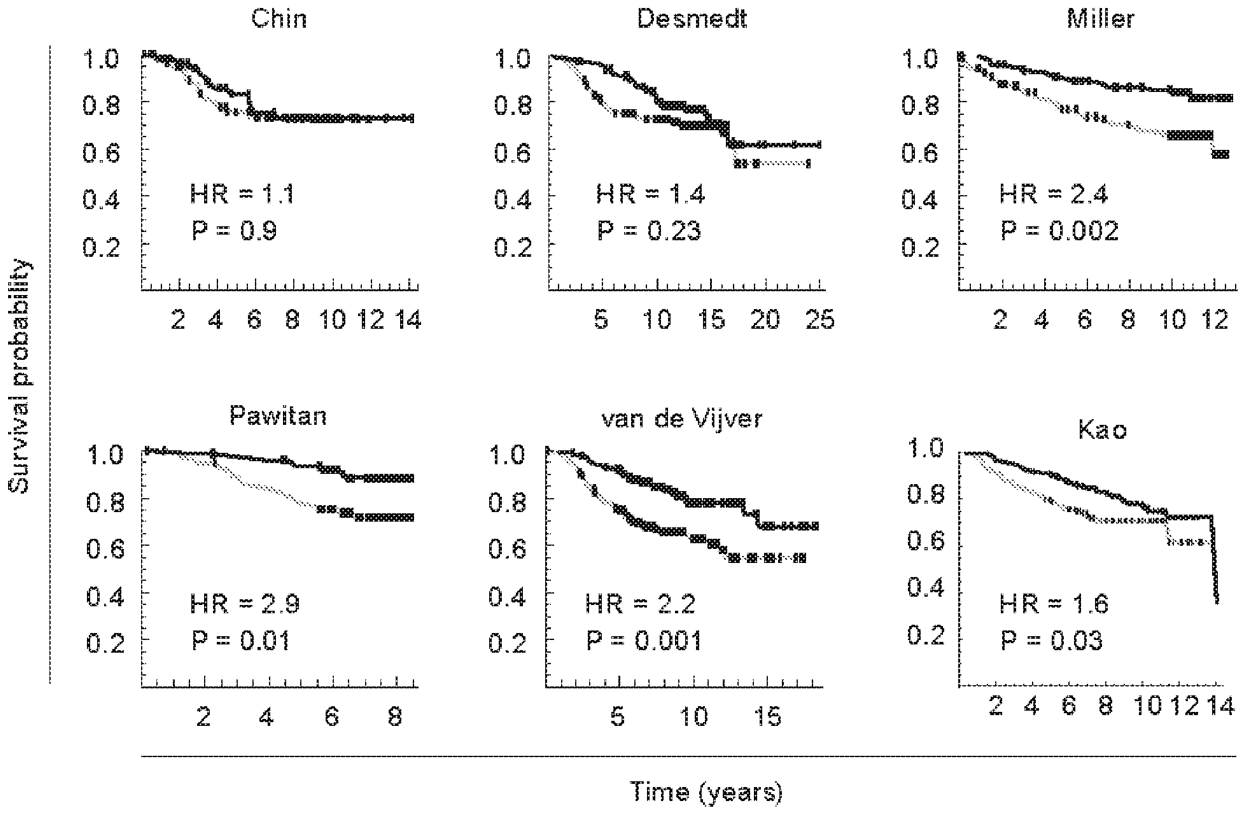
Chin
HR = 1.1
P = 0.9
Desmedt
HR = 1.4
P = 0.23
Miller
HR = 2.4
P = 0.002
Pawitan
HR = 2.9
P = 0.01
van de Vijver
HR = 2.2
P = 0.001
Kao
HR = 1.6
P = 0.03
Survival probability
Time (years)

FIG. 2A

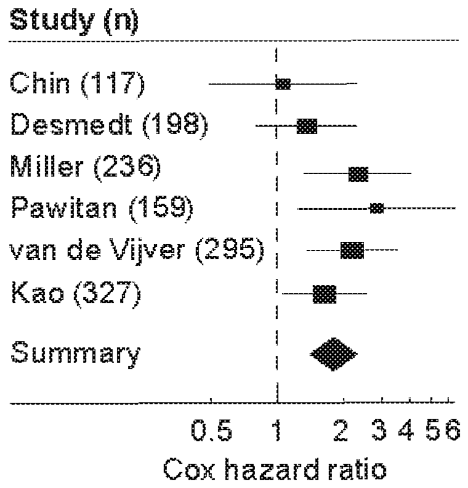
Study (n)
Chin (117)
Desmedt (198)
Miller (236)
Pawitan (159)
van de Vijver (295)
Kao (327)
Summary
0.5 1 2 3 4 5 6
Cox hazard ratio

FIG. 2B

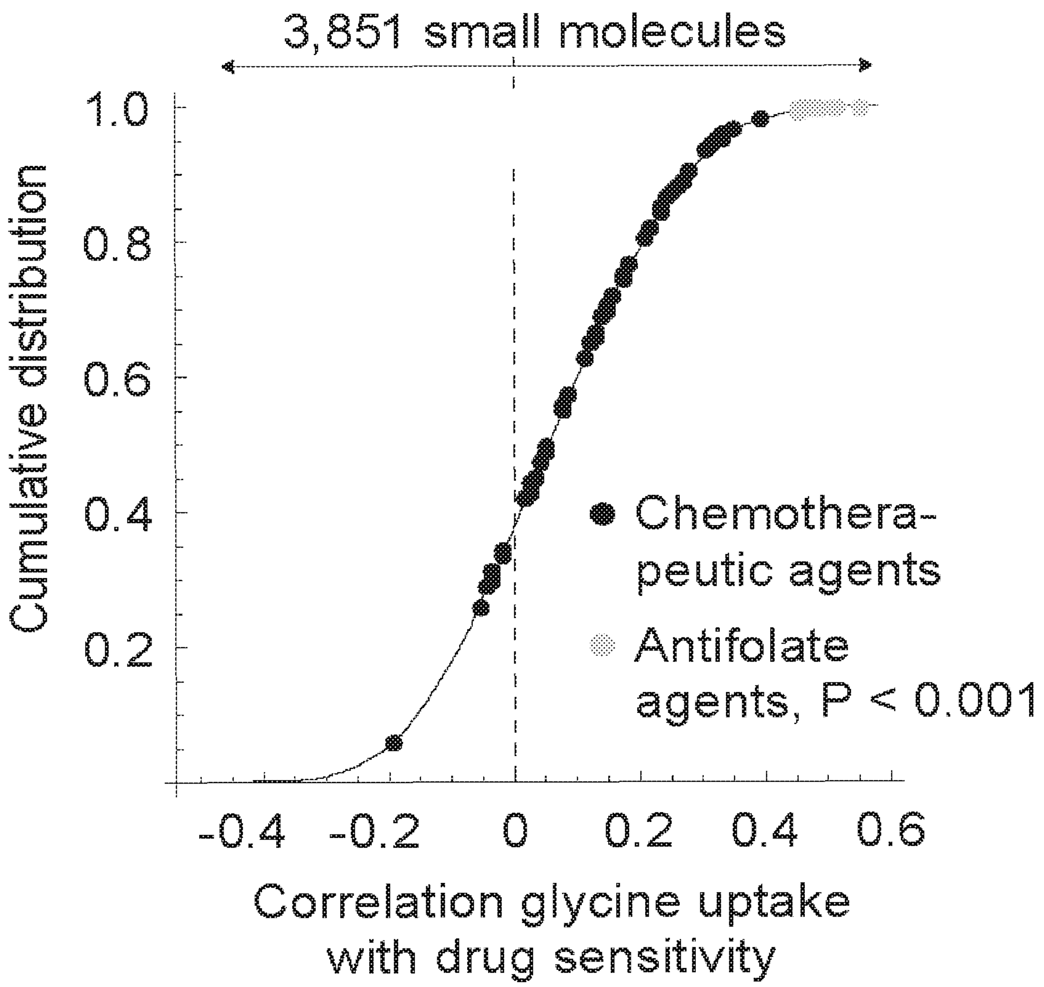
3,851 small molecules
Cumulative distribution
1.0
0.8
0.6
0.4
0.2
Chemotherapeutic agents
Antifolate agents, P < 0.001
-0.4 -0.2 0 0.2 0.4 0.6
Correlation glycine uptake with drug sensitivity

FIG. 3

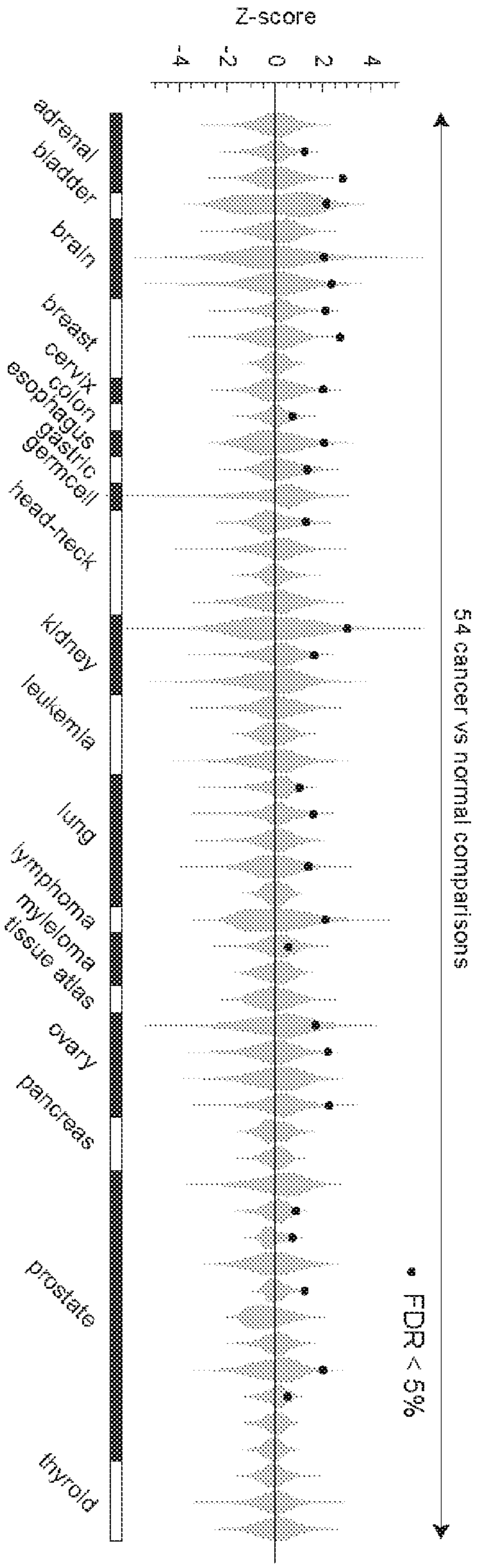
Z-score
-4
-2
0
2
4
adrenal
bladder
brain
breast
cervix
colon
esophagus
gastric
germcell
head-neck
kidney
leukemia
lung
lymphoma
myleloma
tissue atlas
ovary
pancreas
prostate
thyroid
54 cancer vs normal comparisons
FDR < 5%

FIG. 4

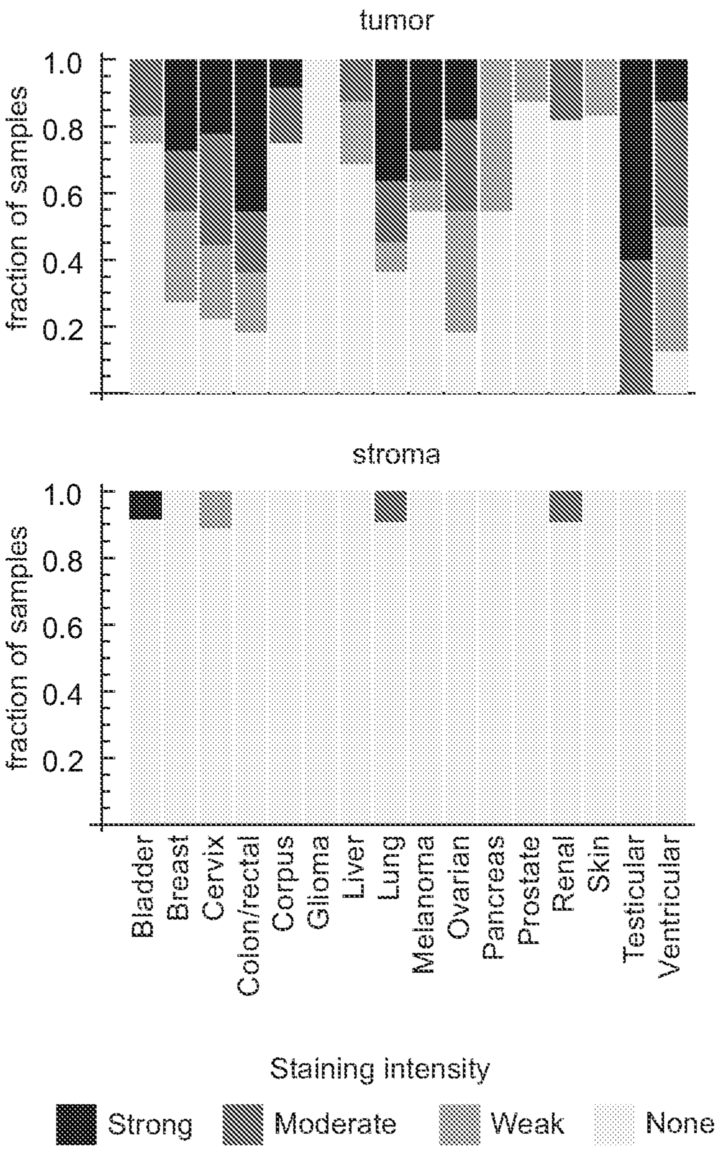
tumor
fraction of samples
1.0
0.8
0.6
0.4
0.2
stroma
fraction of samples
1.0
0.8
0.6
0.4
0.2
Bladder
Breast
Cervix
Colon/rectal
Corpus
Glioma
Liver
Lung
Melanoma
Ovarian
Pancreas
Prostate
Renal
Skin
Testicular
Ventricular
Staining intensity
Strong
Moderate
Weak
None

FIG. 5

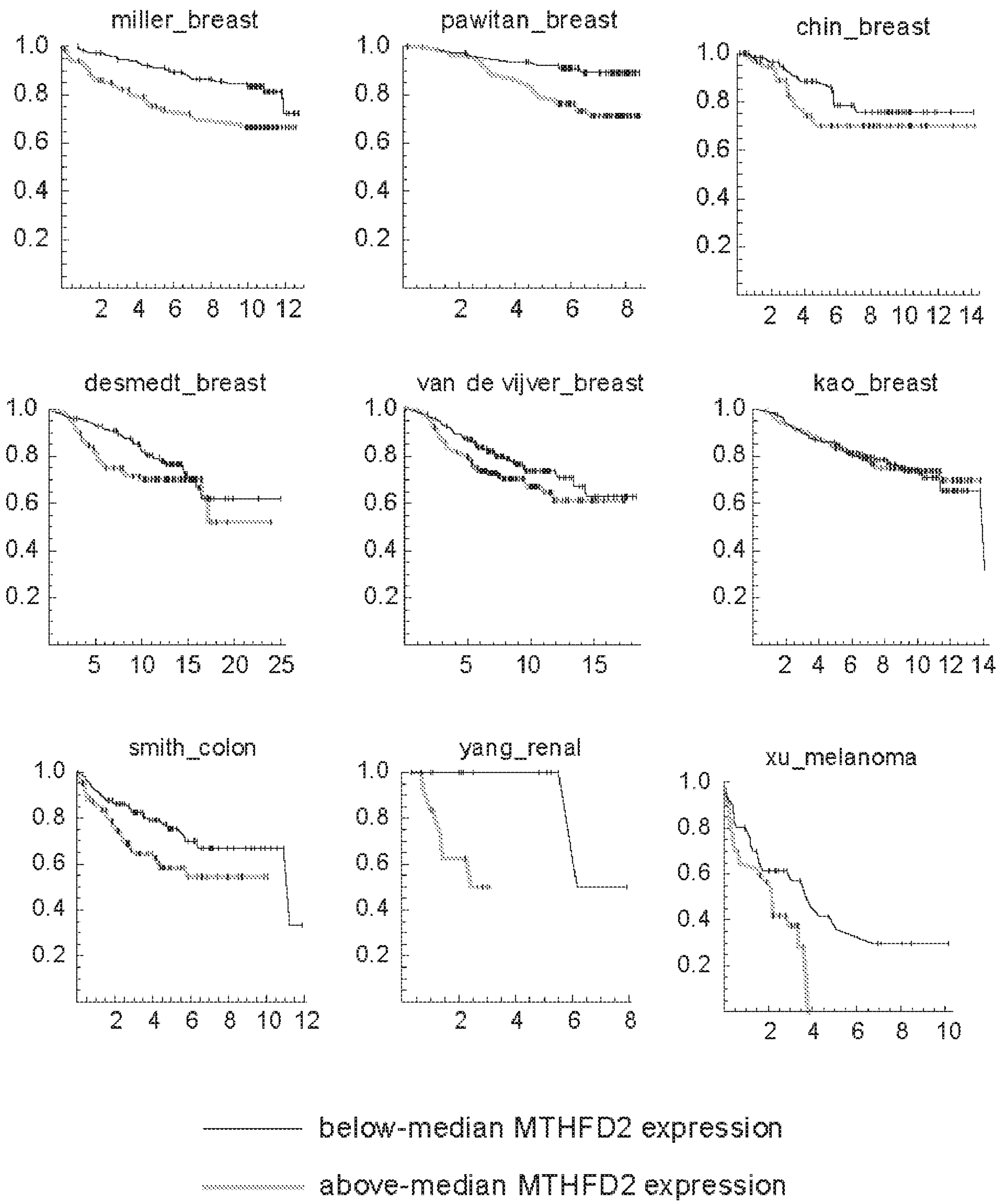
miller_breast
pawitan_breast
chin_breast
desmedt_breast
van de vijver_breast
kao_breast
smith_colon
yang_renal
xu_melanoma
below-median MTHFD2 expression
above-median MTHFD2 expression

FIG. 6

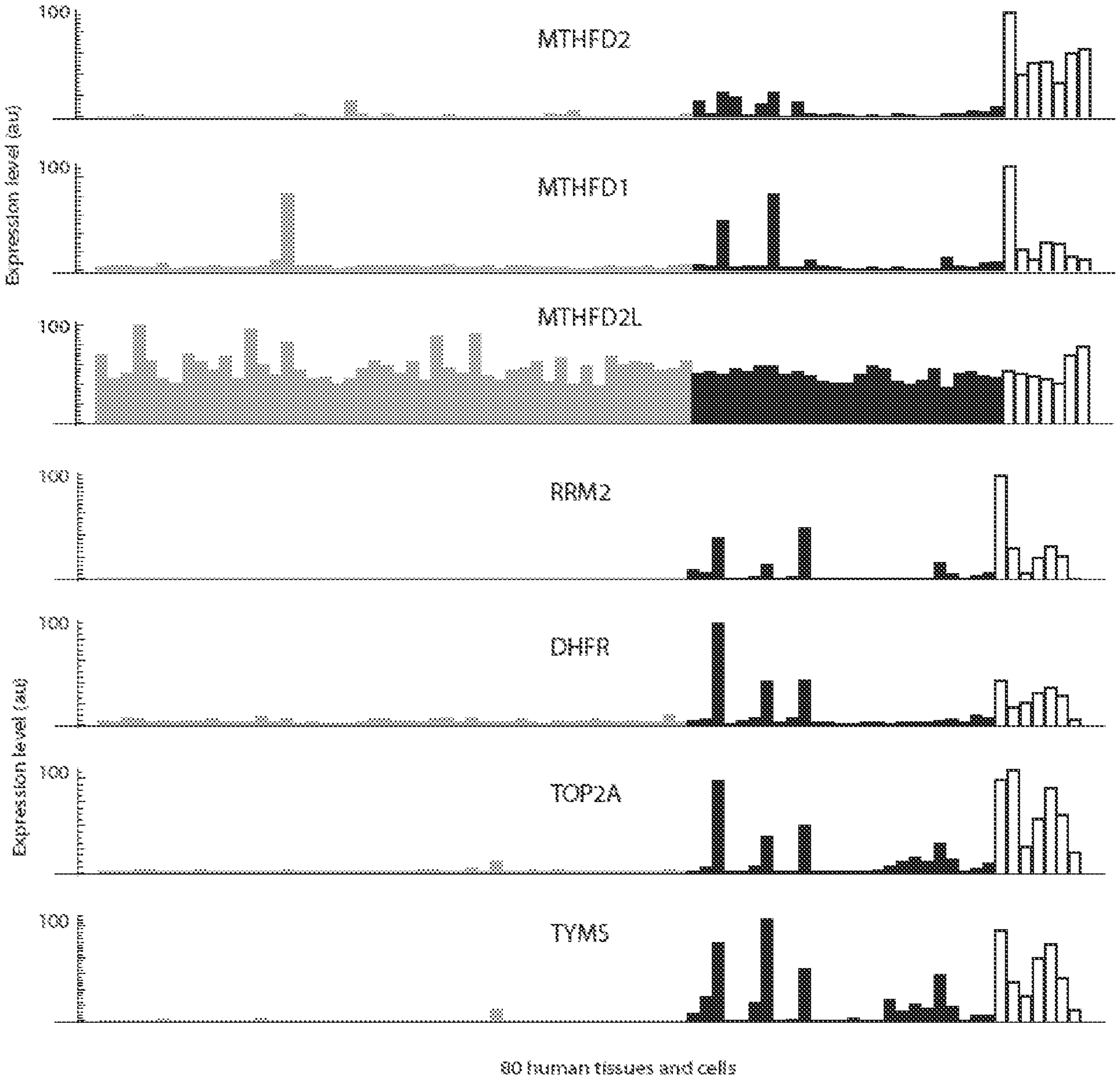
100
MTHFD2
100
MTHFD1
100
MTHFD2L
Expression level (au)
100
RRM2
100
DHFR
100
TOP2A
100
TYMS
Expression level (au)
80 human tissues and cells

FIG. 7A

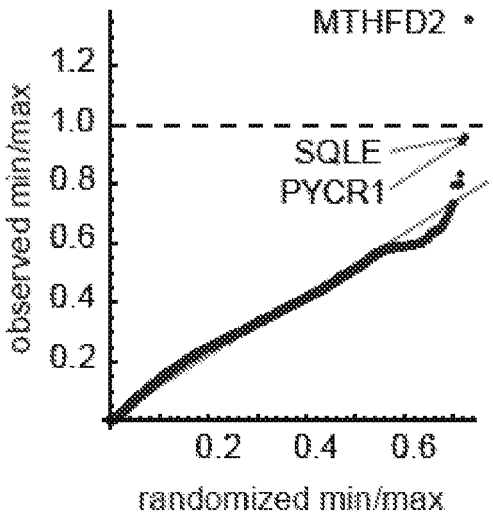
MTHFD2
1.2
1.0
0.8
0.6
0.4
0.2
SQLE
PYCR1
observed min/max
0.2
0.4
0.6
randomized min/max

FIG. 7B

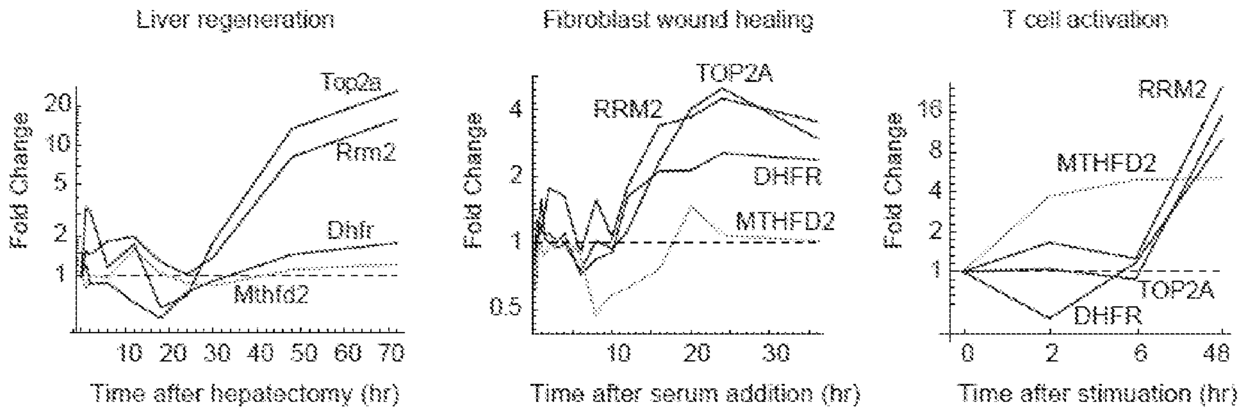
Liver regeneration
Fibroblast wound healing
T cell activation
Top2a
Rrm2
Dhfr
Mthfd2
Fold Change
Time after hepatectomy (hr)
TOP2A
RRM2
DHFR
MTHFD2
Time after serum addition (hr)
Time after stimuation (hr)

FIG. 7C

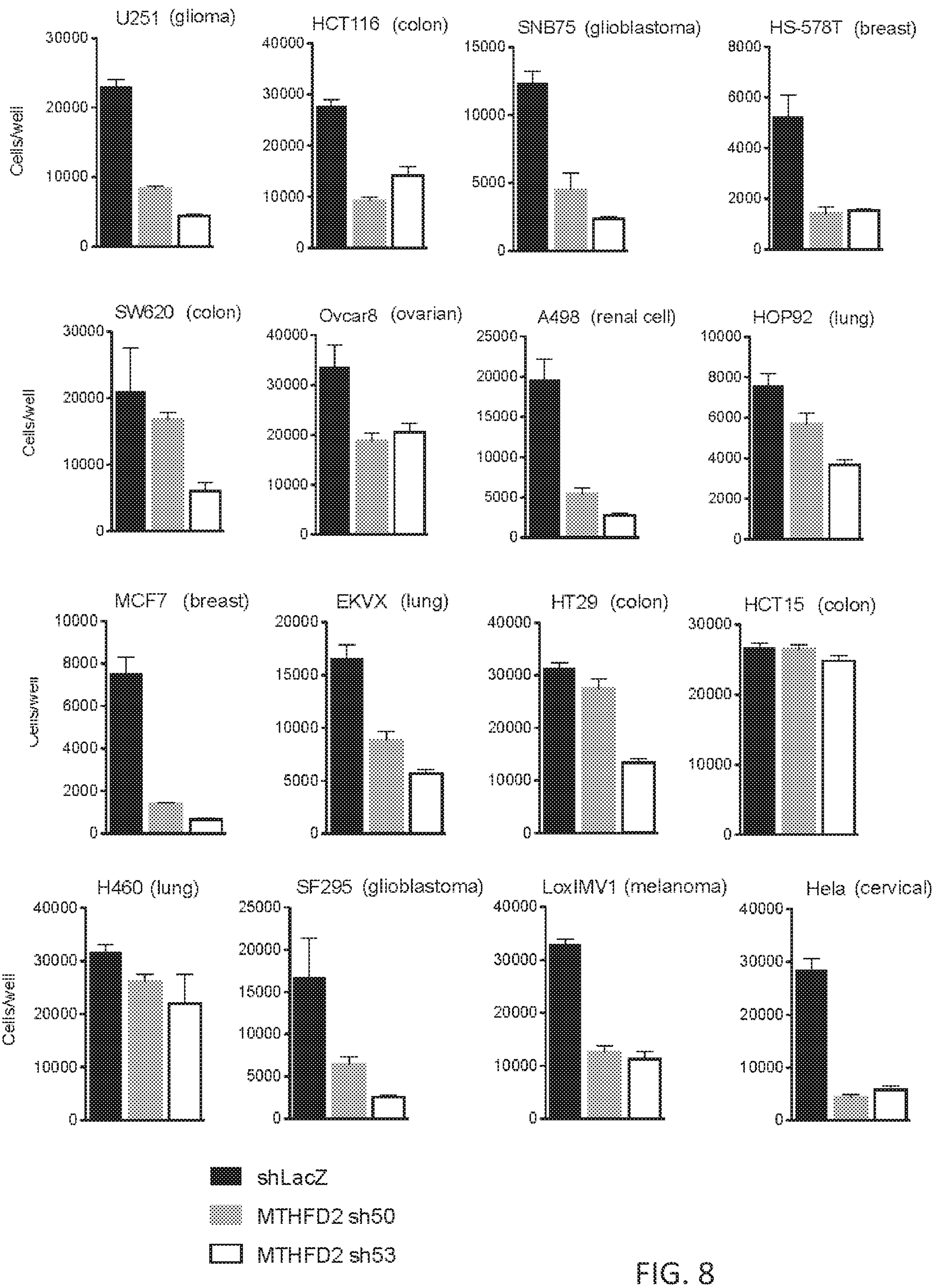
U251 (glioma)
HCT116 (colon)
SNB75 (glioblastoma)
HS-578T (breast)
SW620 (colon)
Ovcar8 (ovarian)
A498 (renal cell)
HOP92 (lung)
MCF7 (breast)
EKVX (lung)
HT29 (colon)
HCT15 (colon)
H460 (lung)
SF295 (glioblastoma)
LoxIMV1 (melanoma)
Hela (cervical)
Cells/well
shLacZ
MTHFD2 sh50
MTHFD2 sh53

FIG. 8

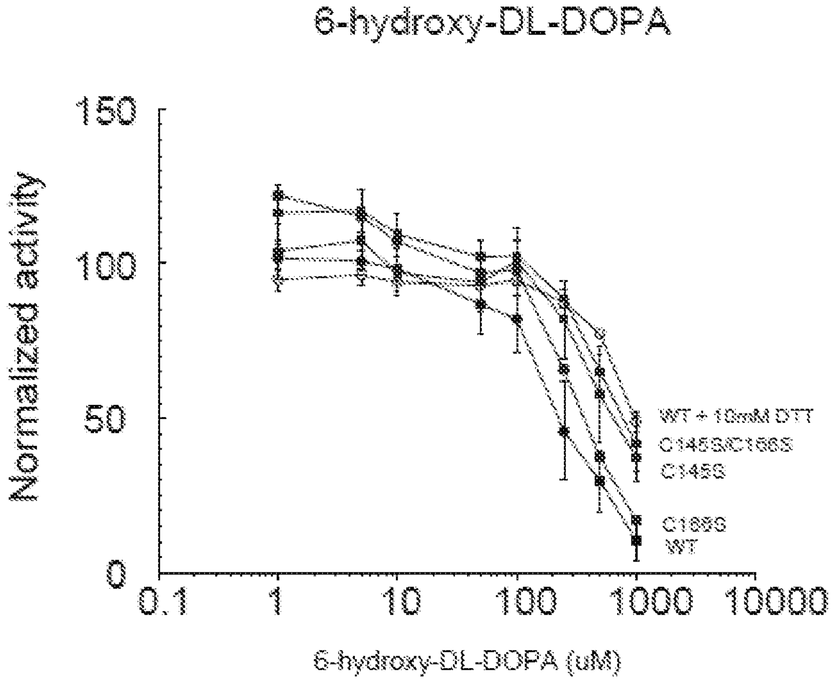
6-hydroxy-DL-DOPA
150
100
50
0
Normalized activity
0.1
1
10
100
1000
10000
6-hydroxy-DL-DOPA (uM)
WT + 10mM DTT
C145S/C166S
C145S
C166S
WT

FIG. 9A

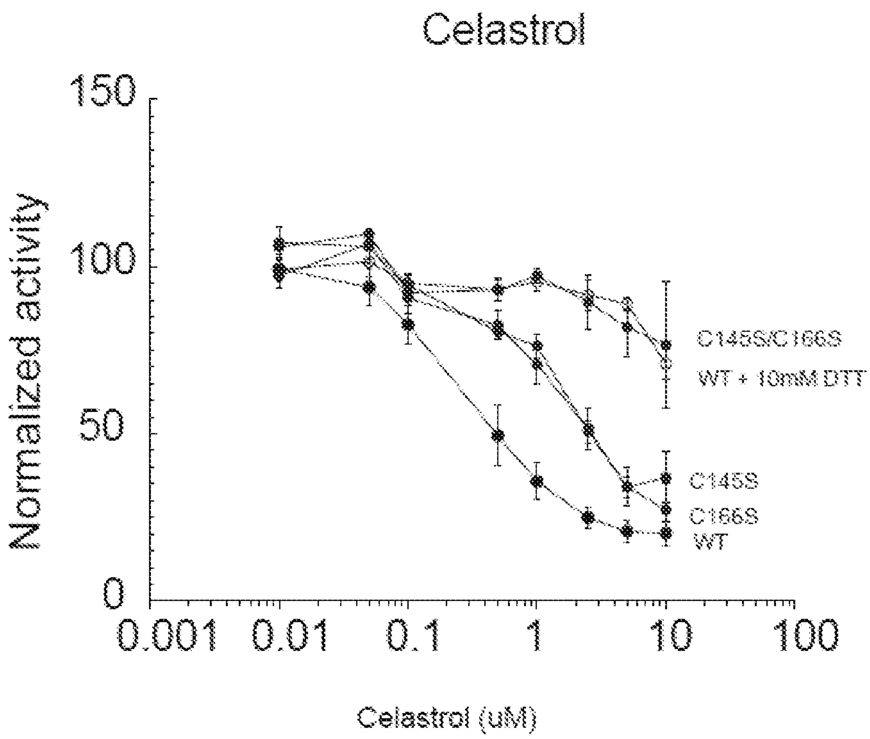
Celastrol
150
100
50
0
Normalized activity
0.001
0.01
0.1
1
10
100
Celastrol (uM)
C145S/C166S
WT + 10mM DTT
C145S
C166S
WT

FIG. 9B

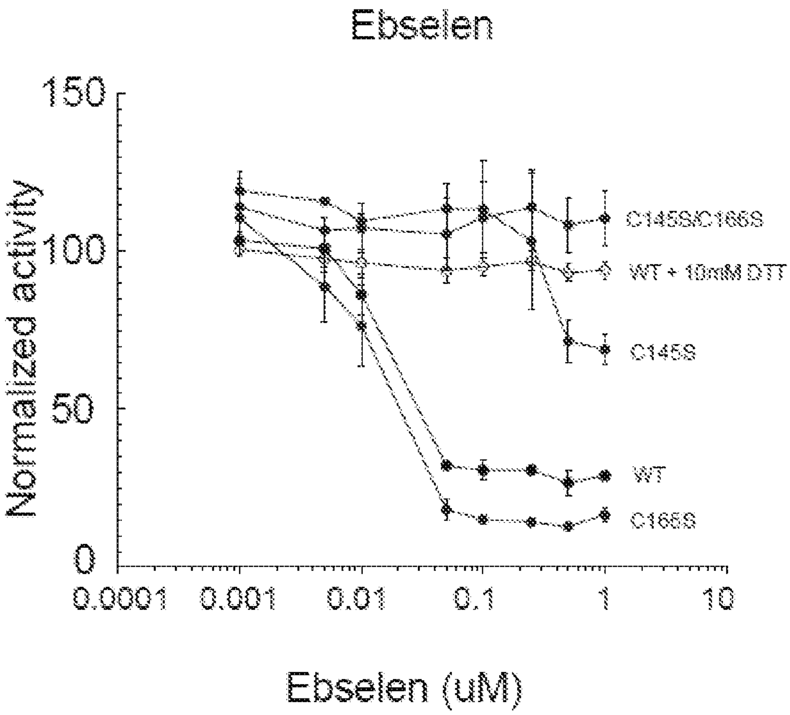
Ebselen
150
100
50
0
Normalized activity
0.0001
0.001
0.01
0.1
1
10
Ebselen (uM)
C145S/C166S
WT + 10mM DTT
C145S
WT
C166S

FIG. 9C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2010203508 A1
- US 5834228 A
- US 5939528 A
- US 5856116 A
- US 9816879 W
- WO 9909148 A
- US 8198328 B
- US 5093246 A
- US 4987071 A, Cech
- US 5116742 A, Cech
- US 5582981 A, Toole
- US 6468798 B
- US 4522811 A
- US 6503713 B
- US 61791082 B


### Non-patent literature cited in the description


- **NOWELL.** *Science,* 1976, vol. 194, 23
- **HANAHAN ; WEINBERG.** *Cell,* 2011, vol. 144, 646
- **STRATTON et al.** *Nature,* 2009, vol. 458, 719
- **HSU ; SABATINI.** *Cell,* 2008, vol. 134, 703
- **SREEKUMAR et al.** *Nature,* 2009, vol. 457, 910
- **DEBERARDINIS et al.** *Proc Natl Acad Sci U S A,* 2007, vol. 104, 19345
- **M. JAIN et al.** *Science,* 2012, vol. 336 (6084), 1040-1044
- **S. D. SELCUKLU et al.** *Journal of Biological Chemistry,* 2012, vol. 287 (35), 29516-29528
- **UNHEE LIM et al.** *Blood,* 2007, vol. 109 (7), 3050-3059
- **STÉPHANE PUYO et al.** *Cancer Research,* vol. 70 (8), 2692-2692
- **VAZQUEZ A et al.** *Cancer Research,* 2012, vol. 73, 478-482
- **ZHANG et al.** *Cell,* 2012, vol. 148, 259
- **MORROW et al.** *FEBS Lett.,* 1998, vol. 439 (3), 334-340
- **WILLIAMS et al.** *Anal. Biochem.,* 2003, vol. 321 (1), 31-37
- **ALLAN et al.** *Combinatorial Chemistry & High Throughput Screening,* 2006, vol. 9, 9-14
- **KOPEK et al.** *J Biomol Screen.,* 2009, vol. 14 (10), 1185-94
- **BOLSTER et al.** *Int J Rad Appl Instrum Part A,* 1986, vol. 37 (9), 985-7
- **JOHNSTROM et al.** *Int J Rad Appl Instrum A.,* 1987, vol. 38 (9), 729-34
- **ANDERSON et al.** *Proc Natl Acad Sci U S A.,* 2011, vol. 108, 15163-15168
- **MCENTEE et al.** *Proc Natl Acad Sci U S A.,* 2011, vol. 108, 15157-15162
- **SAMSONOFF et al.** *The Journal of biological chemistry,* 1997, vol. 272, 13281-13285
- **MEJIA et al.** *The Journal of biological chemistry,* 1985, vol. 260, 14616-14620
- **TIBBETTS.** *Appling, Annu Rev Nutr.,* 2010, vol. 30, 57-81
- **BOLUSANI et al.** *The Journal of biological chemistry,* 2011, vol. 286, 5166-5174
- **CHRISTENSEN et al.** *J. Biol. Chem.,* 2005, vol. 280 (9), 7597-602
- **APPAJI RAO et al.** *Biochim Biophys Acta,* 11 April 2003, vol. 1647 (1-2), 24-9
- **STOVER et al.** *J. Biol. Chem.,* vol. 265 (24), 14227-33
- **SAMBROOK et al.** Molecular Cloning: A Laboratory Manual. Cold Spring Harbor Press, 2001
- Genomics. Modern genetic Analysis. 1999
- **W. H. FREEMAN ; EKINS ; CHU.** *Trends in Biotechnology,* 1999, vol. 17, 217-218
- **MACBEATH ; SCHREIBER.** *Science,* 2000, vol. 289 (5485), 1760-1763
- **SIMPSON.** Proteins and Proteomics: A Laboratory Manual. Cold Spring Harbor Laboratory Press, 2002
- **HARDIMAN.** Microarrays Methods and Applications: Nuts & Bolts. DNA Press, 2003
- **TAYLOR ; WEISSBACH.** *Anal. 1 Biochem.,* 1965, vol. 13, 80-84
- **ZHANG et al.** *Anal Biochem.,* 15 April 2008, vol. 375 (2), 367-9
- **MCGUIRE.** *Curr Pharm Des.,* 2003, (31), 2593-613
- **GANGJEE et al.** *Anticancer Agents Med Chem.,* September 2007, vol. 7 (5), 524-42
- **GONEN ; ASSARAF.** *Drug Resist Updat.,* August 2012, vol. 15 (4), 183-210

- **PEREIRA et al.** *J Am Chem Soc.,* 2011, vol. 133 (10), 3260-3
- **ZIEGLER et al.** *Biochemistry,* 2007, vol. 46 (10), 2674-83
- **PARKER et al.** *Mamm Genome.,* 2010, vol. 21 (11-12), 565-76
- **SINGH et al.** *Nature reviews. Drug discovery,* 2011, vol. 10, 307-317
- **JONES et al.** *J. Mol. Biol.,* 1995, 43-53
- **KRAULIS.** *J. Appl. Crystallogr.,* 1991, vol. 24, 946-950
- **BACON ; ANDERSON.** *J. Mol. Graph.,* 1998, vol. 6, 219-220
- **DELANO.** *The PyMOL Molecular Graphics System,* 2002
- **WISSNER et al.** *J Med Chem.,* 2003, vol. 46 (1), 49-63
- **AHN et al.** *Chem Biol.,* 2009, vol. 16 (4), 411-20
- **SUN ; HAI LIU.** *Cancer Lett.,* 08 September 2006, vol. 241 (1), 124-34
- **NETO et al.** *Mol Nutr Food Res.,* June 2008, vol. 52 (1), 18-27
- **BAGELLA et al.** *J. Cell. Physiol.,* 1998, vol. 177, 206-213
- **GAULTIER et al.** *Nucleic Acids. Res.,* 1987, vol. 15, 6625-6641
- **INOUE et al.** *Nucleic Acids Res.,* 1987, vol. 15, 6131-6148
- **INOUE et al.** *FEBS Lett.,* 1987, vol. 215, 327-330
- **HEASMAN.** *Dev. Biol.,* 2002, vol. 243, 209-14
- **IVERSEN.** *Curr. Opin. Mol. Ther.,* 2001, vol. 3, 235-8
- **SUMMERTON.** *Biochim. Biophys. Acta,* 1999, vol. 1489, 141-58
- **HELENE.** *C. Anticancer Drug Des.,* 1991, vol. 6, 569-84
- **HELENE, C.** *Ann. N.Y. Acad. Sci.,* 1992, vol. 660, 27-36
- **MAHER.** *Bioassays,* 1992, vol. 14, 807-15
- **HASELHOFF ; GERLACH.** *Nature,* 1988, vol. 334, 585-591
- **BARTEL ; SZOSTAK.** *Science,* 1993, vol. 261, 1411-1418
- **BOCK et al.** *Nature,* 1992, vol. 355, 564-566
- **FAMULOK.** *Curr. Opin. Struct. Biol.,* 1999, vol. 9, 324
- **HERMAN ; PATEL, J.** *Science,* 2000, vol. 287, 820-825
- **KELLY et al.** *J. Mol. Biol.,* 1996, vol. 256, 417
- **FEIGON et al.** *Chem. Biol.,* 1996, vol. 3, 611
- Remington: The Science and Practice of Pharmacy. 2005
- Drugs and the Pharmaceutical Sciences: a Series of Textbooks and Monographs. Dekker
- **OBRECHT ; VILLALGORDO.** Solid-Supported Combinatorial and Parallel Synthesis of Small-Molecular-Weight Compound Libraries. Pergamon-Elsevier Science Limited, 1998
- **CZARNIK.** *Curr. Opin. Chem. Bio,* 1997, vol. 1, 60-6
- **SHOEMAKER.** *Nat Rev Cancer.,* 2006, vol. 6, 813-823
- **O'CONNOR et al.** *Cancer Res.,* 1997, vol. 57, 4285-4300
- **ALPERT.** *J Chromatogr.,* 1990, vol. 499, 177-196
- **LUO et al.** *J Chromatogr A.,* 2007, vol. 1147, 153-164
- **LUO et al.** *Proc Natl Acad Sci USA.,* 1998, vol. 95, 14863-14868
- **DE LEEUW ; MAIR.** *Journal of Statistical Software,* 2009, 31
- **RANTANEN et al.** *Metab Eng.,* 2002, vol. 4, 285-294
- **ZAMBONI et al.** *Nat Protoc.,* 2009, vol. 4, 878-892
- **MOFFATT et al.** *Cell.,* 2006, vol. 124, 1283-1298
- **SUBRAMANIAN et al.** *Proc Natl Acad Sci USA.,* 2005, vol. 102, 15545-15550
- **SAKAUE-SAWANO et al.** *Cell,* 2008, vol. 132, 487-498
- **TOETTCHER et al.** *Proc Natl Acad Sci USA.,* 2009, vol. 106, 785-790
- **CHIN et al.** *Cancer Cell,* 2006, vol. 10, 529-541
- **VAN DE VIJVER et al.** *N Engl J Med.,* 2002, vol. 347, 1999-2009
- **DESMEDT et al.** *Clin Cancer Res.,* 2007, vol. 13, 3207-3214
- **PAWITAN et al.** *Breast Cancer Res.,* 2005, vol. 7, 953-964
- **MILLER et al.** *Proc Natl Acad Sci U S A,* 20 September 2005, vol. 102 (38), 13550-5
- **KAO et al.** *BMC Cancer.,* 2011, vol. 11, 143
- **COX.** *Journal of the Royal Statistical Society,* 1972, vol. 34, 187-220
- **BLAND ; ALTMAN.** *BMJ.,* 2004, vol. 328, 1073
- **DERSIMONIAN ; LAIRD.** *Control Clin Trials,* 1986, vol. 7, 177-188
- **CHRISTENSEN et al.** *The Journal of biological chemistry,* 2005, vol. 280, 34316-34323
- **SCRIMGEOUR ; HUENNEKENS.** *Biochem. Biophys. Res. Commun.,* 1960, vol. 2, 230-233
- **MEJIA ; MACKENZIE.** *The Journal of biological chemistry,* 1985, vol. 260, 14616-14620
- **YANG.** *MacKenzie Biochemistry,* 1993, vol. 32, 11118-11123
- **NOWELL PC.** The clonal evolution of tumor cell populations. *Science,* 1976, vol. 194, 23-28

- **HANAHAN D ; WEINBERG RA.** Hallmarks of cancer: The next generation. *Cell,* 2011, vol. 144, 646-674
- **STRATTON MR ; CAMPBELL PJ ; FUTREAL PA.** The cancer genome. *Nature,* 2009, vol. 458, 719-724
- **HSU PP ; SABATINI DM.** Cancer cell metabolism: Warburg and beyond. *Cell,* 2008, vol. 134, 703-707
- **SREEKUMAR A ; POISSON LM ; RAJENDIRAN TM ; KHAN AP ; CAO Q ; YU J ; LAXMAN B ; MEHRA R ; LONIGRO RJ ; LI Y.** Metabolomic profiles delineate potential role for sarcosine in prostate cancer progression. *Nature,* 2009, vol. 457, 910-914
- **DEBERARDINIS RJ ; MANCUSO A ; DAIKHIN E ; NISSIM I ; YUDKOFF M ; WEHRLI S ; THOMPSON CB.** Beyond aerobic glycolysis: Transformed cells can engage in glutamine metabolism that exceeds the requirement for protein and nucleotide synthesis. *Proc Natl Acad Sci USA.,* 2007, vol. 104, 19345-19350
- **ANDERSON DD ; QUINTERO CM ; STOVER PJ.** Identification of a de novo thymidylate biosynthesis pathway in mammalian mitochondria. *Proc Natl Acad Sci USA.,* 2011, vol. 108, 15163-15168
- **MCENTEE G ; MINGUZZI S ; O'BRIEN K ; BEN LARBI N ; LOSCHER C ; O'FAGAIN C ; PARLE-MCDERMOTT A.** The former annotated human pseudogene dihydrofolate reductase-like 1 (dhfrll) is expressed and functional. *Proc Natl Acad Sci USA.,* 2011, vol. 108, 15157-15162
- **SAMSONOFF WA ; RESTON J ; MCKEE M ; O'CONNOR B ; GALIVAN J ; MALEY G ; MALEY F.** Intracellular location of thymidylate synthase and its state of phosphorylation. *The Journal of biological chemistry,* 1997, vol. 272, 13281-13285
- **MEJIA NR ; MACKENZIE RE.** Nad-dependent methylenetetrahydrofolate dehydrogenase is expressed by immortal cells. *The Journal of biological chemistry,* 1985, vol. 260, 14616-14620
- **TIBBETTS AS ; APPLING DR.** Compartmentalization of mammalian folate-mediated one-carbon metabolism. *Annu Rev Nutr.,* 2010, vol. 30, 57-81
- **BOLUSANI S ; YOUNG BA ; COLE NA ; TIBBETTS AS ; MOMB J ; BRYANT JD ; SOLMONSON A ; APPLING DR.** Mammalian mthfd21 encodes a mitochondrial methylenetetrahydrofolate dehydrogenase isozyme expressed in adult tissues. *The Journal of biological chemistry,* 2011, vol. 286, 5166-5174
- **YOUSIF LF ; STEWART KM ; KELLEY SO.** Targeting mitochondria with organelle-specific compounds: Strategies and applications. *Chembiochem : a European journal of chemical biology,* 2009, vol. 10, 1939-1950
- **FILIPOVSKA A ; KELSO GF ; BROWN SE ; BEER SM ; SMITH RA ; MURPHY MP.** Synthesis and characterization of a triphenylphosphonium-conjugated peroxidase mimetic. Insights into the interaction of ebselen with mitochondria. *The Journal of biological chemistry,* 2005, vol. 280, 24113-24126
- **SERAFIMOVA IM ; PUFALL MA ; KRISHNAN S ; DUDA K ; COHEN MS ; MAGLATHLIN RL ; MCFARLAND JM ; MILLER RM ; FRODIN M ; TAUNTON J.** Reversible targeting of noncatalytic cysteines with chemically tuned electrophiles. *Nature chemical biology,* 2012, vol. 8, 471-476
- **J, PETTER RC ; BAILLIE TA ; WHITTY A.** The resurgence of covalent drugs. *Nature reviews. Drug discovery,* 2011, vol. 10, 307-317
- **SHOEMAKER RH.** The nci60 human tumour cell line anticancer drug screen. *Nat Rev Cancer,* 2006, vol. 6, 813-823
- **ALLEN J ; DAVEY HM ; BROADHURST D ; HEALD JK ; ROWLAND JJ ; OLIVER SG ; KELL DB.** High-throughput classification of yeast mutants for functional genomics using metabolic footprinting. *Nat Biotechnol.,* 2003, vol. 21, 692-696
- **SHAHAM O ; SLATE NG ; GOLDBERGER O ; XU Q ; RAMANATHAN A ; SOUZA AL ; CLISH CB ; SIMS KB ; MOOTHA VK.** A plasma signature of human mitochondrial disease revealed through metabolic profiling of spent media from cultured muscle cells. *Proc Natl AcadSci USA,* 2010, vol. 107, 1571-1575
- **O'CONNOR PM ; JACKMAN J ; BAE I ; MYERS TG ; FAN S ; MUTOH M ; SCUDIERO DA ; MONKS A ; SAUSVILLE EA ; WEINSTEIN JN.** Characterization of the p53 tumor suppressor pathway in cell lines of the national cancer institute anticancer drug screen and correlations with the growth-inhibitory potency of 123 anticancer agents. *Cancer Res.,* 1997, vol. 57, 4285-4300
- **KATZ-BRULL R ; DEGANI H.** Kinetics of choline transport and phosphorylation in human breast cancer cells; nmr application of the zero trans method. *Anticancer Res.,* 1996, vol. 16, 1375-1380
- **DUARTE NC ; BECKER SA ; JAMSHIDI N ; THIELE I ; MO ML ; VO TD ; SRIVAS R ; PALSSON BO.** Global reconstruction of the human metabolic network based on genomic and bibliomic data. *Proc Natl Acad Sci USA.,* 2007, vol. 104, 1777-1782
- **NIKIFOROV MA ; CHANDRIANI S ; O'CONNELL B ; PETRENKO O ; KOTENKO I ; BEAVIS A ; SEDIVY JM ; COLE MD.** A functional screen for myc-responsive genes reveals serine hydroxymethyltransferase, a major source of the one-carbon unit for cell metabolism. *Mol Cell Biol.,* 2002, vol. 22, 5793-5800
- **KAO F ; CHASIN L ; PUCK TT.** Genetics of somatic mammalian cells. X. Complementation analysis of glycine-requiring mutants. *Proc Natl Acad Sci USA.,* 1969, vol. 64, 1284-1291

- **ZHANG WC ; SHYH-CHANG N ; YANG H ; RAI A ; UMASHANKAR S ; MA S ; SOH BS ; SUN LL ; TAI BC ; NGA ME.** Glycine decarboxylase activity drives non-small cell lung cancer tumor-initiating cells and tumorigenesis. *Cell,* 2012, vol. 148, 259-272
- **PATEL H ; PIETRO ED ; MACKENZIE RE.** Mammalian fibroblasts lacking mitochondrial nad+-dependent methylenetetrahydrofolate dehydrogenase-cyclohydrolase are glycine auxotrophs. *The Journal of biological chemistry,* 2003, vol. 278, 19436-19441
- **CHIN K ; DEVRIES S ; FRIDLYAND J ; SPELLMAN PT ; ROYDASGUPTA R ; KUO WL ; LAPUK A ; NEVE RM ; QIAN Z ; RYDER T.** Genomic and transcriptional aberrations linked to breast cancer pathophysiologies. *Cancer Cell,* 2006, vol. 10, 529-541
- **DESMEDT C ; PIETTE F ; LOI S ; WANG Y ; LALLEMAND F ; HAIBE-KAINS B ; VIALE G ; DELORENZI M ; ZHANG Y ; D'ASSIGNIES MS.** Strong time dependence of the 76-gene prognostic signature for node-negative breast cancer patients in the transbig multicenter independent validation series. *Clin Cancer Res.,* 2007, vol. 13, 3207-3214
- **PAWITAN Y ; BJOHLE J ; AMLER L ; BORG AL ; EGYHAZI S ; HALL P ; HAN X ; HOLMBERG L ; HUANG F ; KLAAR S.** Gene expression profiling spares early breast cancer patients from adjuvant therapy: Derived and validated in two population-based cohorts. *Breast Cancer Res.,* 2005, vol. 7, 953-964
- **VAN DE VIJVER MJ ; HE YD ; VAN'T VEER LJ ; DAI H ; HART AA ; VOSKUIL DW ; SCHREIBER GJ ; PETERSE JL ; ROBERTS C ; MARTON MJ.** A gene-expression signature as a predictor of survival in breast cancer. *N Engl J Med.,* 2002, vol. 347, 1999-2009
- **KAO KJ ; CHANG KM ; HSU HC ; HUANG AT.** Correlation of microarray-based breast cancer molecular subtypes and clinical outcomes: Implications for treatment optimization. *BMC Cancer,* 2011, vol. 11, 143
- **MILLER LD ; SMEDS J ; GEORGE J ; VEGA VB ; VERGARA L ; PLONER A ; PAWITAN Y ; HALL P ; KLAAR S ; LIU ET.** An expression signature for p53 status in human breast cancer predicts mutation status, transcriptional effects, and patient survival. *Proc Natl Acad Sci U S A.,* 2005, vol. 102, 13550-13555
- **LIU F ; WHITE JA ; ANTONESCU C ; GUSENLEITNER D ; QUACKENBUSH J.** Gcod - genechip oncology database. *BMC bioinformatics,* 2011, vol. 12, 46
- **RHODES DR ; YU J ; SHANKER K ; DESHPANDE N ; VARAMBALLY R ; GHOSH D ; BARRETTE T ; PANDEY A ; CHINNAIYAN AM.** Large-scale meta-analysis of cancer microarray data identifies common transcriptional profiles of neoplastic transformation and progression. *Proc Natl Acad Sci U S A.,* 2004, vol. 101, 9309-9314
- **LEHTINEN L ; KETOLA K ; MAKELA R ; MPINDI JP ; VIITALA M ; KALLIONIEMI O ; ILJIN K.** High-throughput rnai screening for novel modulators of vimentin expression identifies mthfd2 as a regulator of breast cancer cell migration and invasion. *Oncotarget,* 2013, vol. 4, 48-63
- **SELCUKLU SD ; DONOGHUE MT ; REHMET K ; DE SOUZA GOMES M ; FORT A ; KOVVURU P ; MUNIYAPPA MK ; KERIN MJ ; ENRIGHT AJ ; SPILLANE C.** Microrna-9 inhibition of cell proliferation and identification of novel mir-9 targets by transcriptome profiling in breast cancer cells. *The Journal of biological chemistry,* 2012, vol. 287, 29516-29528
- **SMITH GK ; BANKS SD ; RIGUAL R ; DUCH DS ; MULLIN RJ ; HUBER BE.** Activity of an nad-dependent 5,10-methylenetetrahydrofolate dehydrogenase in normal tissue, neoplastic cells, and oncogene-transformed cells. *Archives of biochemistry and biophysics,* 1990, vol. 283, 367-371
- **UHLEN M ; BJORLING E ; AGATON C ; SZIGYARTO CA ; AMINI B ; ANDERSEN E ; ANDERSSON AC ; ANGELIDOU P ; ASPLUND A ; ASPLUND C.** A human protein atlas for normal and cancer tissues based on antibody proteomics. *Molecular & cellular proteomics : MCP,* 2005, vol. 4, 1920-1932
- **CHRISTENSEN KE ; MIRZA IA ; BERGHUIS AM ; MACKENZIE RE.** Magnesium and phosphate ions enable nad binding to methylenetetrahydrofolate dehydrogenase-methenyltetrahydrofolate cyclohydrolase. *The Journal of biological chemistry,* 2005, vol. 280, 34316-34323
- **YANG XM ; MACKENZIE RE.** Nad-dependent methylenetetrahydrofolate dehydrogenase-methenyltetrahydrofolate cyclohydrolase is the mammalian homolog of the mitochondrial enzyme encoded by the yeast mis1 gene. *Biochemistry,* 1993, vol. 32, 11118-11123